# EUROPEAN PATENT APPLICATION

(11) **EP 2 462 981 A1**
(43) Date of publication of application: **13.06.2012**
(21) Application number: 10806509.5
(22) Date of filing: 05.08.2010
(51) Int. Cl.: A61N 1/05, A61N 1/36

(54) **ELECTRIC STIMULATOR**

(30) Priority: 06.08.2009 JP 2009183825; 07.09.2009 JP 2009206381; 14.10.2009 JP 2009237436
(71) Applicant: Terumo Kabushiki Kaisha, Tokyo 151-0072 (JP)
(72) Inventor: FUKUI, Yoshihito, Ashigarakami-gun Kanagawa 259-0151 (JP); UNO, Takuya, Ashigarakami-gun Kanagawa 259-0151 (JP); ONODA, Masahiro, Ashigarakami-gun Kanagawa 259-0151 (JP)
(74) Representative: Dossmann, Gérard
(86) International application number: PCT/JP2010/063269
(87) International publication number: WO 2011/016510

(57) **Abstract**

Stimulation electrodes are facilitated to be precisely and stably placed at a predetermined position in a body cavity, such as an epidural space, and to be removed from a living body. An electric stimulator includes a stimulation circuit block having a stimulation electrode that stimulates nerves or muscles in a living body and a stimulation circuit that is electrically connected to the stimulation electrode to apply a stimulation signal to the stimulation electrode. Additionally, the electric stimulator further includes a support that is connected to the stimulation circuit block to hold the stimulation electrode at an implantation position in the living body. The stimulation circuit block and the support, except for at least one end of the support, have a shape capable of being inserted into the duct of a tubular insertion tool. [FIG. 5]
309: RECHARGEABLE BATTERY
SUPPLY OF ELECTRIC POWER TO RESPECTIVE SECTIONS
212: COIL PORTION
308: CHARGING SECTION
302: COMMUNICATION SECTION
303: CONTROL SECTION
306: OSCILLATION SECTION
304: STIMULATION PARAMETER SETTING SECTION
307: SWITCH SECTION
305: ELECTRODE CONFIGURATION SETTING SECTION
TO RESPECTIVE STIMULATION ELECTRODES 105

## Description

### Technical Field

The present invention relates to an electric stimulator that electrically stimulates a living body, and particularly, to an electric stimulator used so as to be wholly implanted in a living body.

### Background Art

At present, in pain treatment, when conventional medication therapy, nerve block therapy, or surgical therapy do not show any effect or when treatment cannot be continued due to side effects, an electric stimulation therapy that electrically stimulates nerves to relieve pain is achieving results. An electrical spinal cord stimulation therapy which is one of the electric stimulation therapies is a stimulation therapy which electrically stimulates the spinal cord, in order to relieve pain that propagates to the brain via the spinal cord.

In the electrical spinal cord stimulation therapy, usually, in order to confirm the validity of pain relief caused by the electric stimulation, a trial period of 24 hours to several weeks is provided. In the trial period, generally, stimulation electrodes are inserted from the back side and placed in an epidural space outside a spinal dura mater that covers the spinal cord, and then, an electrode lead including these stimulation electrodes is connected to an external stimulator outside of the body, and the degree of pain relief is investigated under various stimulation patterns. Implantation of an electric stimulator is not performed in this period. Only when a predetermined effect is seen in this trial period, a long-term implantation (hereinafter referred to as "implantation") of the electric stimulator carried out.

When the implantation of the electric stimulator is performed, the electrode lead placed in the trial period is removed, and then new stimulation electrodes are again placed in the epidural space, and an electrode lead including these stimulation electrodes is led to the waist, the abdomen, or the chest through a subcutaneous tunnel. Then, the electrode lead is connected to the electric stimulator and the electric stimulator is implanted subcutaneously therein.

Meanwhile, in the trial period in the electrical spinal cord stimulation therapy, the electrode lead is connected to the external stimulator outside the body. Therefore, there are problems, such as the risk of infection, the restriction of the activity of a patient, and affecting judgment of the effectiveness of pain relief since the restriction of the activity acts as stress.

On the other hand, a micro stimulator with no lead including electrodes at both ends of a housing is disclosed in a technique described in PTL 1. In this technique, the entire micro stimulator is wholly implanted near nerves, so that the risk or infection can be reduced, and the restriction of the activity of a patient can be reduced as much as possible.

### Citation List

### Patent Literature

PTL 1: Specification of US Patent No. 5,193,539

### Summary of the Invention

### Technical Problem

However, when the micro stimulator described in Patent Document 1 is placed in a body cavity, such as an epidural space in the electrical spinal cord stimulation therapy, there is a problem in that it is difficult to place the stimulation electrodes of the stimulator precisely at a desired position, and to continue placing the stimulation electrodes at the position stably for a prolonged period of time. Additionally, there is also a problem in that, once the stimulator is placed in the body cavity, it is difficult to remove this stimulator out of the body.

The invention has been made in view of this point, and the object of the invention is to provide an electric stimulator that can easily and precisely place stimulation electrodes at a predetermined position in a body cavity, such as an epidural space, precisely, and can place the stimulation electrodes at the position stably for a prolonged period of time. Additionally, another object of the invention is to provide an electric stimulator which can easily remove an electric stimulator placed in a body cavity.

### Solution to Problem

In order to solve the above problem, the electric stimulator of the invention is a tubular electric stimulator implanted in a living body.
This electric stimulator includes stimulation electrodes that stimulate nerves or muscles in a living body and a stimulation circuit block having a stimulation circuit that is electrically connected to the stimulation electrodes to apply a stimulation signal to the stimulation electrodes, Additionally, the electric stimulator further includes a support that is connected to the stimulation circuit block to hold the stimulation electrodes at an implantation position in the living body. The stimulation circuit block and the support, except for at least one end of the support, have a shape capable of being inserted into a duct of a tubular insertion tool.

According to the above-described configuration of the invention, portions of the stimulation circuit block and the support have a shape capable of being inserted into the duct of the tubular insertion tool. Therefore, a doctor can operate the support from the outside of the body to move the stimulation electrodes to a predetermined position via the tubular insertion tool. Additionally, since the support is connected to the stimulation circuit block, the placing of the stimulation circuit block at the position can be continued by this support. At this time, the electric stimulator can also be removed by pulling the support.

### Advantageous Effects of Invention

According to the invention, a doctor can operate the support to move the stimulation electrodes to a predetermined position via the tubular insertion tool, such as a tubular needle or a cannula, to place the stimulation electrodes. Thereby, there are effects that it becomes easy to move the stimulation electrodes to nerves or muscles to be stimulated, and the stimulation electrodes can be placed at the position stably for a prolonged period of time. Additionally, there is an effect that a doctor can operate the support to easily remove the electric stimulator out of the body.

### Brief Description of the Drawings

Fig. 1 is a perspective view showing an overall electric stimulator related to a first embodiment of the invention.
Figs. 2A to 2C are exploded external views showing the overall electric stimulator related to the first embodiment of the invention.
Fig. 3A is an enlarged view showing the electric stimulator related to the first embodiment of the invention, and Fig. 3B is an axial cross-sectional view of the electric stimulator.
Figs. 4A to 4F are radial cross-sectional views of the electric stimulator related to the first embodiment of the invention.
Fig. 5 is a block diagram centering on a stimulation circuit related to the first embodiment of the invention.
Fig. 6 is an explanatory view for describing a procedure that implants the electric stimulator related to the first embodiment of the invention into the living body.
Fig. 7 is an explanatory view for describing the procedure subsequent to Fig. 6 that implants the electric stimulator related to the first embodiment of the invention into the living body.
Fig. 8 is an explanatory view for describing the procedure subsequent to Fig. 7 that implants the electric stimulator related to the first embodiment of the invention into the living body.
Fig. 9 is an explanatory view for describing the procedure subsequent to Fig. 8 that implants the electric stimulator related to the first embodiment of the invention into the living body.
Fig. 10 is an explanatory view for describing the procedure subsequent to Fig. 9 that implants the electric stimulator related to the first embodiment of the invention into the living body.
Fig. 11 is a perspective view showing an overall electric stimulator related to a second embodiment of the invention.
Figs. 12A to 12C are exploded external views showing the overall electric stimulator related to the second embodiment of the invention.
Fig. 13A is an enlarged view showing the electric stimulator related to the second embodiment of the invention, and Fig. 13B is an axial cross-sectional view of the electric stimulator.
Figs. 14A to 14F are radial cross-sectional views of the electric stimulator related to the second embodiment of the invention.
Fig. 15 is a perspective view showing an overall electric stimulator related to a third embodiment of the invention.
Figs. 16A to 16C are exploded external views showing the overall electric stimulator related to the third embodiment of the invention.
Fig. 17A is an enlarged view showing the electric stimulator related to the third embodiment of the invention, and Fig. 17B is an axial cross-sectional view of the electric stimulator.
Fig. 18 is a perspective view showing an overall electric stimulator related to a fourth embodiment of the invention.
Figs. 19A to 19C are exploded external views showing the overall electric stimulator related to the fourth embodiment of the invention.
Fig. 20A is an enlarged view showing the electric stimulator related to the fourth embodiment of the invention, and Fig. 20B is an axial cross-sectional view of the electric stimulator.
Figs. 21A to 21F are radial cross-sectional views of the electric stimulator related to the fourth embodiment of the invention.
Fig. 22 is a perspective view showing an overall electric stimulator related to a fifth embodiment of the invention.
Figs. 23A to 23C are exploded external views showing the overall electric stimulator related to the fifth embodiment of the invention.
Fig. 24A is an enlarged view showing the electric stimulator related to the fifth embodiment of the invention, and Fig. 24B is an axial cross-sectional view of the electric stimulator.
Figs. 25A to 25F are radial cross-sectional views of the electric stimulator related to the fifth embodiment of the invention.
Fig. 26 is a perspective view showing an overall electric stimulator related to a sixth embodiment of the invention.
Figs. 27A to 27C are exploded external views showing the overall electric stimulator related to the sixth embodiment of the invention.
Fig. 28A is an explanatory view showing a first modification of a support related to the fourth embodiment of the invention, and Fig. 28B is an axial cross-sectional view of the support.
Fig. 29A is an explanatory view showing a first modification of a support related to the fifth or sixth embodiment of the invention, and Fig. 29B is an axial cross-sectional view of the support.
Fig. 30A is an explanatory view showing a second modification of the support related to the fourth embodiment of the invention, and Fig. 30B is an axial cross-sectional view of the support.
Fig. 31A is an explanatory view showing a second modification of the support related to the fifth or sixth embodiment of the invention, and Fig. 31B is an axial cross-sectional view of the support.
Fig. 32 is a perspective view showing an overall electric stimulator related to a seventh embodiment of the invention.
Figs. 33A to 33D are exploded external views showing the overall electric stimulator related to the seventh embodiment of the invention.
Fig. 34A is an enlarged view showing the electric stimulator related to the seventh embodiment of the invention, and Fig. 34B is an axial cross-sectional view of the electric stimulator.
Figs. 35A to 35F are radial cross-sectional views of the electric stimulator related to the seventh embodiment of the invention.
Fig. 36 is a perspective view showing an overall electric stimulator related to an eighth embodiment of the invention.
Figs. 37A to 37D are exploded external views showing the overall electric stimulator related to the eighth embodiment of the invention.
Fig. 38A is an enlarged view showing the electric stimulator related to the eighth embodiment of the invention, and Fig. 38B is an axial cross-sectional view of the electric stimulator.
Figs. 39A to 39F are radial cross-sectional views of the electric stimulator related to the eighth embodiment of the invention.
Fig. 40 is a perspective view showing an overall electric stimulator related to a ninth embodiment of the invention.
Figs. 41A to 41D are exploded external views showing the overall electric stimulator related to the ninth embodiment of the invention.
Fig. 42A is an enlarged view showing the electric stimulator related to the ninth embodiment of the invention, and Fig. 42B is an axial cross-sectional view of the electric stimulator.
Figs. 43A to 43D are explanatory views showing modifications of the electric stimulators related to the seventh to ninth embodiment of the invention.
Fig. 44 is a perspective view showing an overall electric stimulator related to a tenth embodiment of the invention.
Figs. 45A to 45D are exploded external views showing the overall electric stimulator related to the tenth embodiment of the invention.
Fig. 46A is an enlarged view showing the electric stimulator related to the tenth embodiment of the invention, and Fig. 46B is an axial cross-sectional view of the electric stimulator related to the tenth embodiment of the invention.
Figs. 47A to 47F are radial cross-sectional views of the electric stimulator related to the tenth embodiment of the invention.
Fig. 48 is a perspective view showing an overall electric stimulator related to an eleventh embodiment of the invention.
Figs. 49A to 49D are exploded external views showing the overall electric stimulator related to the eleventh embodiment of the invention.
Fig. 50A is an enlarged view showing the electric stimulator related to the eleventh embodiment of the invention, and Fig. 50B is an axial cross-sectional view of the electric stimulator related to the eleventh embodiment of the invention.
Figs. 51A to 51F are radial cross-sectional views of the electric stimulator related to the eleventh embodiment of the invention.
Fig. 52 is a perspective view showing an overall electric stimulator related to a twelfth embodiment of the invention.
Figs. 53A to 53D are exploded external views showing the overall electric stimulator related to the twelfth embodiment of the invention.
Fig. 54A is an enlarged view showing the electric stimulator related to the twelfth embodiment of the invention, and Fig. 54B is an axial cross-sectional view of the electric stimulator.

### Description of Embodiments

Hereinafter, embodiments for carrying out the invention will be described. The embodiments to be described below are preferred specific examples of the invention. Therefore, various technically preferable limitations are given. However, the scope of the invention is not limited to these embodiments so as long as there is no description that limits the invention in the following description. For example, the numerical conditions of respective parameters mentioned in the following description are merely preferred examples, and dimensions, shapes, and arrangement relations of the respective drawings used for description are also substantial or approximate.

Description will be performed according to the following procedure.

### <First embodiment>

(1) Configuration of electric stimulator
(2) Configuration of stimulation circuit or the like
(3) Implantation procedure of electric stimulator

### <Second embodiment>

(4) Configuration of electric stimulator

### <Third Embodiment>

(5) Configuration of electric stimulator

### <Fourth embodiment>

(6) Configuration of electric stimulator

### <Fifth embodiment>

(7) Configuration of electric stimulator

### <Sixth embodiment>

(8) Configuration of electric stimulator

### <Seventh embodiment>

(9) Configuration of electric stimulator

### <Eighth embodiment>

(10) Configuration of electric stimulator

### <Ninth embodiment>

(11) Configuration of electric stimulator

### <Tenth embodiment>

(12) Configuration of electric stimulator

### <Eleventh embodiment>

(13) Configuration of electric stimulator

### <Twelfth embodiment>

(14) Configuration of electric stimulator

### <Modifications>

### <Description of first embodiment of the invention>

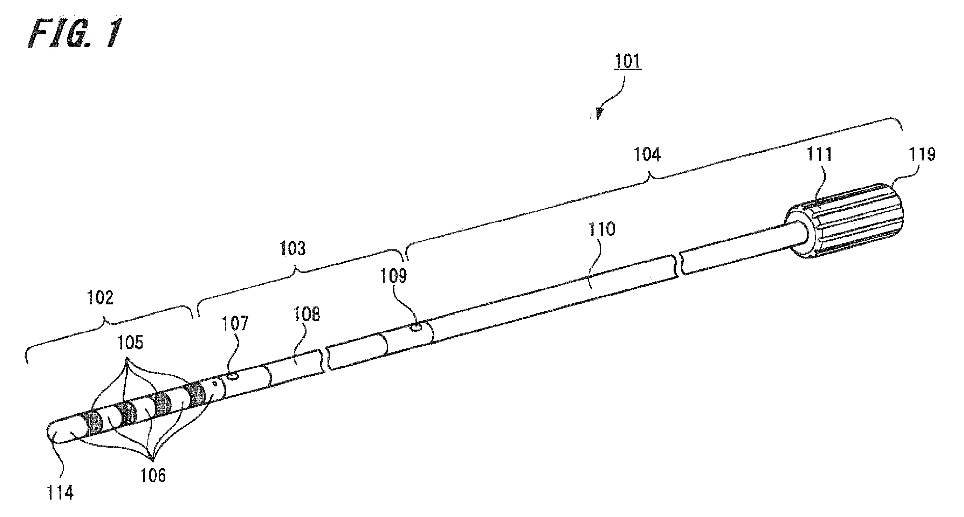

A first embodiment of the invention will be described with reference to Figs. 1 to 10.

### [1. Configuration of electric stimulator]

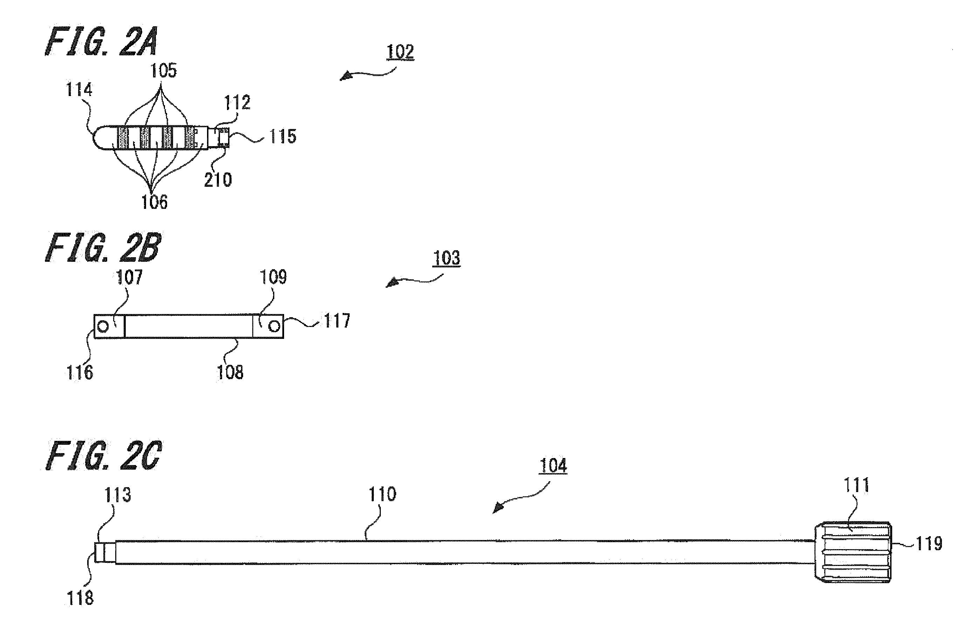

First, the general configuration of an electric stimulator related to the first embodiment will be described with reference to Fig. 1 and Figs. 2A to 2C.
Fig. 1 is a perspective view showing an overall electric stimulator related to the first embodiment of the invention.
Figs. 2A to 2C are exploded external views when the electric stimulator shown in Fig. 1 is seen from the top face.

An electric stimulator 101 is formed in the shape of a rod, generates an electric stimulation signal, and stimulates nerves or the like in a living body by the stimulation signal. When the electric stimulator 101 stimulates the nerves of the spinal cord, the electric stimulator 101 is implanted in the living body (for example, an epidural space where the distance between the spinal dura mater and the ligamentum flavum is about 5 mm). Therefore, as for the electric stimulator 101, the diameter from a distal end 114 to a predetermined portion of a support 104 (that will be described below) is preferably about 1 mm to 3 mm.

The electric stimulator 101 is mainly made up of an electrode block 102, a circuit block 103, and the support 104. In addition, the electrode block 102 and the circuit block 103 are made attachable and detachable by a connector part 107, and the circuit block 103 and the support 104 are made attachable and detachable by a connector part 109. More specifically, as shown in Figs. 2A to 2C, as for the electrode block 102 and the circuit block 103, a connector part 112 on the electrode block 102 side and the connector part 107 on the circuit block 103 side are fixed together, for example, with a screw or the like. Similarly, as for the circuit block 103 and the support 104, the connector part 109 on the circuit block 103 side and a connector part 113 on the support 104 side are fixed together with a screw or the like.

As for the electrode block 102, the distal end 114 is formed in a substantially hemispherical shape, and the other portions are formed in a substantially cylindrical shape. The radius of the substantially hemispherical portion of the distal end 114 is preferably about 0.5 mm to 1.5 mm, and the diameter of the other substantially cylindrical portions is preferably about 1 mm to 3 mm.

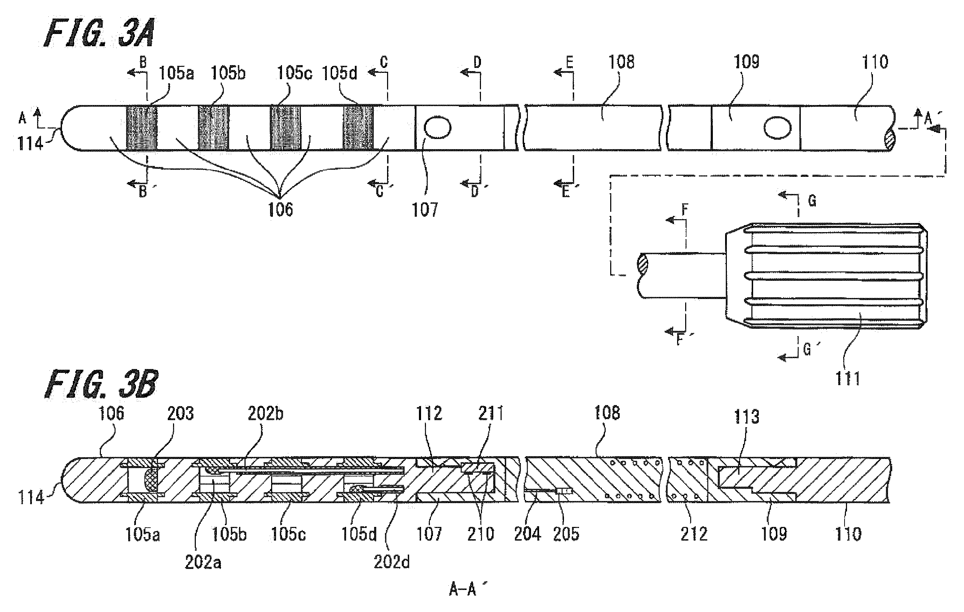

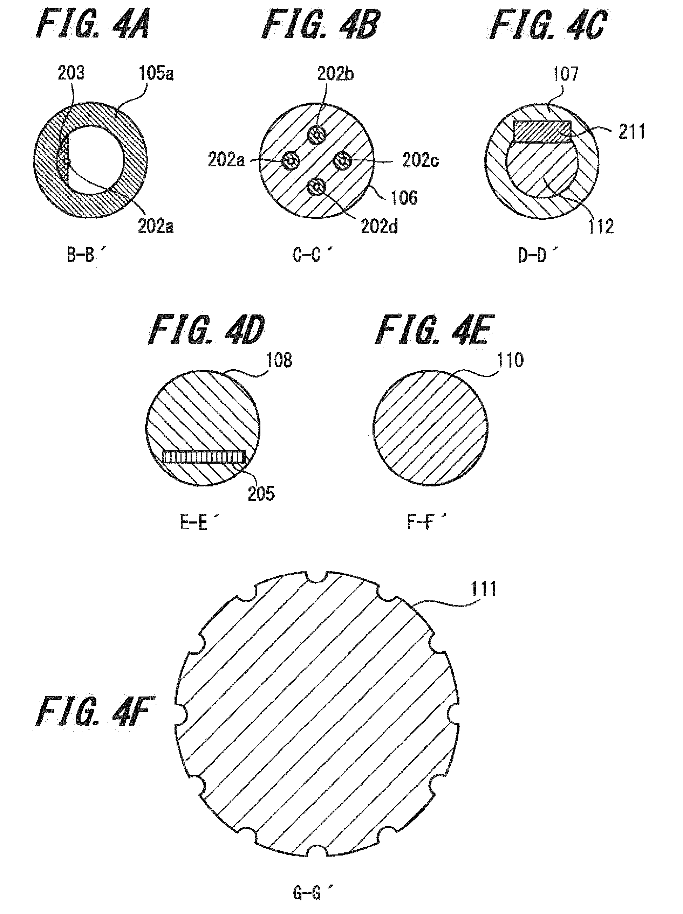

Such an electrode block 102 includes four stimulation electrodes 105 for stimulating nerves or the like, and a body 106 that is arranged at equal intervals such that, when the electric stimulator 101 is implanted in a living body, the respective stimulation electrodes 105 are exposed to the living body. Moreover, the connector part 112 that connects a proximal end 115 side of the body 106 and a distal end 116 of the circuit block 103 so as to be continuous is included. In addition, although the number of the stimulation electrodes 105 is four in the first embodiment, this is merely an example, and the number of the stimulation electrodes 105 can be set arbitrarily. The internal configuration or the like of the electrode block 102 will be described below in Figs. 3A and 3B and Figs. 4A to 4F.

The circuit block 103 is formed in a substantially cylindrical shape with the same diameter as the electrode block 102. The circuit block 103 includes the connector part 107 that is connected to the connector part 112 of the electrode block 102 such that the distal end 116 is continuous with the proximal end 115 side of the body 106 of the electrode block 102. Additionally, the circuit block 103 is provided with a body 108 that is continuous with the connector part 107. Moreover, the circuit block 103 includes the connector part 109 that is continuous with a proximal end 117 of the body 108 and connects the proximal end 117 and the support 104. In addition, the internal configuration or the like of the circuit block 103 will be described below in Figs. 3A and 3B and Figs. 4A to 4F.

The support 104 includes the connector part 113 that is connected to the circuit block 103, a body 110 that is formed in a substantially cylindrical shape with the same diameter as the electrode block 102, and a substantially cylindrical holder part 111 that has a larger diameter than the body 110.
The connector part 113 of the support 104 is connected to the connector part 109 of the circuit block 103 such that a distal end 118 side of the body 110 is continuous with the circuit block 103. The body 110 is a portion that connects the connector part 113 and the holder part 111 arranged on a proximal end 119 side. In addition, the holder part 111 is a place that is held when a doctor inserts the electric stimulator 101 into a living body. In addition, the internal configuration of the support 104 will be described below in Figs. 3A and 3B and Figs. 4A to 4F.

Next, the internal configuration of the electric stimulator 101 will be described with reference to Figs. 3A and 3B and Figs. 4A to 4F.
Figs. 3A and 3B are enlarged views showing the electric stimulator related to the first embodiment of the invention, and its axial internal structure.
Fig. 3A is an enlarged external view when the electric stimulator shown in Fig. 1 is seen from the top face.
Fig. 3B is a cross-sectional view showing an A-A' section of the electric stimulator shown in Fig. 3A.

Additionally, Figs. 4A to 4F are cross-sectional views showing the internal structure of a predetermined location in a vertical direction with respect to the axis of the electric stimulator related to the first embodiment of the invention.
Fig. 4A is a cross-sectional view showing a B-B' section of the electric stimulator shown in Fig. 3A.
Fig. 4B is a cross-sectional view showing a C-C' section of the electric stimulator shown in Fig. 3A.
Fig. 4C is a cross-sectional view showing a D-D' section of the electric stimulator shown in Fig. 3A.
Fig. 4D is a cross-sectional view showing an E-E' section of the electric stimulator shown in Fig. 3A.
Fig. 4E is a cross-sectional view showing an F-F' section of the electric stimulator shown in Fig. 3A.
Fig. 4F is a cross-sectional view showing a G-G' section of the electric stimulator shown in Fig. 3A.

First, the internal configuration of the electrode block 102 will be described.
The body 106 is made of resin materials that have pliability and biocompatibility, for example, materials, such as silicone or polyurethane. The distal end 114 of the body 106 is substantially hemispherical as described above, and the radius thereof is preferably within a range of about 0.5 mm to 1.5 mm. Portions other than the distal end 114 of the body 106 are formed in a substantially cylindrical shape of which a portion is hollow in the axial direction. The four stimulation electrodes 105 are fixed to this hollow portion so as to be exposed to the surface of the body 106.

The stimulation electrodes 105 are made of materials that have conductivity and biocompatibility, for example, materials, such as platinum or platinum alloys (platinum 90%/iridium 10% alloy or the like), and are formed in a substantially hollow cylindrical shape. The external diameter of the stimulation electrodes 105 is made approximately equal to the external diameter of the body 106. In addition, the four stimulation electrodes 105 are defined as stimulation electrodes 105a, 105b, 105c, and 105d sequentially from the stimulation electrode on the distal end 114 side.

One end (end on the distal end 114 side) of each of conducting wires 202a to 202d are bonded to the stimulation electrodes 105a to 105d, respectively (refer to Fig. 4A) with solder 203, and the other end (end on the proximal end 115 side) of each of the conducting wires 202a to 202d are electrically connected to the connector part 112. In addition, locations other than the locations of the conducting wires 202 that are bonded with the solder 203 and the locations of the conducting wires 202 that are electrically connected to the connector part 112 are insulated and coated with PTFE (polytetrafluoroethylene) or ETFE (ethylenetetrafluoroethylene) and are completely embedded in the body 106 (refer to Fig. 4B).

The connector part 112 is formed of the same material as the body 106, and is formed as a cutout part in which a level difference is provided from the external diameter of the substantially cylindrical body 106. This cutout part is formed by a predetermined distance in the axial direction from the proximal end 115 (refer to Fig. 2A). In addition, this cutout part is planar, four connector pins 210 (refer to Fig. 2A) are arranged on the cutout part so as to be exposed, and the conducting wires 202a to 202d are electrically connected to the four connector pins 210, respectively. For this reason, the four connector pins 210 are also electrically connected to the stimulation electrodes 105a to 105d, respectively.

Next, the internal configuration of the circuit block 103 (refer to Fig. 2B) will be described.
The connector part 107 is made of the same material (polyurethane or silicone) as the body 106 except for an electrical connecting portion 211 that will be described below. A hole of almost the same shape as the external diameter of the connector part 112 is opened in the axial direction in this connector part 107 so that the connector part 107 can be connected to the connector part 112 of the electrode block 102 (refer to Fig. 4C). The external diameter of this connector part 107 is approximately equal to the external diameter of the body 106. Additionally, the connector part 107 includes the electrical connecting portion 211 that is independently and electrically connected to the four connector pins 210, respectively, when this connector part 107 is connected to the connector part 112 of the electrode block 102. In addition, although the connector part 109 provided on the proximal end 117 (refer to Fig. 2B) side of the circuit block 103 has the same shape as the connector part 107 provided on the distal end 116 side, something like the electrical connecting portion 211 is not provided.

The body 108 is continuous with the connector parts 107 and 109 and is made of the same material as the connector parts 107 and 109. The body 108 is formed in a substantially cylindrical shape, and is formed such that the diameter thereof is approximately equal to the external diameter of the connector part 107 of the circuit block 103.

A stimulation circuit 205 that is formed by mounting small parts, such as custom ICs, on a flexible circuit board, and that generates an electric stimulation signal, and a coil portion 212 that is electrically connected to the stimulation circuit 205 is embedded in the body 108. In addition, the coil portion 212 is formed so as to be wound with the axial direction as an axis.

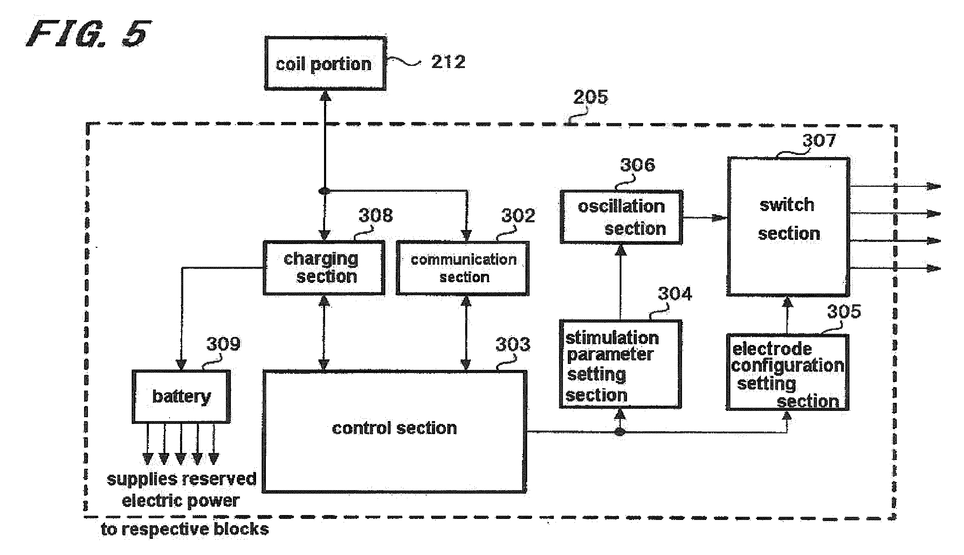

The stimulation circuit 205 is connected to the electrical connecting portion 211 via conducting wires 204 embedded in the body 108 such that the generated electric stimulation signal is independently supplied to the respective stimulation electrodes 105. In addition, the electric configuration of the stimulation circuit 205 and the coil portion 212 will be described below in Fig. 5.

Next, the internal configuration of the support 104 will be described (refer to Fig. 2C).
The connector part 113 is made of, for example, polyurethane or silicone and is formed in the same shape as the connector part 112 of the electrode block 102 described above. It is noted that means for performing electric connection with the outside, such as the connector pins 210, may not be provided, differently from the connector part 112.

The body 110 is made of the same material as the connector part 113, and is formed in a substantially cylindrical shape. The body 110 is formed such that the diameter thereof is approximately equal to the external diameter of the body 108 of the circuit block 103.

The holder part 111 is made of materials, such as a plastics, and is formed in a substantially cylindrical shape. Since the holder part 111 is a part that is held when the electric stimulator 101 is inserted into the inside of a body, the external diameter of the holder part 111 is preferably two times to three times or more the external diameter of the body 110.

### [2. Configuration of stimulation circuit or the like]

Next, a more detailed circuit configuration of the stimulation circuit 205 and the coil portion 212 included in the circuit block 103 will be described with reference to Fig. 5.
Fig. 5 is a functional block diagram showing the stimulation circuit and the coil portion related to the first embodiment of the invention.

The stimulation circuit 205 includes a communication section 302, a stimulation parameter setting section 304, an electrode configuration setting section 305, an oscillation section 306, and a control section 303. Moreover, the stimulation circuit 205 includes a charging section 308, a rechargeable battery 309, and a switch section 307.

The rechargeable battery 309 is, for example, a rechargeable battery, such as a lithium-ion battery. Although not shown in Fig. 5, the rechargeable battery 309 supplies reserved electric power to respective blocks that constitute the stimulation circuit 205.

The coil portion 212 is, for example, a resonant circuit constituted by a coil and a capacitor. The coil portion 212 receives electromagnetic waves for charging that are transmitted from a controller (not shown) outside a body, when charging the rechargeable battery 309. An alternating current that is generated from the coil portion 212 with this reception is output to the charging section 308. Additionally, the coil portion 212 receives the electromagnetic waves that are transmitted from the controller (not shown) outside a body and have predetermined information carried thereon, and the received electromagnetic waves are output to the communication section 302 from the coil portion 212 concerned.

The charging section 308 has a rectifier circuit built therein, and converts the alternating current output from the coil portion 212 into a direct current to acquire electric power. The rechargeable battery 309 is charged with the acquired electric power.

The communication section 302 demodulates the electromagnetic waves received by the coil portion 212, and retrieves the information carried on the electromagnetic waves. Then, the retrieved information is output to the stimulation parameter setting section 304 and the electrode configuration setting section 305 via the control section 303. The information output to the stimulation parameter setting section 304 is information (hereinafter referred to as a "stimulation parameter") on the stimulation intensity of an electric stimulation signal, and the information output to the electrode configuration setting section 305 is information (hereinafter referred to as "electrode configuration information") on the electrode configuration. Since the stimulation intensity of the electric stimulation signal is determined depending on the pulse voltage, pulse current, pulse width, or the frequency of the electric stimulation signal, the stimulation parameter is a signal indicating the value of the pulse voltage or the like. Additionally, the electrode configuration information is a signal including information for changing the polarity of an electric stimulation signal, and information for making the switch section 307 select a stimulation electrode 105 that outputs the electric stimulation signal.

The stimulation parameter setting section 304 generates a stimulation intensity change signal for changing the stimulation intensity of the electric stimulation signal generated in the oscillation section 306, on the basis of the stimulation parameter input from the communication section 302.
The electrode configuration setting section 305 generates an electrode configuration selection signal for selecting the stimulation electrode 105 that outputs the electric stimulation signal generated in the oscillation section 306, on the basis of the electrode configuration information input from the communication section 302. In addition, the stimulation intensity change signal output from the stimulation parameter setting section 304 is output to the oscillation section 306, and the electrode configuration selection signal output from the electrode configuration setting section 305 is output to the switch section 307.

The oscillation section 306 generates an electric stimulation signal and outputs the signal to the switch section 307, on the basis of the stimulation intensity change signal input from the stimulation parameter setting section 304.
The switch section 307 determines a stimulation electrode 105 that outputs the electric stimulation signal input from the oscillation section 306, on the basis of the electrode configuration selection signal input from the electrode configuration setting section 305. In addition, the control section 303 is, for example, a microcomputer or the like, and controls the respective blocks of the stimulation circuit 205.

### [3. Implantation procedure of electric stimulator]

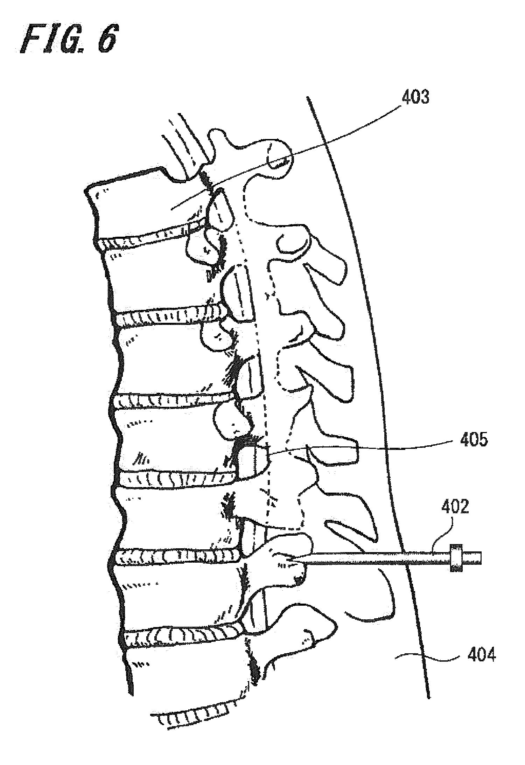

Next, an example of a procedure of implanting the electric stimulator 101 into an epidural space, and performing an electric stimulation of the nerves of the spinal cord with the electric stimulator 101 will be described with reference to Figs. 6 to 10.
Figs. 6 to 10 are longitudinal cross-sectional views of a human body showing the vicinity of the back.

First, a doctor determines a target stimulation site of the spinal cord in advance, on the basis of the pattern of pain distribution in a patient. Then, an insertion is carried out from a patient's back side under X-ray illumination, and an epidural needle 402 serving as a tubular insertion tool is inserted into an epidural space 405. As a position where the epidural needle 402 is inserted into the epidural space 405, generally, a position that is lower than a target stimulation site by three vertebral levels or more is selected (refer to Fig. 6).

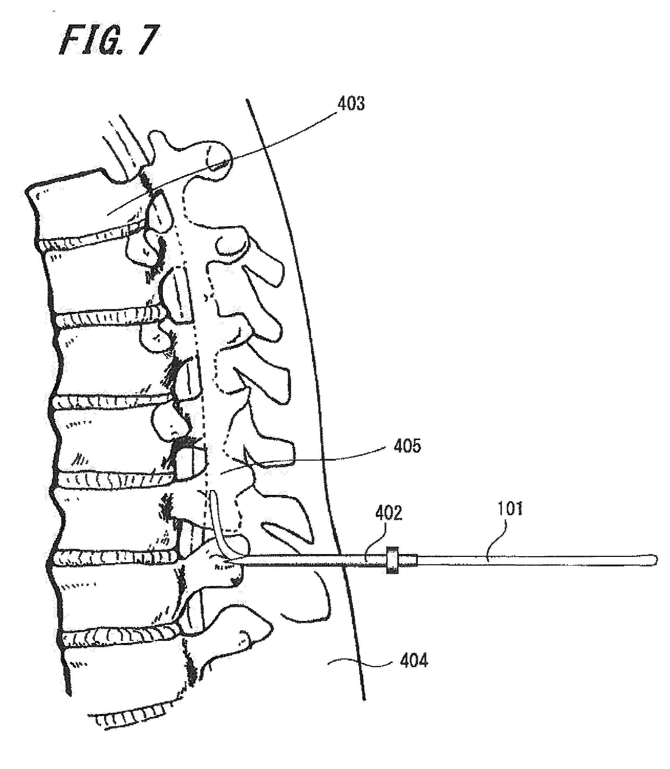

Next, the doctor passes the distal end 114 (refer to Fig. 1) of the electric stimulator 101 through the epidural needle 402, and inserts the electric stimulator 101 into a living body 404. Then, the electric stimulator 101 is inserted into the epidural space 405 through the epidural needle 402 by pushing the holder part 111 in the axial direction (refer to Fig. 7; the holder part 111 of the electric stimulator 101 is not shown).

Subsequently, the doctor pushes the holder part 111 further in the axial direction, moves the electric stimulator 101 upward in the epidural space 405, and locates the stimulation electrodes 105 of the electric stimulator 101 near the stimulation site.
Next, the doctor operates the controller (not shown) outside of the body to perform nerve stimulation, moving the position of the stimulation electrodes 105 little by little. At this time, in the stimulation circuit 205 of the electric stimulator 101, an electric stimulation signal with predetermined intensity is generated on the basis of the doctor's operation, the generated electric stimulation signal is output to the stimulation electrodes 105, and nerve stimulation of a portion near the position of the stimulation electrodes 105 is performed. Then, the doctor determines an optimal position of the stimulation electrodes 105, hearing the reaction to the nerve stimulation of the patient.

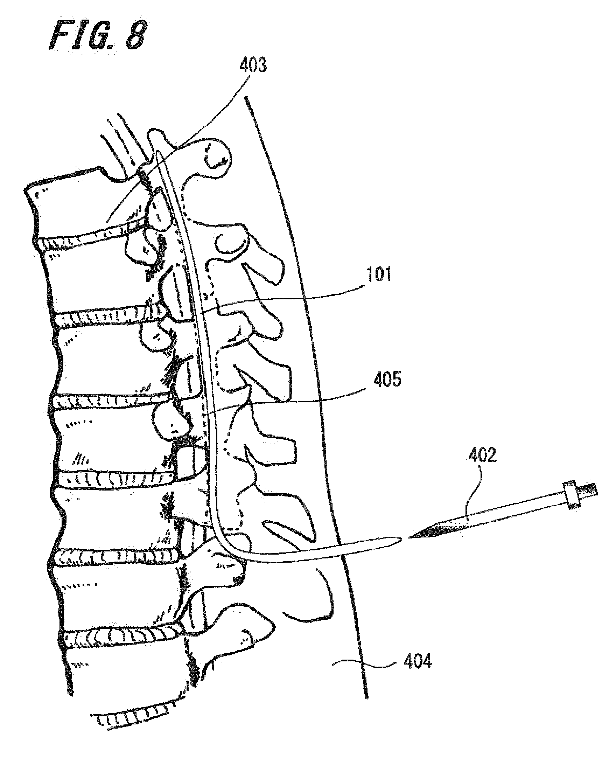

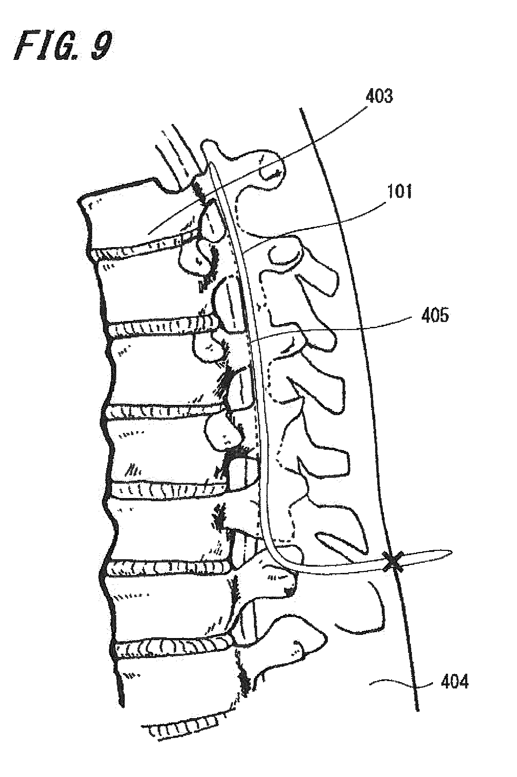

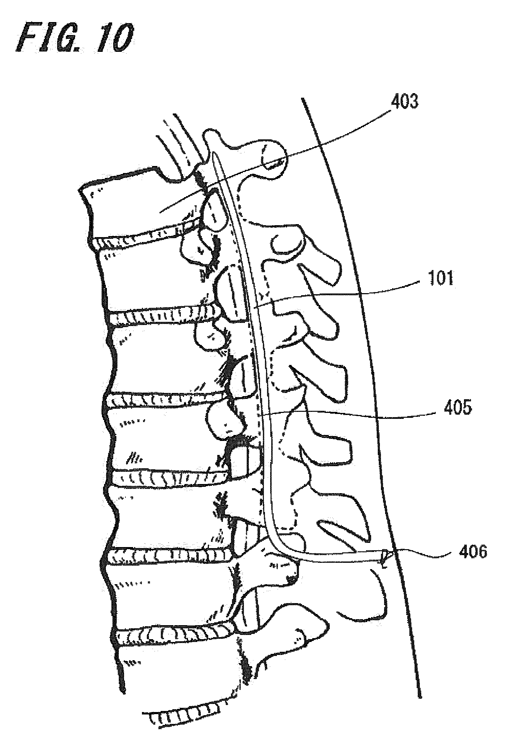

Subsequently, the doctor removes the epidural needle 402 from the living body 404 after cutting the holder part 111 of the electric stimulator 101 so that the electric stimulator 101 is wholly implanted in the living body 404 (refer to Fig. 8). Then, finally, after the portion (equivalent to a portion of the support 104) of the electric stimulator 101 that sticks out from the living body 404 is cut (refer to Fig. 9), the cut portion and the living body 404 are sewn with thread 406 so that the cut electric stimulator 101 is fixed in the state of being wholly implanted in the living body 404 (refer to Fig. 10). This treatment is performed so as not to develop an infection or the like from an insertion opening of the electric stimulator 101.
Meanwhile, it is also possible insert the electric stimulator 101 into the epidural space 405 through a cannula instead of the epidural needle 402. After the cannula is led to near a location where stimulation is performed through the epidural needle in advance and the epidural needle is removed, an electric stimulator is passed through this cannula and inserted into a living body. Here, the cannula refers to a tube that is made of resin materials that have pliability and biocompatibility, for example, materials, such as silicone or polyurethane.

As described above, in the first embodiment of the invention, the electrode block, the circuit block, and a portion of the support are formed in a shape such that these can be inserted through the inside of the epidural needle. Therefore, there are effects that a doctor can operate the support to move the stimulation electrodes to a predetermined position via the epidural needle, and the stimulation electrodes are stably placed at the position for a prolonged period of time. Additionally, there is an effect that a doctor can operate the support to remove the electric stimulator easily out of the body.

### <Description of second embodiment of the invention>

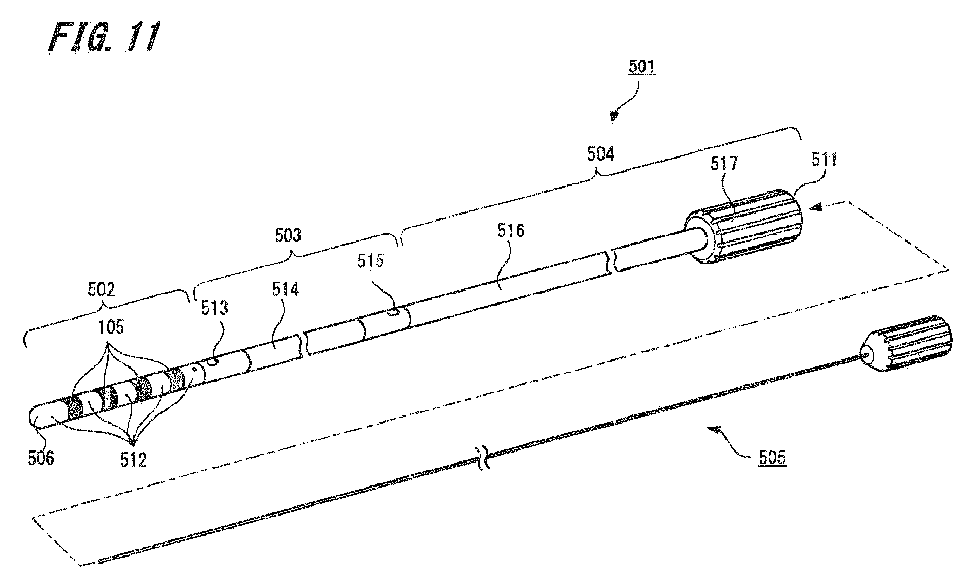

Next, a second embodiment of the invention will be described with reference to Figs. 11 to 14. An electric stimulator 501 related to the second embodiment shown in Figs. 11 to 14 includes a cylindrical hole (hereinafter referred to as a ""lumen for a stylet") for inserting a stylet 505 into the electric stimulator 101 related to the first embodiment. Common portions of the configuration will be designated by the same reference numerals, and the description thereof will be omitted.

### [4. Configuration of electric stimulator]

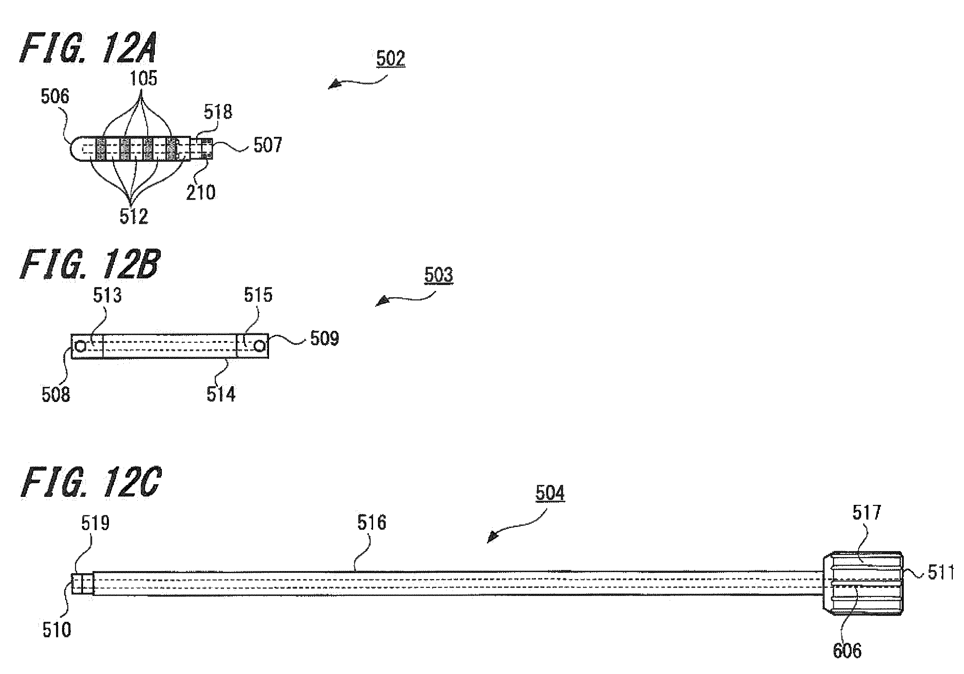

First, the general configuration of the electric stimulator related to the second embodiment will be described with reference to Fig. 11 and Figs. 12A to 12C.
Fig. 11 is a perspective view showing an overall electric stimulator related to the second embodiment of the invention.
Figs. 12A to 12C are exploded external views when the electric stimulator shown in Fig. 11 is seen from the top face.

Similarly to the electric stimulator 101, the electric stimulator 501 is formed in the shape of a rod, generates an electric stimulation signal, and stimulates nerves or the like in a living body by the stimulation signal. When the electric stimulator 501 stimulates the nerves of the spinal cord, the electric stimulator 501 is implanted in the living body (for example, an epidural space where the distance between the spinal dura mater and the ligamentum flavum is about 5 mm). Therefore, as for the electric stimulator 501, it is preferable that the diameter from a distal end 506 to a predetermined portion of a support 504 (that will be described below) is about 1 mm to 3 mm.

The electric stimulator 501 is mainly made up of an electrode block 502, a circuit block 503, and the support 504. In addition, the electrode block 502 and the circuit block 503 are made attachable and detachable by a connector part 513, and the circuit block 503 and the support 504 are made attachable and detachable by a connector part 515. More specifically, as shown in Figs. 12A to 12C, as for the electrode block 502 and the circuit block 503, a connector part 518 on the electrode block 502 side and the connector part 513 on the circuit block 503 side are fixed together, for example, with a screw or the like. Similarly, as for the circuit block 503 and the support 504, the connector part 515 on the circuit block 503 side and a connector part 519 on the support 504 side are fixed together with a screw or the like. When the electrode block 502, the circuit block 503, and the support 504 are connected together, these respective blocks have the lumen for a stylet that communicates with the axial direction of the blocks. It is noted that the lumen for a stylet opens to a proximal end 511, and is provided up to the vicinity of the distal end 506. In addition, the diameter of the lumen for a stylet is preferably approximately equal to or slightly larger than the diameter of the stylet 505.

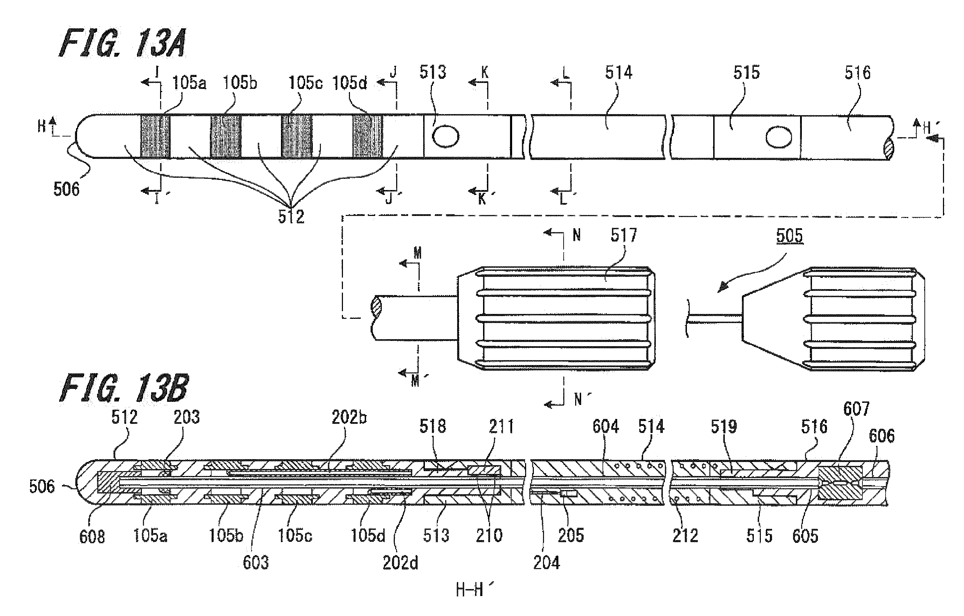

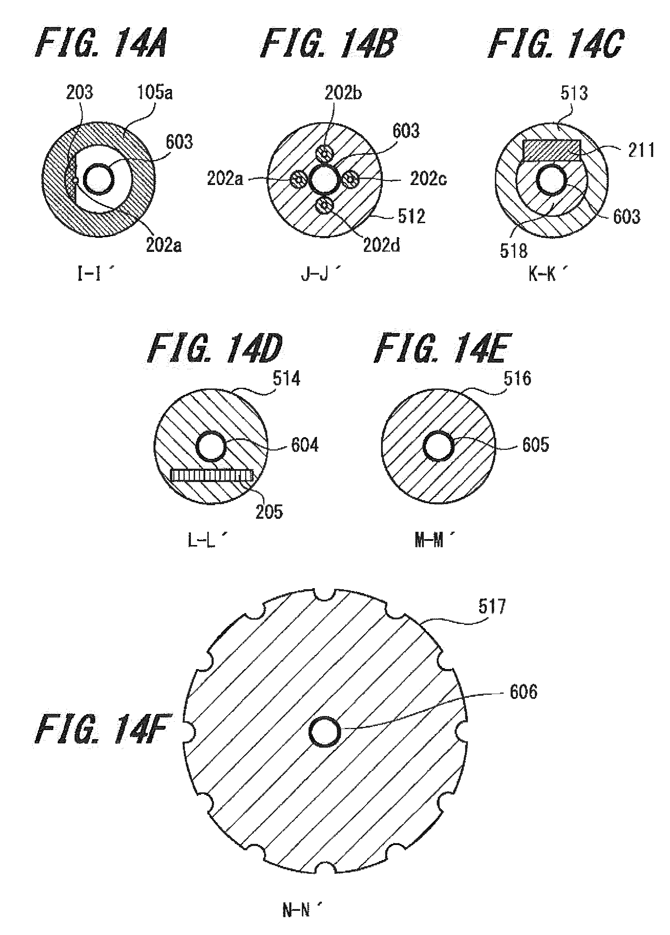

As for the electrode block 502, the distal end 506 is formed in a substantially hemispherical shape, and the other portions are formed in a substantially cylindrical shape. The radius of the substantially hemispherical portion of the distal end 506 is preferably about 0.5 mm to 1.5 mm, and the diameter of the other substantially cylindrical portions is preferably about 1 mm to 3 mm.
Such an electrode block 502 includes the four stimulation electrodes 105 for stimulating nerves or the like, and a body 512 that is arranged at equal intervals such that, when the electric stimulator 501 is arranged in a living body, the respective stimulation electrodes 105 are exposed to the living body. Moreover, the connector part 518 that connects a proximal end 507 side of the body 512 and a distal end 508 of the circuit block 503 so as to become continuous is included. In addition, the internal configuration or the like of the electrode block 502 will be described below in Figs. 13A and 13B and Figs. 14A to 14F.

The circuit block 503 is formed in a substantially cylindrical shape with the same diameter as the electrode block 502. The circuit block 503 includes the connector part 513 that is connected to the connector part 518 of the electrode block 502 such that the distal end 508 is continuous with the proximal end 507 side of the body 512 of the electrode block 502. Additionally, the circuit block 503 is provided with a body 514 that is continuous with the connector part 513. Moreover, the circuit block 503 includes the connector part 515 that is continuous with a proximal end 509 side of the body 514 and connects the proximal end 509 and the support 504. In addition, the internal configuration or the like of the circuit block will be described below in Figs. 13A and 13B and Figs. 14A to 14F.

The support 504 includes the connector part 519 that is connected to the circuit block 503, a body 516 that is formed in a substantially cylindrical shape with the same diameter as the electrode block 502, and a substantially cylindrical holder part 517 that has a larger diameter than the body 516.
The connector part 519 of the support 504 is connected to the connector part 515 of the circuit block 503 such that a distal end 510 side of the body 516 is continuous with the circuit block 503. The body 516 is a portion that connects the connector part 519 and the holder part 517 arranged on the proximal end 511 side. In addition, the holder part 517 is a place that is held when a doctor inserts the electric stimulator 501 into a living body. In addition, the internal configuration or the like of the support 504 will be described below in Figs. 13A and 13B and Figs. 14A to 14F.

Next, the internal configuration of the electric stimulator 501 related to the second embodiment will be described with reference to Figs. 13A and 13B and Figs. 14A to 14F.
Figs. 13A and 13B are enlarged views showing the electric stimulator related to the second embodiment of the invention, and its axial internal structure.
Fig. 13A is an enlarged external view when the electric stimulator shown in Fig. 11 is seen from the top face.
Fig. 13B is a cross-sectional view showing an H-H' section of the electric stimulator shown in Fig. 13A.

Additionally, Figs. 14A to 14F are cross-sectional views showing the internal structure of a predetermined location in a vertical direction with respect to the axis of the electric stimulator related to the second embodiment of the invention.
Figs. 14A is a cross-sectional views showing an I-I' section of the electric stimulator shown in Fig. 13A.
Fig. 14B is a cross-sectional view showing a J-J' section of the electric stimulator shown in Fig. 13A.
Fig. 14C is a cross-sectional view showing a K-K' section of the electric stimulator shown in Fig. 13A.
Fig. 14D is a cross-sectional view showing an L-L' section of the electric stimulator shown in Fig. 13A.
Fig. 14E is a cross-sectional view showing an M-M' section of the electric stimulator shown in Fig. 13A.
Fig. 14F is a cross-sectional view showing an N-N' section of the electric stimulator shown in Fig. 13A.

First, the internal configuration of the electrode block 502 will be described.
A pipe 603 is made of materials that have biocompatibility, insulation, and pliability, for example, PTFE or ETFE, and is formed in a hollow, substantially cylindrical shape. It is desirable that the external diameter of the pipe 603 be about 0.1 to 1 mm, and the internal diameter of the pipe 603 be approximately equal to or slightly longer than the diameter of the stylet 505 so that the stylet 505 can pass through the inside of the pipe 603. One end (end on the distal end 506 side) of such a pipe 603 is connected to a receiving portion 608.

The receiving portion 608 is made of stainless steel and is formed in a substantially cylindrical shape, and a substantially cylindrical hole opens to the axial center. The total axial length and diameter of this hole are respectively rather shorter than the total axial length and external diameter of the receiving portion 608. Additionally, the diameter of the hole of the receiving portion 608 is preferably made approximately equal to the external diameter of the pipe 603 so that the pipe 603 can be fixed so as not to move perpendicularly to the axial direction or the axis. The pipe 603 and the receiving portion 608 are housed in and fixed to an outer layer part made up of the body 512, the stimulation electrodes 105, and the connector part 518.

The body 512 is made of resin materials that have pliability and biocompatibility, for example, materials, such as silicone or polyurethane. The distal end 506 of the body 512 is substantially hemispherical as described above, and the radius thereof is preferably within a range of about 0.5 mm to 1.5 mm. Portions other than the distal end 506 of the body 512 are formed in a hollow, substantially cylindrical shape.

The internal diameter of the portion that is formed in a hollow, substantially cylindrical shape differs in the portion of the body 512 that comes into contact with the receiving portion 608 and the portion of the body 512 that comes into contact with the pipe 603. The internal diameter of the portion that comes into contact with the receiving portion 608 is approximately equal to the external diameter of the receiving portion 608 so as to fix the receiving portion 608. Additionally, the internal diameter of the portion that comes into contact with the pipe 603 is approximately equal to the external diameter of the pipe 603 so as to fix the pipe 603. The four stimulation electrodes 105 are fixed to such a portion of the body 512 that is formed in a hollow, substantially cylindrical shape, so that the stimulation electrodes 105 can be exposed to the surface of the body 512, as described above. In addition, since the stimulation electrodes 105 are the same as the stimulation electrodes 105 included in the electric stimulator 101 of the first embodiment, the description thereof is simplified.

One end (end on the distal end 506 side) of each of the conducting wires 202a to 202d are bonded to the stimulation electrodes 105a to 105d, respectively (refer to Fig. 14A) with the solder 203, and the other end (end on the proximal end 507 side) of each of the conducting wires 202a to 202d are electrically connected to the connector part 518. In addition, locations other than the locations of the conducting wires 202 that are bonded with the solder 203 and the locations of the conducting wires 202 that are electrically connected to the connector part 518 are completely embedded in the body 512 (refer to Fig. 14B).

The connector part 518 is formed of the same material as the body 512, and is formed as a cutout part in which a level difference is provided from the external diameter of the substantially cylindrical body 512. This cutout part is formed by a predetermined distance in the axial direction from the proximal end 507 (refer to Fig. 12A). In addition, this cutout part is planar, the four connector pins 210 (refer to Fig. 12A) are arranged on the cutout part so as to be exposed, and the conducting wires 202a to 202d are electrically connected to the four connector pins 210, respectively.

Next, the internal configuration of the circuit block 503 (refer to Fig. 12B) will be described.
A pipe 604 arranged in the circuit block 503 is the same as the pipe 603 except the length thereof. The pipe 604 is housed in and fixed to an outer layer part including the connector part 513, the body 514 that is continuous with the connector part 513, and the connector part 515 that is continuous with the body 514 (refer to Fig. 13B).

The connector part 513 is made of the same material (polyurethane or silicone) as the body 512 except for the electrical connecting portion 211. A hole of almost the same shape as the external diameter of the connector part 518 is opened in the axial direction in this connector part 513 so that the connector part 513 can be connected to the connector part 518 of the electrode block 502 (refer to Fig. 14C). The external diameter of this connector part 513 is approximately equal to the external diameter of the body 512. Additionally, the connector part 513 includes the electrical connecting portion 211 that is independently and electrically connected to the four connector pins 210, respectively, when this connector part 513 is connected to the connector part 518 of the electrode block 502. In addition, although the connector part 515 provided on the proximal end 509 side of the circuit block 503 has the same shape as the connector part 513 provided on the distal end 508 side, something like the electrical connecting portion 211 is not provided.

The body 514 is continuous with the connector parts 513 and 515 and is made of the same material as the connector parts 513 and 514. The body 514 is formed in a hollow, substantially cylindrical shape, and is formed such that the external diameter thereof is approximately equal to the external diameter of the connector parts 513 and 515, and the internal diameter thereof is approximately equal to the external diameter of the pipe 604. In addition, similarly to that described in Fig. 3B, the stimulation circuit 205 and the coil portion 212 are embedded in the body 514.

The stimulation circuit 205 is connected to the electrical connecting portion 211 via the conducting wires 204 embedded in the body 514 such that the generated electric stimulation signal is independently supplied to the respective stimulation electrodes 105, In addition, since the electric configuration of the stimulation circuit 205 and the coil portion 212 has been described in Fig. 5, the description thereof is omitted.

Next, the internal configuration of the support 504 will be described (refer to Fig. 12C).
As shown in Fig. 13B, pipes 605 and 606 arranged in the support 504 are the same as the pipes 603 and 604 except the length thereof. A valve 607 is provided between the pipe 605 and the pipe 606 in the axial direction.

The valve 607 is made of an elastic material (particularly, a soft material is preferable) with biocompatibility, like for example, silicone rubber. The valve 607 has a first slit that opens to one end face at the pipe 605 and does not open to the other end face, and a second slit that internally crosses this first slit, opens to one end face at the pipe 606, and does not open to the other end face. By providing the valve 607, liquids, such as body fluids, can be prevented from entering the insides of the electrode block 502 and the circuit block 503 from the pipe 606 even if the stylet 505 is inserted and removed via the valve 607.

The pipes 605 and 606 and the valve 607 are housed in and fixed to an outer layer part made up of the connector part 519, the body 516 that is continuous with the connector part 519, and the holder part 517 that is provided on the proximal end 511 side of the body 516.

The connector part 519 is made of, for example, polyurethane or silicone and is formed in the same shape as the connector part 518 of the electrode block 502 described above (refer to Fig. 12C). It is noted that means for performing electric connection with the outside, such as the connector pins 210, may not be provided, differently from the connector part 518.

The body 516 is made of the same material as the connector part 519, and is formed in a hollow, substantially cylindrical shape. The external diameter of the body 516 is approximately equal to the external diameter of the body 514 of the circuit block 503.

The holder part 517 is made of materials, such as a plastic, and is formed in a hollow, substantially cylindrical shape. The internal diameter of the holder part 517 is approximately equal to the external diameter of the respective pipes 603 to 606. Additionally, since the holder part 517 is a part that is held when the electric stimulator 501 is inserted into the inside of a body, the external diameter of the holder part 517 is preferably two times to three times or more the external diameter of the body 516.

As described above, the lumen for a stylet is formed by the receiving portion 608, the pipes 603 to 606, the valve 607, and the holder part 517.

In addition, the procedure of implanting the electric stimulator 501 when an electric stimulation of the nerves of the spinal cord is performed by the electric stimulator 501 will be described.
After the epidural needle 402 is inserted up to the epidural space 405 (refer to Fig. 6), the stylet 505 is inserted into the lumen for a stylet from the proximal end 511 of the holder part 517 of the electric stimulator 501. The distal end 506 (refer to Fig. 11) of the electric stimulator 501 is passed through the epidural needle 402, and the receiving portion 608 of the electric stimulator 501 is pushed by the stylet 505 to move the stimulation electrodes 105 to a targeted stimulation site of the epidural space 405. Then, after the controller (not shown) outside a body is operated to determine an optimal position of the stimulation electrodes 105, the stylet 505 is extracted from the electric stimulator 501. Since the subsequent procedure is the same as the procedure described from Figs. 8 to 10, the description thereof is omitted.

As described above, since the second embodiment includes the lumen for a stylet, a stylet can be used when the electric stimulator is implanted in a living body. Therefore, there are effects that implantation of the electric stimulator into the body can be performed easily, and the precision of the arrangement of the stimulation electrodes into the living body can be improved. In addition, it is needless to say that there is the same effect as the first embodiment.

### <Description of third embodiment of the invention>

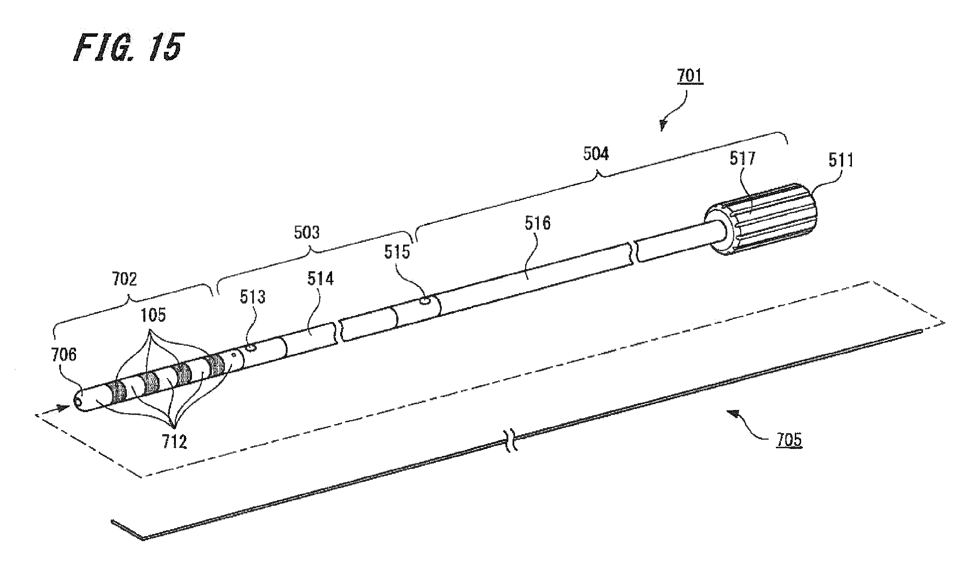

Next, a third embodiment of the invention will be described with reference to Figs. 15 to 17. An electric stimulator 701 related to the third embodiment shown in Figs. 15 to 17 includes a cylindrical hole (hereinafter referred to as a "lumen for guide wire") for inserting a guide wire into the electric stimulator 101 related to the first embodiment. Common portions of the configuration will be designated by the same reference numerals, and the description thereof will be omitted.

### [5. Configuration of electric stimulator]

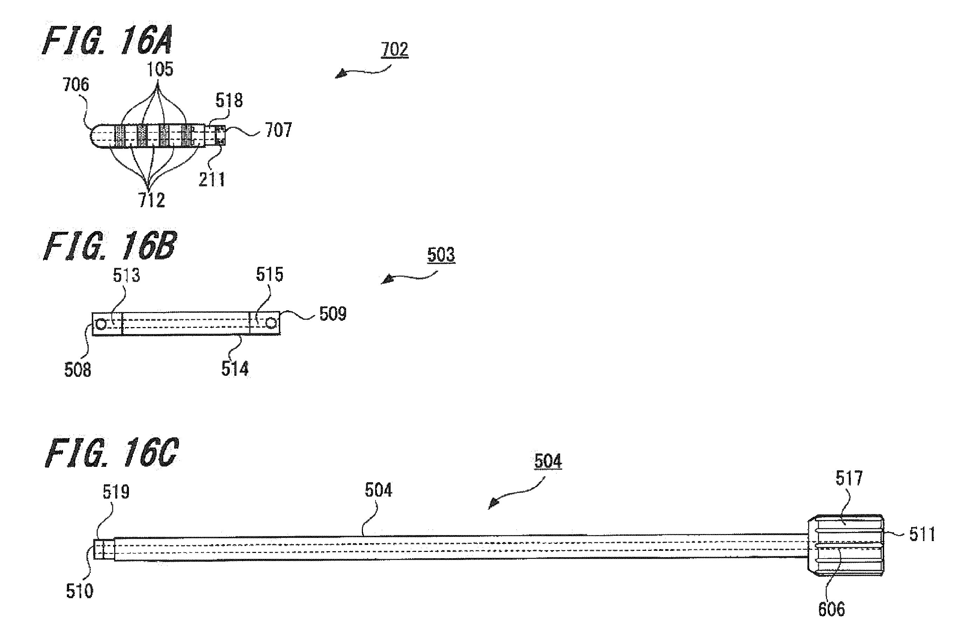

First, the general configuration of the electric stimulator related to the third embodiment will be described with reference to Fig. 15 and Figs. 16A to 16C.
Fig. 15 is a perspective view showing an overall electric stimulator related to the third embodiment of the invention.
Figs. 16A to 16C are exploded external views when the electric stimulator shown in Fig. 15 is seen from the top face.

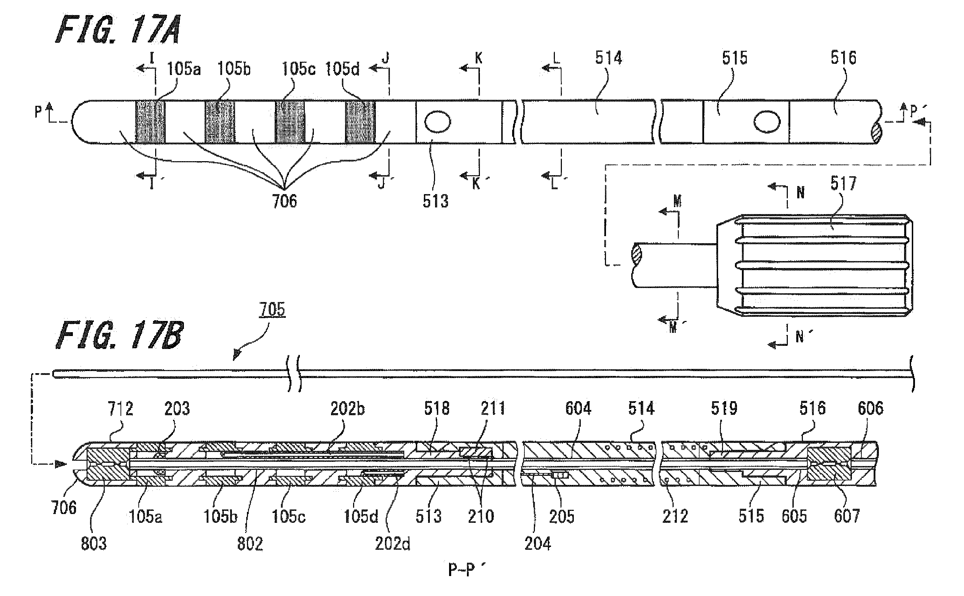

Similarly to the electric stimulators 101 and 501, the electric stimulator 701 is formed in the shape of a rod, generates an electric stimulation signal, and stimulates nerves or the like in a living body by the stimulation signal. Since the electric stimulator 701 has the lumen for a guide wire on the axis as described above, the electric stimulator 701 is formed in a hollow, substantially cylindrical shape. It is noted that the lumen for a guide wire is provided as a through hole that opens to the proximal end 511 and also opens to a distal end 706. Therefore, the electric stimulator 701 has a configuration in which the electrode block 502 of the electric stimulator 501 of the second embodiment is replaced with a substantially cylindrical electrode block 702 formed with a hollow portion that communicates in the axial direction. In addition, the internal configuration of the electrode block 702 will be described in Figs. 17A and 17B.

Next, the internal configuration of the electrode block 702 in the electric stimulator related to the third embodiment will be described with reference to Figs. 17A and 17B and the above-described Figs. 14A to 14F.
Figs. 17A and 17B are enlarged views showing the electric stimulator related to the third embodiment of the invention, and its axial internal structure.
Fig. 17A is an enlarged external view when the electric stimulator shown in Fig. 15 is seen from the top face.
Fig. 17B is a cross-sectional view showing a P-P' section of the electric stimulator shown in Fig. 17A.

Additionally, although Figs. 14A to 14F, as described above, are cross-sectional views showing the internal structure of a predetermined location in a radial direction with respect to the axis of the electric stimulator 501 related to the second embodiment of the invention, these drawings are also cross-sectional views showing the internal structure in a radial direction with respect to the electric stimulator 701 related to the third embodiment.

Here, only the electrode block 702 will be described (refer to Fig. 16A).
A pipe 802 is the same as the pipes 604 to 606 except for the axial length thereof.
Additionally, a valve 803 is the same as the valve 607. The valve 803 has a first slit that opens to one end face at the pipe 802 and does not open to the other end face, and a second slit that internally crosses this first slit, opens to one end face on the distal end 706 side of the body 712, and does not open to the other end face. Even if a guide wire 705 is inserted and removed via the valve 803, liquids, such as body fluids, can be prevented from entering the insides of the electrode block 702 and the circuit block 503 from the hole provided in the distal end 706 of the body 712.

Although the body 712 is made of, for example, materials, such as silicone or polyurethane, the body 712 has a substantially cylindrical hole in the distal end 706 thereof. The diameter of this hole is approximately equal to the external diameter of the pipe 802. In addition, the external diameter of the body 712 is the same as that of the body 512 (refer to Figs. 13A and 13B) of the second embodiment. Since the connector part 518 is the same as that described in the second embodiment, the description thereof is omitted.

In addition, the procedure of implanting the electric stimulator 701 when an electric stimulation of the nerves of the spinal cord is performed by the electric stimulator 701 will be described.
First, the epidural needle 402 is inserted up to the epidural space 405 (refer to Fig. 6), and the guide wire 705 is inserted into the epidural space 405 through the epidural needle 402. Then, a distal end of the guide wire 705 is advanced up to a targeted stimulation site. Subsequently, a proximal end of the guide wire 705 is inserted into the distal end 706 of the electric stimulator 701, the holder part 517 is pushed so as to crawl on the electric stimulator 701 on the guide wire 705, and the stimulation electrodes 105 of the electric stimulator 701 is moved to a targeted stimulation site. Then, after the controller (not shown) outside a body is operated to determine an optimal position of the stimulation electrodes 105, the guide wire 705 is extracted from the electric stimulator 701. Since the subsequent procedure is the same as the procedure described from Figs. 8 to 10, the description thereof is omitted.

As described above, since the third embodiment includes the lumen for a guide wire, a guide wire can be used when the electric stimulator is implanted in a living body. Therefore, implantation of the electric stimulator into the body can be performed easily, and the precision of the arrangement of the stimulation electrodes into the living body can be improved. In addition, it is needless to say that there is the same effect as the first embodiment.

### <Description of fourth embodiment of the invention>

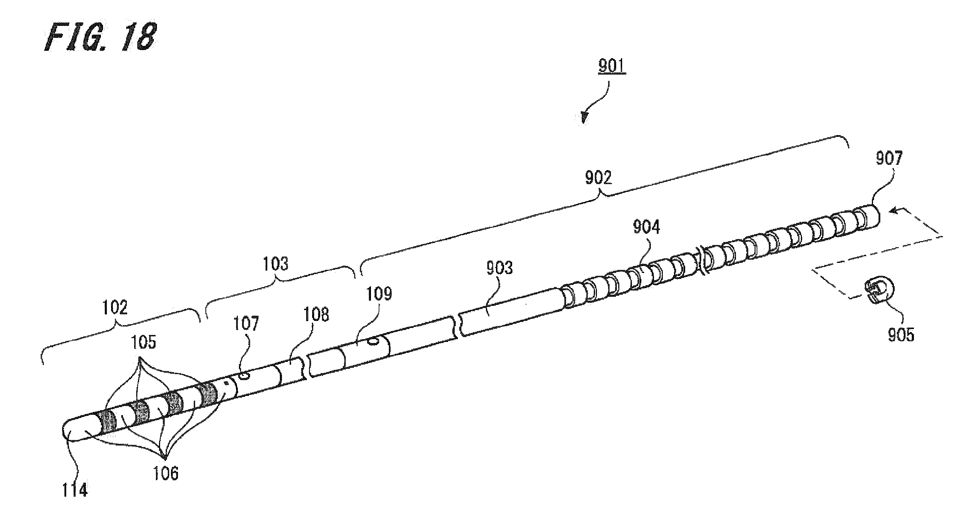

Next, a fourth embodiment of the invention will be described with reference to Figs. 18 to 21. Since the configuration of an electric stimulator 901 related to the fourth embodiment shown in Figs. 18 to 21 is hardly changed from that of the electric stimulators related to the respective embodiments described above, common portions will be designated by the same reference numerals, and the description thereof will be omitted. In addition, since the implantation procedure of the electric stimulator 901 is almost the same as the implantation procedure of the electric stimulator 101 related to the first embodiment, the description thereof will be omitted.

### [6. Configuration of electric stimulator]

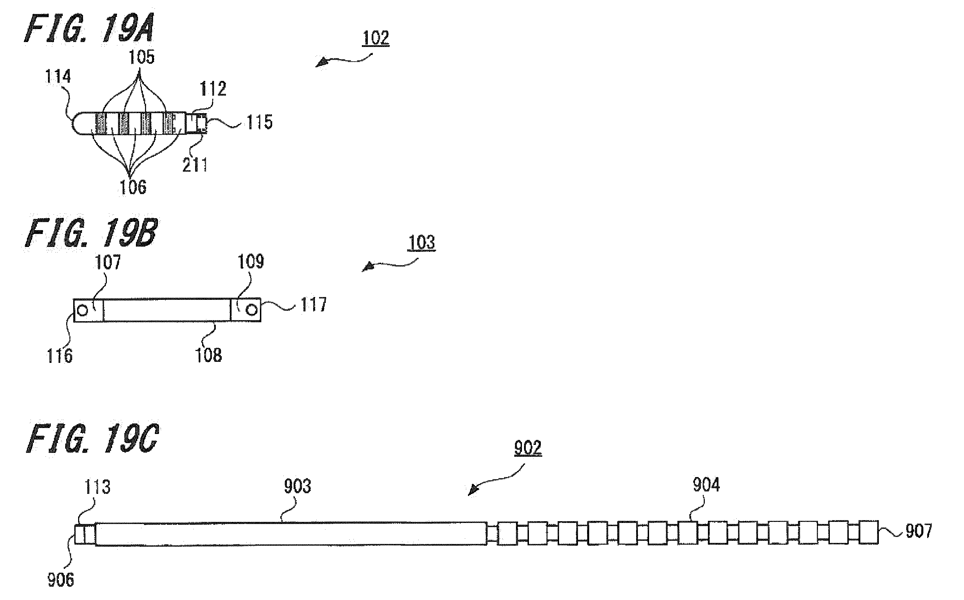

First, the general configuration of the electric stimulator related to the fourth embodiment will be described with reference to Fig. 18 and Figs. 19A to 19C.
Fig. 18 is a perspective view showing an overall electric stimulator related to the fourth embodiment of the invention.
Figs. 19A to 19C are exploded external views when the electric stimulator shown in Fig. 18 is seen from the top face.

Similarly to the above-described respective electric stimulators, the electric stimulator 901 is formed in the shape of a rod, generates an electric stimulation signal, and stimulates nerves or the like in a living body by the stimulation signal. The electric stimulator 901, as shown in Fig. 18 and Figs. 19A to 19C, has a support 902 instead of the support 104 (refer to Fig. 1) of the electric stimulator 101 related to the first embodiment, and further includes a fixture 905.

The support 902 includes the connector part 113 that is connected to the circuit block 103, a first body 903 that is formed in a substantially cylindrical shape with the same diameter as the electrode block 102, and a second body 904 that is continuous with the first body 903.

The connector part 113 is connected to the connector part 109 of the circuit block 103 such that a distal end 906 side of the first body 903 is continuous with the circuit block 103. The first body 903 is a portion that connects the connector part 113 and the second body 904 arranged on a proximal end 907 side. The second body 904 is allowed to be cut so that the electric stimulator 901 is completely implanted in a living body. The detachable fixture 905 (refer to Fig. 18) is provided at a cut surface of the second body 904.

The fixture 905 is made of materials with biocompatibility and flexibility, such as silicone, and is formed in a substantially hemispherical shape. A bottom face of the fixture 905 is formed with a substantially cylindrical cavity into which the cut surface of the second body 904 is inserted, and a lateral face of the fixture 905 is formed with a ring-shaped groove. The diameter of the substantially cylindrical cavity is made approximately equal to or slightly larger than the second body 904 so that the cut surface of the second body 904 can be inserted into the cavity of this substantially cylindrical shape. In addition, the fixture 905 is fixed to the second body 904 by a thread that is passed through the groove provided in the lateral face, in a state where the cut surface of the second body 904 is inserted into the cavity of this substantially cylindrical shape. Thereby, inflammation or a feeling of foreign matter accompanying the contact between the cut surface of the second body 904 and a living body can be prevented.

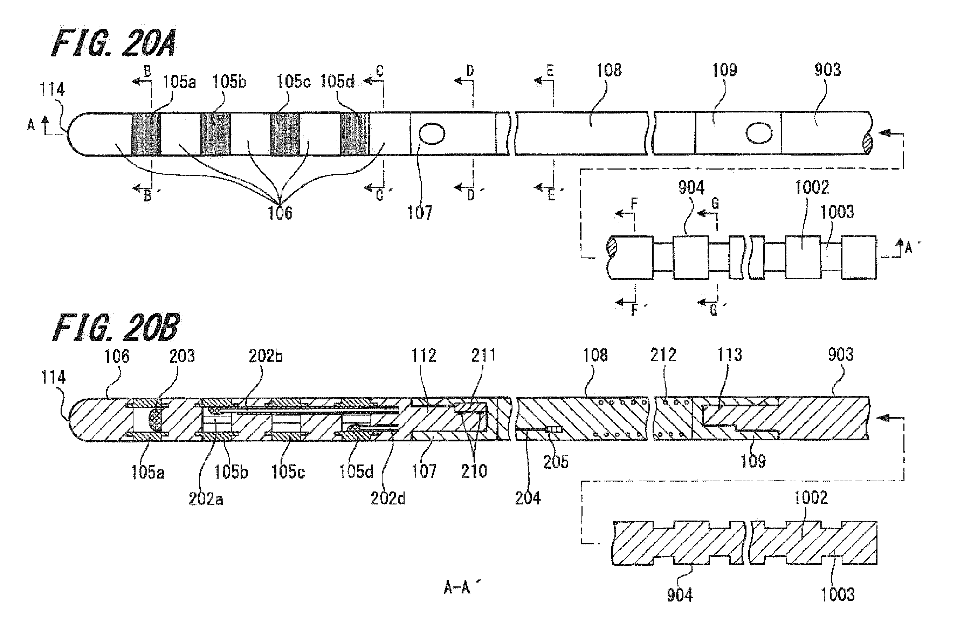

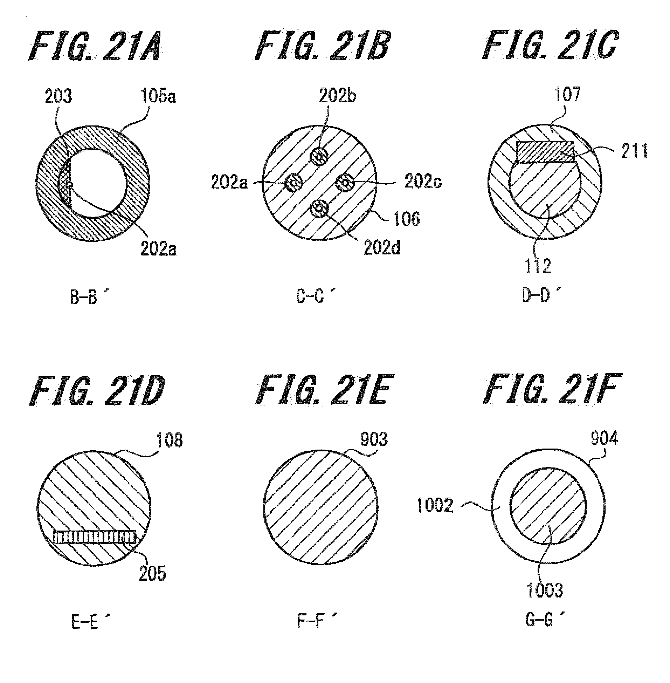

Next, the internal configuration of the support 902 related to the fourth embodiment will be described with reference to Figs. 20A and 20B and Figs. 21 A to 21F.
Figs. 20A and 20B are enlarged views showing the electric stimulator 901 related to the fourth embodiment of the invention, and its axial internal structure.
Fig. 20A is an enlarged external view when the electric stimulator shown in Fig. 18 is seen from the top face.
Fig. 20B is a cross-sectional view showing an A-A' section of the electric stimulator shown in Fig. 20A.

Additionally, Figs. 21A to 21F are cross-sectional views showing the internal structure of a predetermined location in a vertical direction with respect to the axis of the electric stimulator related to the fourth embodiment of the invention.
Fig. 21A is a cross-sectional view showing a B-B' section of the electric stimulator shown in Fig. 20A.
Fig. 21B is a cross-sectional view showing a C-C' section of the electric stimulator shown in Fig. 20A.
Fig. 21C is a cross-sectional view showing a D-D' section of the electric stimulator shown in Fig. 20A.
Fig. 21D is a cross-sectional view showing an E-E' section of the electric stimulator shown in Fig. 20A.
Fig. 21E is a cross-sectional view showing an F-F' section of the electric stimulator shown in Fig. 20A.
Fig. 21F is a cross-sectional view showing a G-G' section of the electric stimulator shown in Fig. 20A.

The internal configuration of the support 902 will be described.
The connector part 113 is made of, for example, polyurethane or silicone, and is the same as the connector part 113 (refer to Figs. 2C and 3B) of the support 104 described in the first embodiment.
The first body 903 is made of the same material as the connector part 113, and is formed in a substantially cylindrical shape. The diameter of the first body 903 is made approximately equal to the external diameter of the body 110 (refer to Figs. 2C, 3A, and 3B) of the support 104 described in the first embodiment (refer to Fig. 21 E).

The second body 904 is continuous with the first body 903, and is made of the same material as the first body 903. The second body 904 is made up of a fixture fixing portion 1002 that is formed in a substantially cylindrical shape with the same diameter as the diameter of the first body 903, and an easily-cut portion 1003 that is formed with a diameter smaller than the diameter of the fixture fixing portion 1002 (refer to Figs. 20B and 21F). A plurality of the fixture fixing portions 1002 and the easily-cut portions 1003 are alternately provided such that the axes thereof coincide with each other.

### <Description of fifth embodiment of the invention>

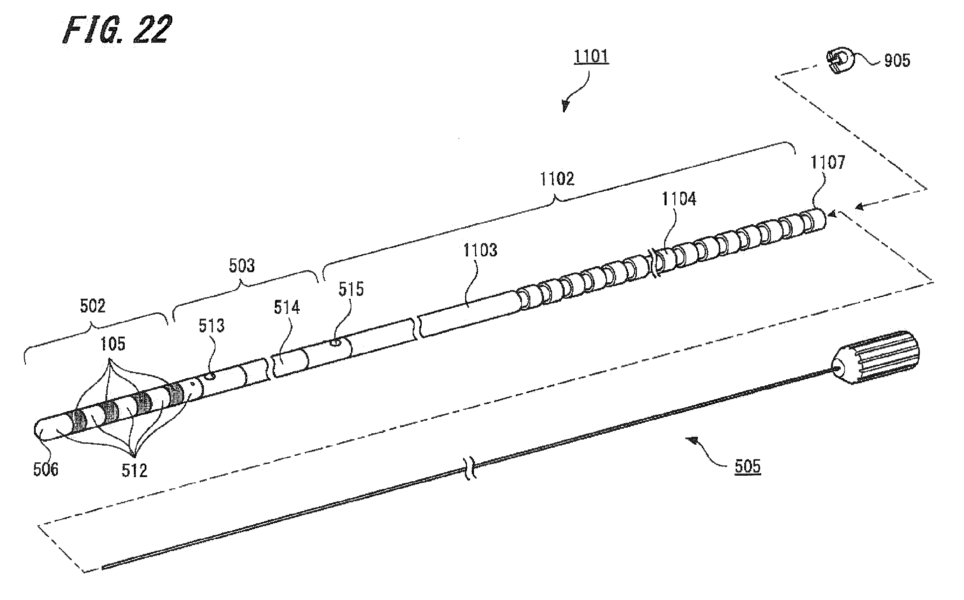

Next, a fifth embodiment of the invention will be described with reference to Figs. 22 to 25. An electric stimulator 1101 related to the fifth embodiment shown in Figs. 22 to 25 forms a lumen for a stylet in the electric stimulator 901 related to the fourth embodiment. Since the configuration of this electric stimulator 1101 is hardly changed from that of the electric stimulators related to the respective embodiments described above, common portions will be designated by the same reference numerals, and the description thereof will be omitted. In addition, since the implantation procedure of the electric stimulator 1101 related to the fifth embodiment is almost the same as the implantation procedure of the electric stimulator 501 related to the second embodiment, the description thereof will be omitted.

### [7. Configuration of electric stimulator]

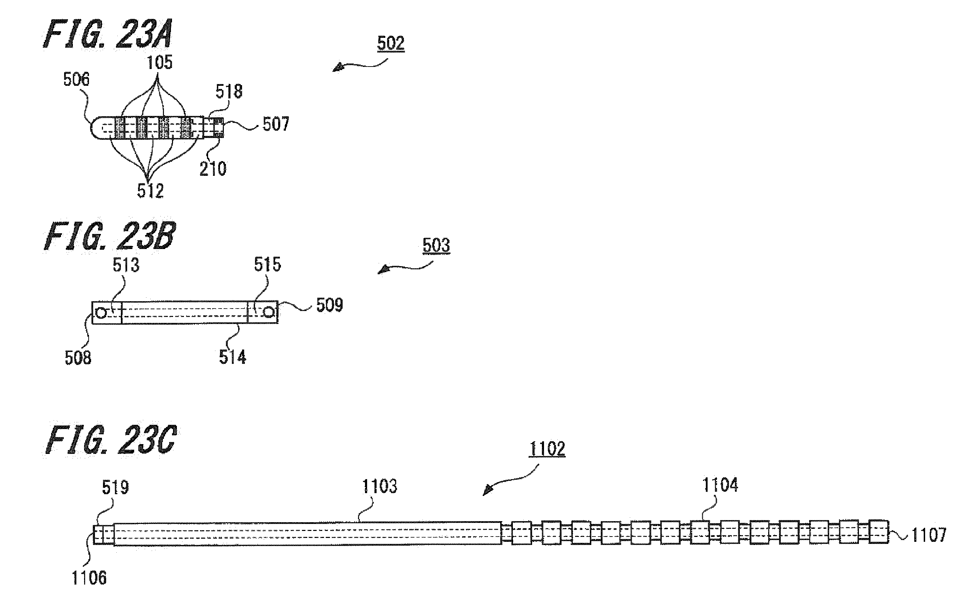

First, the general configuration of the electric stimulator related to the fifth embodiment will be described with reference to Fig. 22 and Figs. 23A to 23C.
Fig. 22 is a perspective view showing an overall electric stimulator related to the fifth embodiment of the invention.
Figs. 23A to 23C are exploded external views when the electric stimulator shown in Fig. 22 is seen from the top face.

Similarly to the above-described respective electric stimulators, the electric stimulator 1101 is formed in the shape of a rod, generates an electric stimulation signal, and stimulates nerves or the like in a living body by the stimulation signal. The electric stimulator 1101, as shown in Fig. 22 and Figs. 23A to 23C, has a support 1102 instead of the support 504 (refer to Fig. 11) of the electric stimulator 501 related to the second embodiment, and further includes the above-described fixture 905.

The support 1102 includes the connector part 519 for being connected to the circuit block 503, a first body 1103 that is formed in a substantially cylindrical shape with the same diameter as the electrode block 502, and a second body 1104 that is continuous with the first body 1103.

The connector part 519 of the support 1102 is connected to the connector part 515 of the circuit block 503 such that a distal end 1106 side of the first body 1103 is continuous with the circuit block 503. The first body 1103 is a portion that connects the connector part 519 and the second body 1104 arranged on a proximal end 1107 side.

The second body 1104 is allowed to be cut so that the electric stimulator 1101 is completely implanted in a living body. The detachable fixture 905 is attached to the cut surface of the cut second body 1104 so as to cover the cut surface. In addition, since the fixture 905 has already been described in Fig. 18, the detailed description thereof is omitted. It is noted that the fixture 905 is fixed to the second body 1104 by a thread. This can prevent inflammation or a feeling of foreign matter accompanying the contact between the cut surface of the second body 1104 and a living body, and can prevent entering of a liquid from a lumen for a stylet on the proximal end 1107 side of the second body 1104.

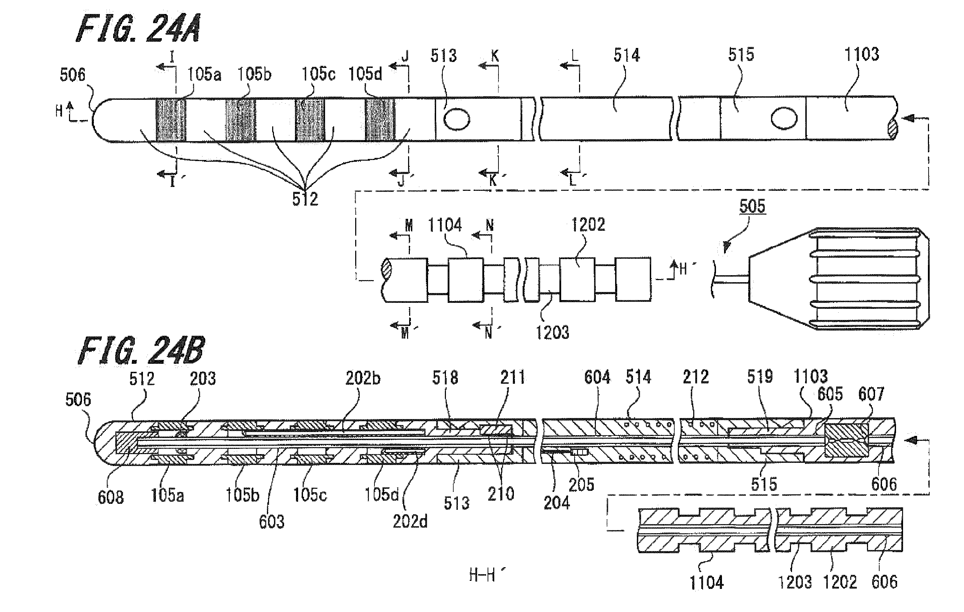

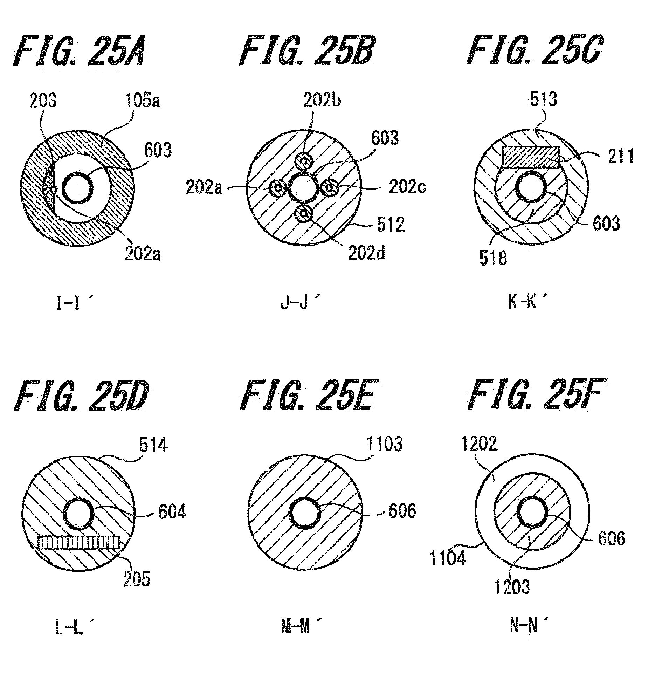

Next, the internal configuration of the support 1102 related to the fifth embodiment will be described with reference to Figs. 24A and 24B and Figs. 25A to 25F. Figs. 24A and 24B are enlarged views showing the electric stimulator related to the fifth embodiment of the invention, and its axial internal structure.
Fig. 24A is an enlarged external view when the electric stimulator shown in Fig. 22 is seen from the top face.
Fig. 24B is a cross-sectional view showing an H-H' section of the electric stimulator shown in Fig. 24A.

Additionally, Figs. 25A to 25F are cross-sectional views showing the internal structure of a predetermined location in a vertical direction with respect to the axis of the electric stimulator related to the fifth embodiment of the invention.
Fig. 25A is a cross-sectional view showing an I-I' section of the electric stimulator shown in Fig. 24A.
Fig. 25B is a cross-sectional view showing a J-J' section of the electric stimulator shown in Fig. 24A.
Fig. 25C is a cross-sectional view showing a K-K' section of the electric stimulator shown in Fig. 24A.
Fig. 25D is a cross-sectional view showing an L-L' section of the electric stimulator shown in Fig. 24A.
Fig. 25E is a cross-sectional view showing an M-M' section of the electric stimulator shown in Fig. 24A.
Fig. 25F is a cross-sectional view showing an N-N' section of the electric stimulator shown in Fig. 24A.

The support 1102, similarly to the second embodiment, includes the pipes 605 and 606, the valve 607 provided between the pipe 605 and the pipe 606 in the axial direction, the connector part 519 in which the pipes 605 and 606 and the valve 607 are housed and fixed, and the first and second bodies 1103 and 1104.

The first body 1103 is made of, for example, polyurethane or silicone and is formed in a hollow, substantially cylindrical shape. The external diameter of the first body 1103 is approximately equal to the external diameter of the body 514 of the circuit block 503 (refer to Fig. 25E).

The second body 1104 is continuous with the first body 1103, and is made of the same material as the first body 1103. The second body 1104 is made up of a fixture fixing portion 1202 that is formed in a substantially cylindrical shape with the same diameter as the diameter of the first body 1103, and an easily-cut portion 1203 that has a diameter smaller than the diameter of the fixture fixing portion 1202 (refer to Fig. 25F). A plurality of the fixture fixing portions 1202 and the easily-cut portions 1203 are alternately provided such that the axes thereof coincide with each other. In addition, the connector part 519, the first body 1103, and the second body 1104 have a cavity of a diameter equal to the external diameter of the pipe 606 in the axial direction on the axis thereof.

### <Description of sixth embodiment of the invention>

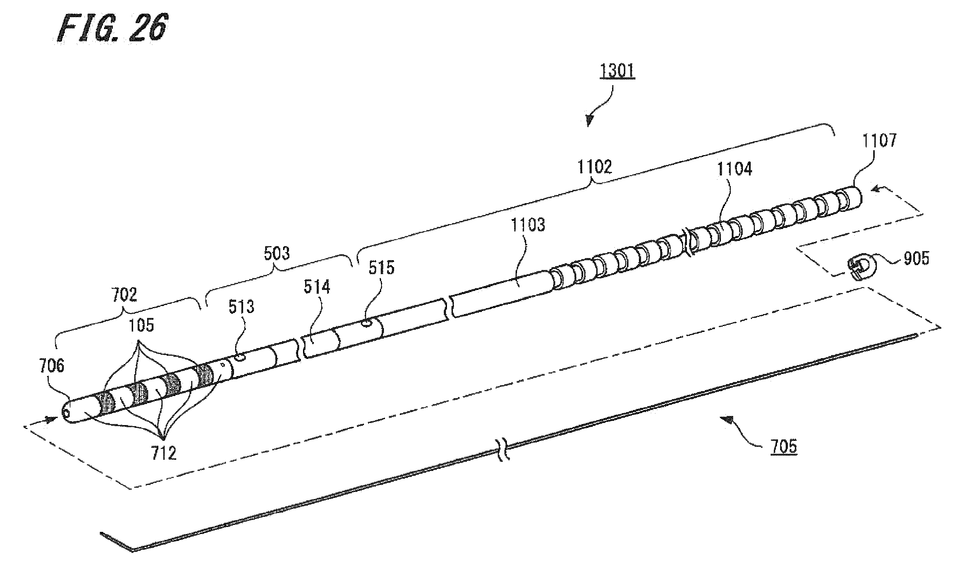

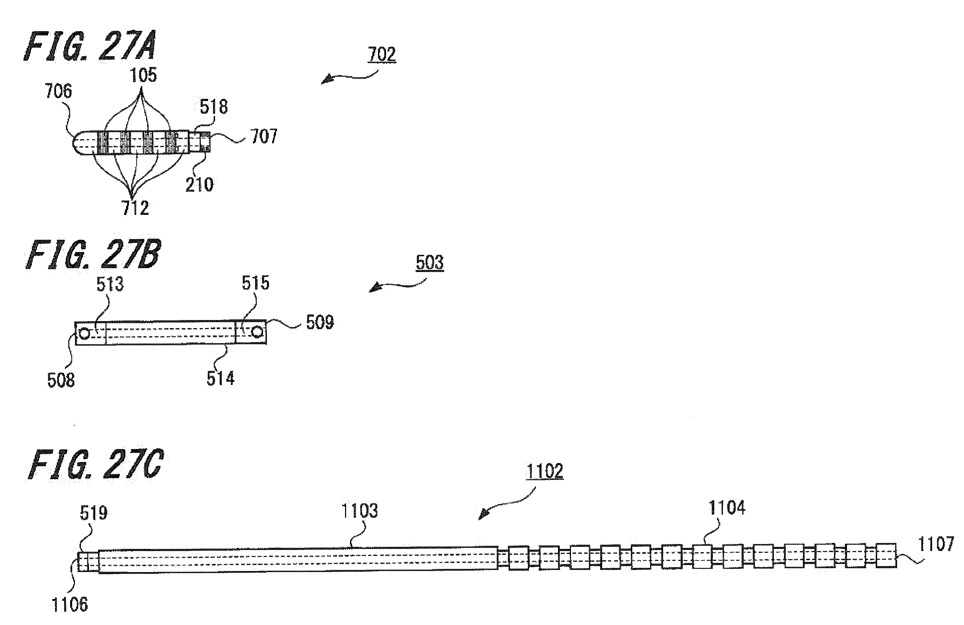

Next, a sixth embodiment of the invention will be described with reference to Fig. 26 and Figs. 27A to 27C. An electric stimulator 1301 related to the sixth embodiment shown in Fig. 26 and Figs. 27A to 27C forms a lumen for a guide wire in the electric stimulator 901 related to the fourth embodiment. Since the configuration of this electric stimulator 1301 is hardly changed from that of the electric stimulators of the above-described respective embodiments, common portions will be designated by the same reference numerals, and the description thereof will be omitted.

### [8. Configuration of electric stimulator]

First, the general configuration of the electric stimulator related to the sixth embodiment will be described with reference to Fig. 26 and Figs. 27A to 27C.
Fig. 26 is a perspective view showing an overall electric stimulator related to the sixth embodiment of the invention.
Figs. 27A to 27C are exploded external views when the electric stimulator shown in Fig. 26 is seen from the top face.

Similarly to the above-described respective electric stimulators, the electric stimulator 1301 is formed in the shape of a rod, generates an electric stimulation signal, and stimulates nerves or the like in a living body by the stimulation signal. The electric stimulator 1301, as shown in Fig. 26 and Figs. 27A to 27C, is provided with the electrode block 702 related to the third embodiment, instead of the electrode block 502 (refer to Fig. 22) related to the fifth embodiment.

As described above, in the fourth to sixth embodiment, the support is provided with the easily-cut portions. Thus, there is an effect that cutting of the support can be easily performed. Additionally, when the support and a living body are sewn with a thread in implantation processing, the thread is wound around the easily-cut portion, so that the thread can be prevented from moving in the axial direction. In addition to these, it is needless to say that there are the same effects as those of the first to third embodiments.

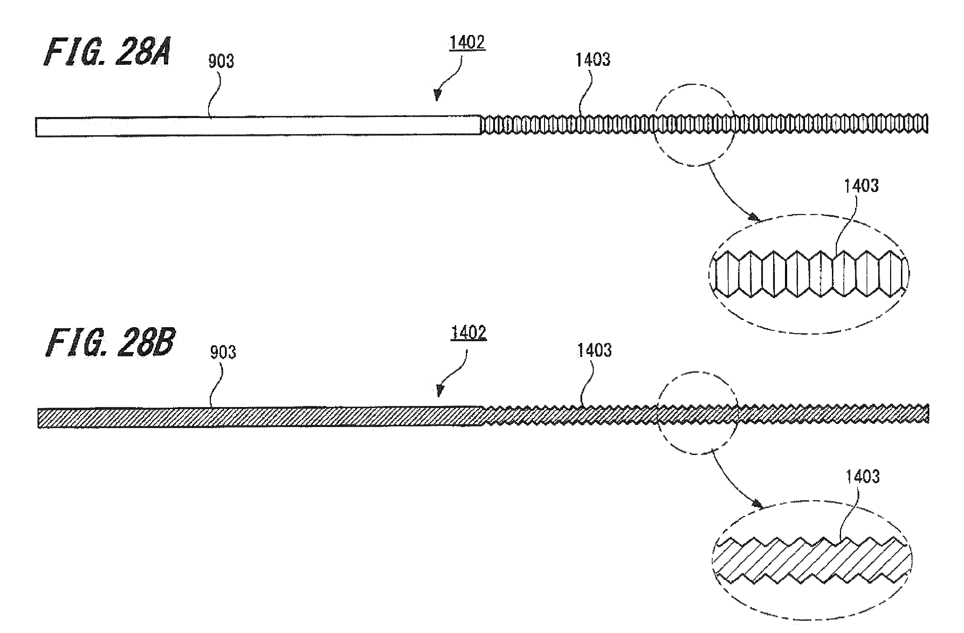

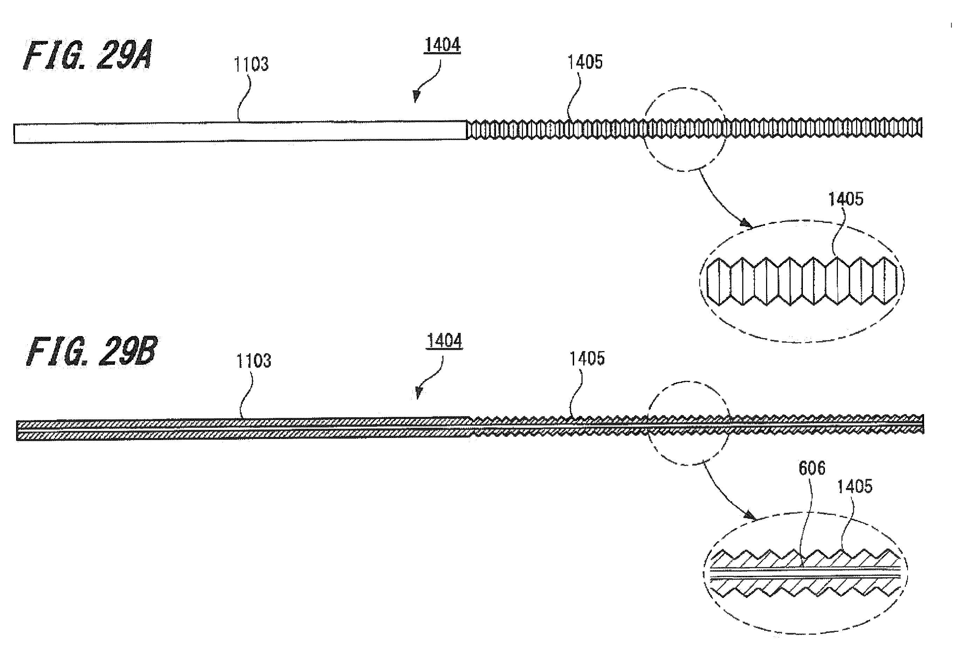

Here, a first modification of the electric stimulators related to the fourth to sixth embodiments will be described with reference to Figs. 28A and 28B and Figs. 29A and 29B.
Figs. 28A and 28B are explanatory views showing a support.
The first modification provides a support 1402 instead of the support 902 of the electric stimulator 901. More specifically, a configuration in which a second body 1403 as shown in Figs. 28A and 28B is provided is adopted as an alternative to the second body 904 (refer to Fig. 18) of the support.

The second body 1403 is made of the same material as the second body 904, and is formed in a shape (a shape in which a plurality of abacus balls are arranged in the direction of a minor axis) in which a plurality of substantial bicones are arranged in the axial direction. In addition, portions of the second body 1403 with the shortest diameter have the same function as the easily-cut portions 1003 (refer to Figs. 20A and 20B) of the second body 904. That is, in order to adjust the axial length of the electric stimulator, a portion of the second body 1403 with the shortest diameter is cut, and this portion is sewn on a tissue in a living body with a thread to fix the electric stimulator.

In addition, by forming a substantially cylindrical cavity in the support 1402 in the axial direction as in a support 1404 of Figs. 29A and 29B, it is also possible to use the support 1404 as an alternative to the support 1102 (refer to Figs. 22 and 26) of the electric stimulators 1101 and 1301 of the fifth and sixth embodiments.

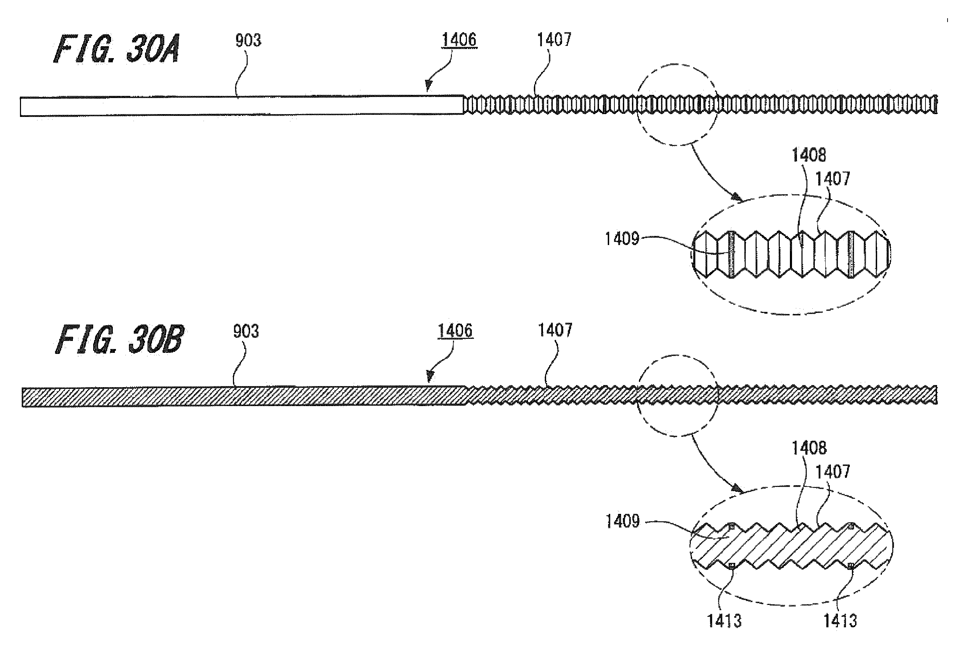

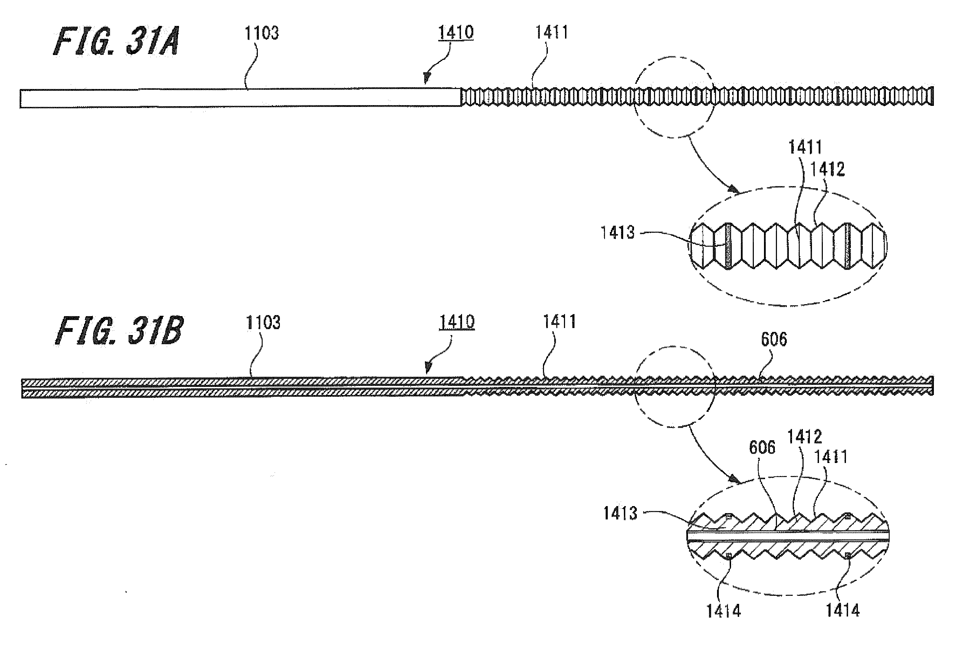

Next, a second modification of the electric stimulators related to the fourth to sixth embodiments will be described with reference to Figs. 30A and 30B and Figs. 31A and 31B Figs. 30A and 30B are explanatory views showing a support.
The second modification provides a support 1406 instead of the support 902 of the electric stimulator 901. More specifically, a configuration in which a second body 1407 shown in Figs. 30A and 30B is provided is adopted as an alternative to the second body 904 (refer to Fig. 18) of the support 902.

The second body 1407 is also made of the same material as the second body 904, and is formed including a plurality of biconical portions 1408 and a plurality of marking portions 1409.

The biconical portions 1408 have portions formed in the shape of a substantial bicone. Additionally, the marking portions 1409 include a first substantially conical portion, a substantially cylindrical portion with a top face that coincides with the bottom face of the first substantially conical portion, and a second substantially conical portion with a bottom face that forms the undersurface of the substantially cylindrical portion. A colored ring 1413 is embedded in this substantially cylindrical portion such that the axes thereof overlap each other. In addition, as described above, since the materials of the support 1406, i.e., the materials of the marking portions 1409, are transparent materials, such as silicone or polyurethane, the ring 1413 can be seen therethrough.

Such marking portions 1409 are provided at predetermined intervals between the respective biconical portions 1408. A doctor can know the depth when an electric stimulator is implanted in a living body, with the position of each marking portion 1409, i.e., the see-through ring 1413 as an indication.

Meanwhile, the portions of the second body 1407 with the shortest diameter, that is, connecting portions between the respective biconical portions 1408 and connecting portions between the biconical portions 1408 and the marking portions 1409 have the same function as the easily-cut portions 1003 of the second body 904 (refer to Figs. 20A and 20B). That is, in order to adjust the axial length of the electric stimulator, the portion of the second body 1407 with the shortest diameter is cut, and this portion is sewn on a tissue in a living body with a thread to fix the electric stimulator.

In addition, by forming a substantially cylindrical cavity in the support 1406 of Figs. 30A and 30B in the axial direction as in a support 1410 of Figs. 31A and 31B, it is also possible to use the support 1410 as an alternative to the support 1102 (refer to Figs. 22 and 26) of the electric stimulators 1101 and 1301 of the fifth and sixth embodiments.

Additionally, it is needless to say that performing marking on each second body of the above-described respective supports with a pigment with biocompatibility at every predetermined interval can be an alternative to the marking portion 1409 (refer to Figs. 30A and 30B) or 1413 (refer to Figs. 31A and 31B).

### <Description of seventh embodiment of the invention>

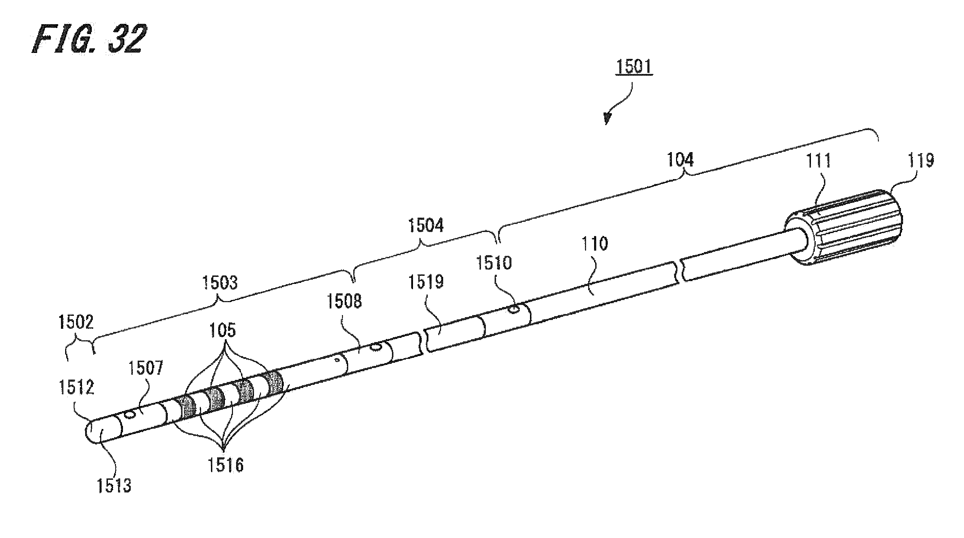

Next, a seventh embodiment of the invention will be described with reference to Figs. 32 to 35. Since the configuration of an electric stimulator 1501 related to the seventh embodiment shown in Figs. 32 to 35 is hardly changed from that of the electric stimulators related to the respective embodiments described above, common portions will be designated by the same reference numerals, and the description thereof will be omitted.
In addition, since the implantation procedure of the electric stimulator 1501 related to the seventh embodiment is the same as the implantation procedure of the electric stimulator 101 related to the first embodiment, the description thereof will be omitted.

### [9. Configuration of electric stimulator]

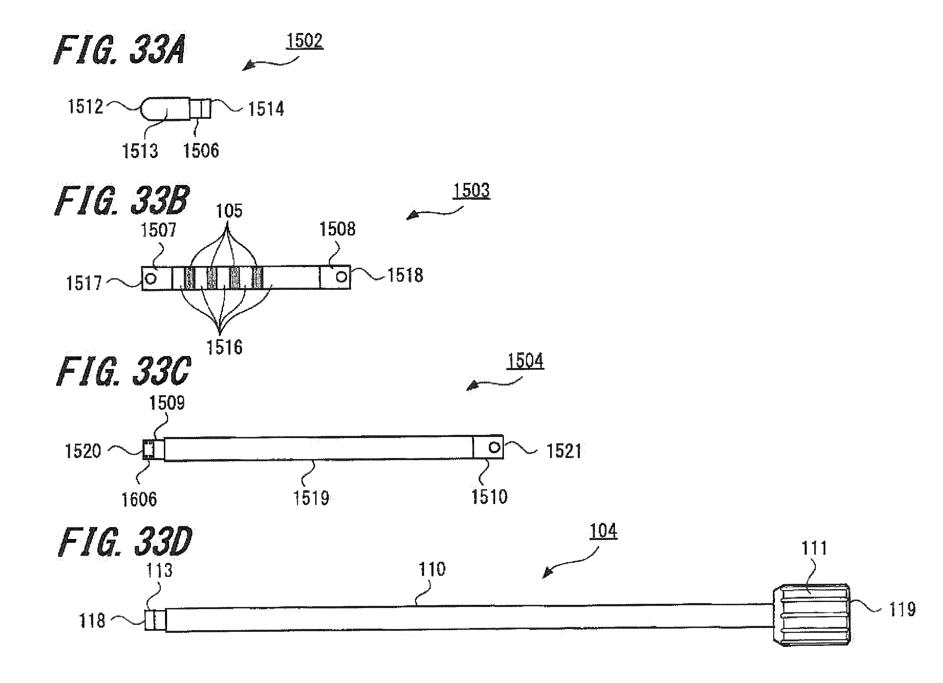

First, the general configuration of the electric stimulator related to the seventh embodiment will be described with reference to Fig. 32 and Figs. 33A to 33D.
Fig. 32 is a perspective view showing an overall electric stimulator related to the seventh embodiment of the invention.
Figs. 33A to 33D are exploded external views when the electric stimulator shown in Fig. 32 is seen from the top face.

Similarly to the above-described respective electric stimulators, the electric stimulator 1501 is formed in the shape of a rod, generates an electric stimulation signal, and stimulates nerves or the like in a living body by the stimulation signal. The electric stimulator 1501, as shown in Fig. 32 and Figs. 33A to 33D, includes an electrode block 1503 and a circuit block 1504, respectively, instead of the electrode block 102 and the circuit block 103 of the electric stimulator 101 (refer to Fig. 1)related to the first embodiment, and further includes an insertion block 1502.

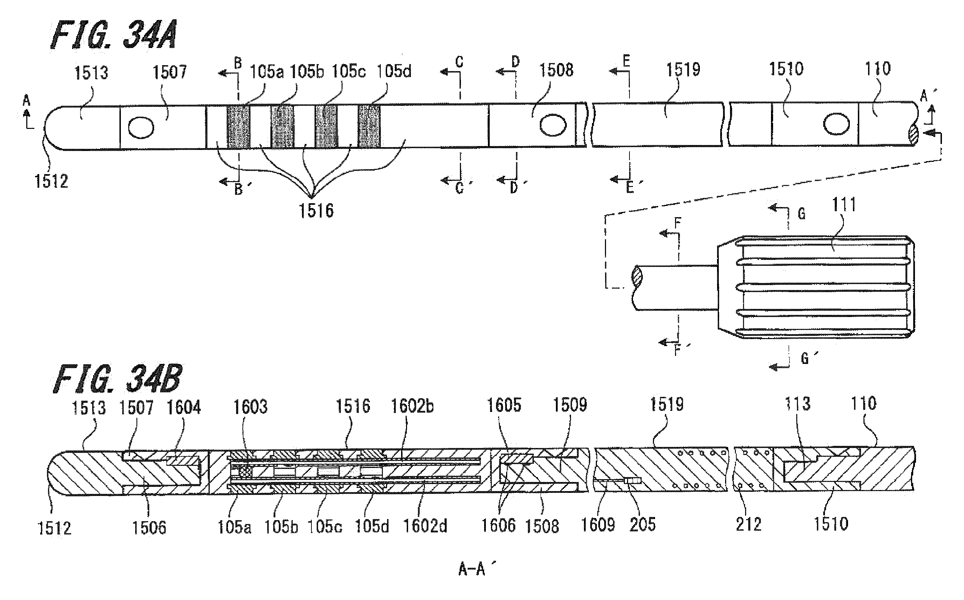

The insertion block 1502 includes a body 1513 in which a distal end 1512 is formed in a substantially hemispherical shape, and the other portions are formed in a substantially cylindrical shape. A connector part 1506 for connecting the insertion block 1502 to the electrode block 1503 is disposed on a proximal end 1514 side of the body 1513. In addition, the internal configuration of the insertion block 1502 will be described in Figs. 34A and 34B.

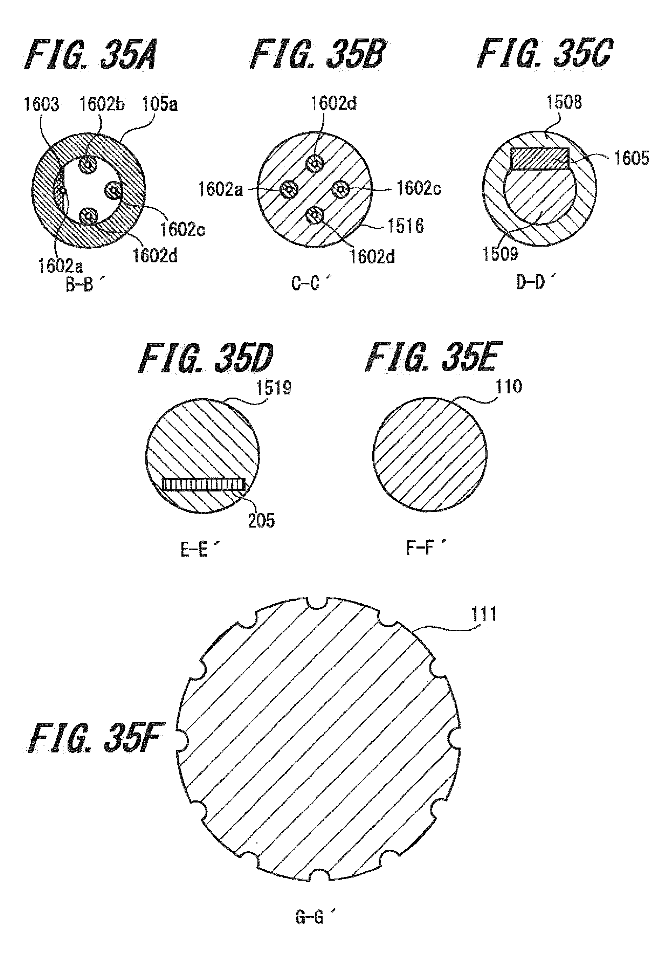

The electrode block 1503 is formed in a substantially cylindrical shape with the same diameter as the substantially cylindrical portion of the insertion block 1502. This electrode block 1503 includes the above-described stimulation electrodes 105, and a body 1516 that is arranged at equal intervals such that, when the electric stimulator 1501 is implanted in a living body, the respective stimulation electrodes 105 are exposed to the living body. A connector part 1507 to be connected to the connector part 1506 of the insertion block 1502 is provided on a distal end 1517 side of the body 1516 such that the distal end 1517 of the electrode block 1503 and the proximal end 1514 side of the body 1513 of the insertion block 1502 are continuous with each other. Moreover, a connector part 1508 for connecting the circuit block 1504 to a proximal end 1518 of the electrode block 1503 is disposed on the proximal end 1518 side of the body 1516. In addition, the internal configuration of the electrode block 1503 will be described below in Figs. 34A and 34B and Figs. 35A to 35F.

The circuit block 1504 is formed in a substantially cylindrical shape with the same diameter as the electrode block 1503. The circuit block 1504 includes a connector part 1509 that is connected to the connector part 1508 of the electrode block 1503 such that a distal end 1520 of a body 1519 is continuous with the proximal end 1518 of the electrode block 1503. Additionally, the circuit block 1504 is provided with the body 1519 that is continuous with the connector part 1509. Moreover, the circuit block 1504 includes a connector part 1510 that is continuous with a proximal end 1521 side of the body 1519 to connect the support 104 to the proximal end 1521 of the circuit block 1504. In addition, the internal configuration of the circuit block 1504 will be described below in Figs. 34A and 34B and Figs. 35A to 35F.

Next, the internal configuration of the electric stimulator 1501 related to the seventh embodiment will be described with reference to Figs. 34A and 34B and Figs. 35A to 35F.
Figs. 34A and 34B are enlarged views showing the electric stimulator related to the seventh embodiment of the invention, and its axial internal structure.
Fig. 34A is an enlarged external view when the electric stimulator shown in Fig. 32 is seen from the top face.
Fig. 34B is a cross-sectional view showing an A-A' section of the electric stimulator shown in Fig. 34A.

Additionally, Figs. 35A to 35F are cross-sectional views showing the internal structure of a predetermined location in a vertical direction with respect to the axis of the electric stimulator related to the seventh embodiment of the invention.
Fig. 35A is a cross-sectional view showing a B-B'section of the electric stimulator shown in Fig. 34A.
Fig. 35B is a cross-sectional view showing a C-C' section of the electric stimulator shown in Fig. 34A.
Fig. 35C is a cross-sectional view showing a D-D' section of the electric stimulator shown in Fig. 34A.
Fig. 35D is a cross-sectional view showing an E-E' section of the electric stimulator shown in Fig. 34A.
Fig. 35E is a cross-sectional view showing an F-F' section of the electric stimulator shown in Fig. 34A.
Fig. 35F is a cross-sectional view showing a G-G' section of the electric stimulator shown in Fig. 34A.

First, the internal configuration of the insertion block 1502 (refer to Fig. 33A) will be described.
The body 1513 is made of resin materials that have pliability and biocompatibility, for example, materials, such as silicone or polyurethane. The distal end 1512 of the body 1513 is substantially hemispherical as described above, and the radius thereof is preferably within a range of about 0.5 mm to 1.5 mm. Portions of the body 1513 other than the distal end 1512 are formed in a substantially cylindrical shape, and the diameter thereof is preferably in a range of about 1 to 3 mm.

The connector part 1506 is formed of the same material as the body 1513, and is formed as a cutout part in which a level difference is provided from the external diameter of the substantially cylindrical body 1513. This cutout part is formed by a predetermined distance in the axial direction from the proximal end 1514 (refer to Fig. 33A).

Next, the internal configuration of the electrode block 1503 (refer to Fig. 33B) will be described. The body 1516 is made of, for example, material, such as polyurethane or silicone.
A portion of the body 1516 in the axial direction is formed in a hollow, substantially cylindrical shape, and the external diameter of the body 1516 is equal to the diameter of the substantially cylindrical portion of the body 1513 of the insertion block 1502. The body 1516 is fixed such that the four stimulation electrodes 105a to 105d described in Fig. 3 are exposed to the surface of the body 1516.

Portions of conducting wires 1602a to 1602d are bonded to the stimulation electrodes 105a to 1 05d, respectively, with solder 1603 (refer to Figs. 34B and 35A). One end of each of the conducting wires 1602a to 1602d is electrically connected to the connector part 1507, and the other end of each of the conducting wires 1602a to 1602d is electrically connected to the connector part 1508. In addition, locations other than the locations of the conducting wires 1602 that are bonded with the solder 1603 and the locations of the conducting wires 1602 that are electrically connected to the connector part 1507 or 1508 are insulated and coated with PTFE or ETFE and are completely embedded in the body 1516 (refer to Fig. 35B).

The connector parts 1507 and 1508 are made of the same material as the body 1516 except for electrical connecting portions 1604 and 1605 that will be described below, and are formed in a hollow, substantially cylindrical shape in the axial direction. The external diameter of the connector parts 1507 and 1508 is approximately equal to the external diameter of the body 1516, and the hollow portions of the connector parts 1507 and 1508 are formed in almost the same shape as the outer shell of the connector part 1506 of the insertion block 1502 so that the connector parts 1507 and 1508 are respectively connectable to the connector part 1506 of the insertion block 1502 or the connector part 1509 of the circuit block 1504. Moreover, the connector parts 1507 and 1508 respectively include the electrical connecting portions 1604 and 1605 including four electrodes (not shown) that are independently electrically connected to the conducting wires 1602a to 1602d, respectively.

Next, the internal configuration of the circuit block 1504 (refer to Fig. 33C) will be described.
The connector part 1509 of the circuit block 1504, similarly to the connector [part 1506 of the insertion block 1502, is also formed of, for example, materials, such as polyurethane or silicone. The connector part 1509, as described above, has the same shape as the connector part 1506 of the insertion block 1502 connectable to the connector part 1508 so as to be connectable to the connector part 1508 of the electrode block 1503. Additionally, four connector pins 1606 are exposed and arranged on a cutout part. The four connector pins 1506 of the connector part 1509 are respectively electrically connected to the four electrodes of the electrical connecting portion 1605 of the connector part 1508, in a state of the connector part 1509 being connected to the connector part 1508 of the electrode block 1503.

The body 1519 is made of the same material as the connector part 1509. The body 1519 is formed in a substantially cylindrical shape, and is formed such that the diameter thereof is approximately equal to the external diameter of the body 1516 of the electrode block 1503.

Additionally, the stimulation circuit 205 described in Figs. 3B and 5 and the coil portion 212 electrically connected to the stimulation circuit 205 are embedded in the body 1519. In addition, the coil portion 212 is formed so as to be wound with the axial direction as an axis.

The stimulation circuit 205 is respectively connected to the four connector pins 1606 of the connector part 1509 via conducting wires 1609 embedded in the body 1519 in order to be able to supply a generated electric stimulation signal to each stimulation electrode 105, independently.

The connector part 1510 has the same shape as the connector parts 1507 and 1508 of the electrode block 1503, and all of these are made of the same material as the body 1519.

### <Description of eighth embodiment of the invention>

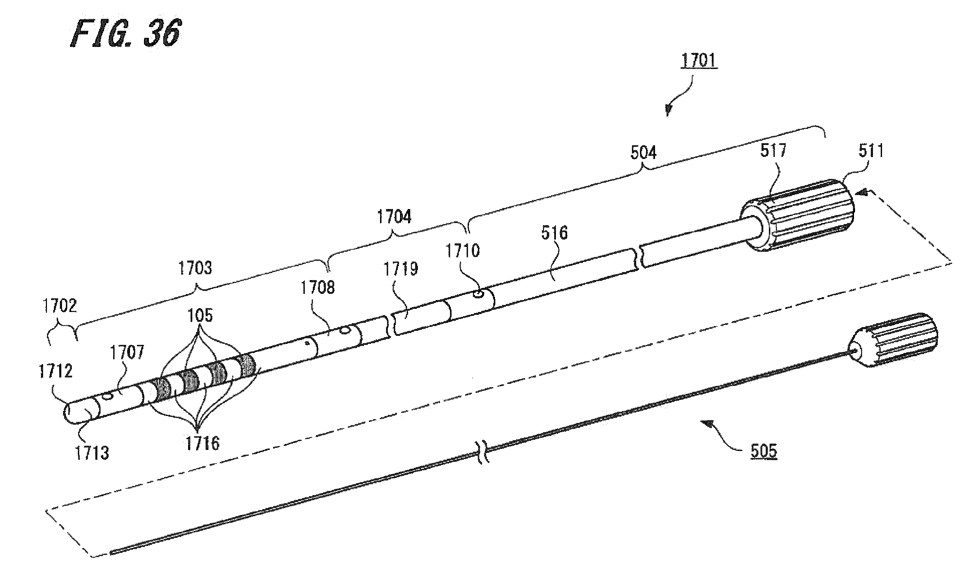

Next, an eighth embodiment of the invention will be described with reference to Figs. 36 to 39. An electric stimulator 1701 related to the eighth embodiment shown in Figs. 36 to 39 forms a lumen for a stylet in the electric stimulator 1501 related to the seventh embodiment. Since the configuration of this electric stimulator 1701 is almost the same as that of the electric stimulators related to the respective embodiments described above, common portions will be designated by the same reference numerals, and the description thereof will be omitted. In addition, since the implantation procedure of the electric stimulator 1701 related to the eighth embodiment is the same as the implantation procedure of the electric stimulator 501 related to the second embodiment, the description thereof will be omitted.

### [10. Configuration of electric stimulator]

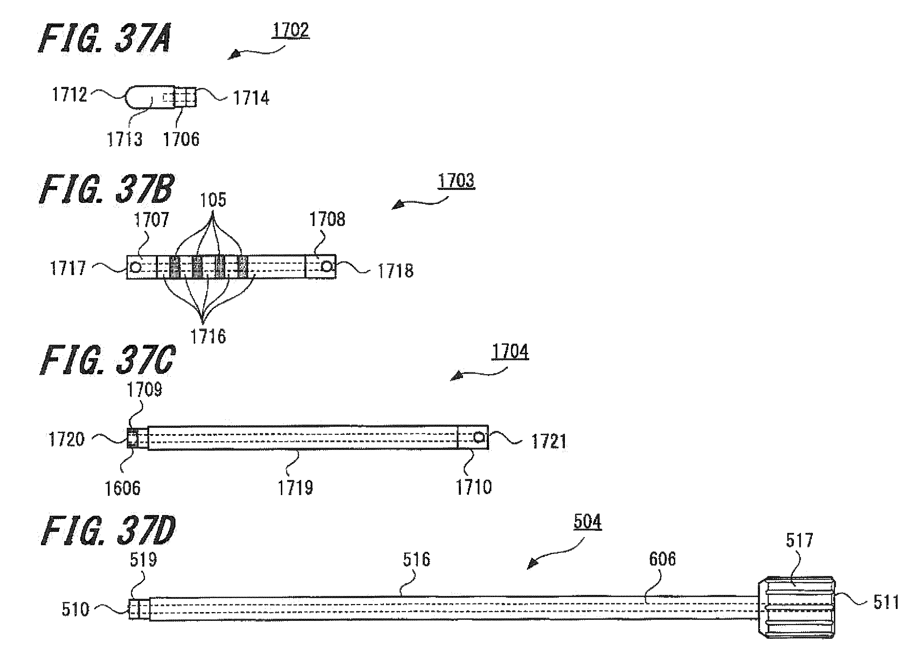

First, the general configuration of the electric stimulator related to the eighth embodiment will be described with reference to Fig. 36 and Figs. 37A to 37D.
Fig. 36 is a perspective view showing an overall electric stimulator related to the eighth embodiment of the invention.
Figs. 37A to 37D are exploded external views when the electric stimulator shown in Fig. 36 is seen from the top face.

Similarly to the above-described respective electric stimulators, the electric stimulator 1701 is formed in the shape of a rod, generates an electric stimulation signal, and stimulates nerves or the like in a living body by the stimulation signal. As described above, a lumen for a stylet is provided in the electric stimulator 1701 in the axial direction of the electric stimulator 1501 of the seventh embodiment. Therefore, the electric stimulator 1701 is constituted by an insertion block 1702, an electrode block 1703, a circuit block 1704, and the support 504 that form a hollow portion in the axial direction, instead of the insertion block 1502, the electrode block 1503, the circuit block 1504, and the support 104 shown in Fig. 32. In addition, since the support 504 has been described in the second embodiment, the description thereof is omitted.

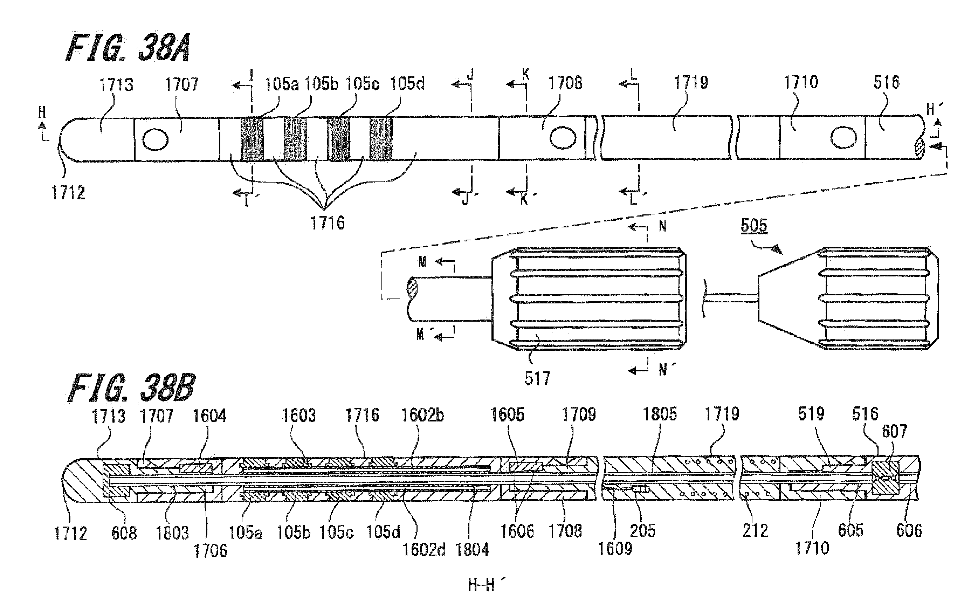

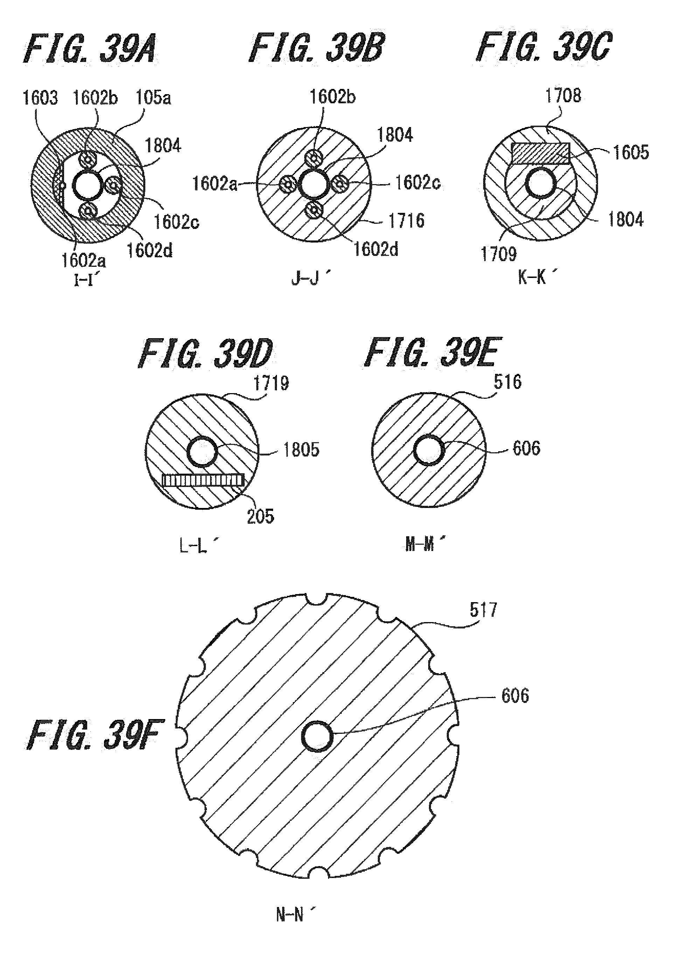

In the electric stimulator 1701, as shown in Figs. 37A to 37D, when the respective blocks are connected together in order of the insertion block 1702, the electrode block 1703, the circuit block 1704, and the support 504, each of these blocks forms a lumen for a stylet that communicates in the axial direction of the blocks. In this case, as for the lumen for a stylet, an opening is provided in the proximal end 511, and this opening is connected up to the vicinity of a distal end 1712. In addition, the diameter of the lumen for a stylet is preferably approximately equal to or slightly longer than the diameter of the stylet 505. In addition, the internal configuration of the insertion block 1702, the electrode block 1703, and the circuit block 1704 will be described below in Figs. 38A and 38B and Figs. 39A to 39F.

Next, the internal configuration of the electric stimulator related to the eighth embodiment will be described with reference to Figs. 38A and 38B and Figs. 39A to 39F.
Figs. 38A and 38B are enlarged views showing the electric stimulator related to the eighth embodiment of the invention, and its axial internal structure.
Fig. 38A is an enlarged external view when the electric stimulator shown in Fig. 36 is seen from the top face.
Fig. 38B is a cross-sectional view showing an H-H' section of the electric stimulator shown in Fig. 38A.

Additionally, Figs. 39A to 39F are cross-sectional views showing the internal structure of a predetermined location in a vertical direction with respect to the axis of the electric stimulator related to the eighth embodiment of the invention.
Fig. 39A is a cross-sectional view showing an I-I' section of the electric stimulator shown in Fig. 38A.
Fig. 39B is a cross-sectional view showing a J-J' section of the electric stimulator shown in Fig. 38A.
Fig. 39C is a cross-sectional view showing a K-K' section of the electric stimulator shown in Fig. 38A.
Fig. 39D is a cross-sectional view showing an L-L' section of the electric stimulator shown in Fig. 38A.
Fig. 39E is a cross-sectional view showing an M-M' section of the electric stimulator shown in Fig. 38A.
Fig. 39F is a cross-sectional view showing an N-N' section of the electric stimulator shown in Fig. 38A.

First, the internal configuration of the insertion block 1702 will be described.
A pipe 1803 is made of materials that have biocompatibility, insulation, and pliability, for example, PTFE or ETFE, and is formed in a hollow, substantially cylindrical shape in the axial direction. It is desirable that the external diameter of the pipe 1803 be about 0.1 to 1 mm, and the internal diameter of the pipe 1803 be approximately equal to or slightly longer than the diameter of the stylet 505 so that the stylet 505 can pass through the inside of the pipe 1803. One end (end at the distal end 1712) of such a pipe 1803 is connected to the receiving portion 608 described in Fig. 13B. The pipe 1803 and the receiving portion 608 are housed in and fixed to an outer layer part made up of a body 1713, and a connector part 1706 (refer to Fig. 38B).

The body 1713 is made of resin materials that have pliability and biocompatibility, for example, materials, such as silicone or polyurethane. The distal end 1712 of the body 1713 is substantially hemispherical, and the radius thereof is preferably within a range of about 0.5 mm to 1.5 mm. Portions of the body 1713 other than the distal end 1712 are formed in a substantially cylindrical shape that is hollow in the axial direction. The external diameter of the hollow, substantival cylindrical portion is desirably in a range of about 1 to 3 mm, and the internal diameter is approximately equal to the external diameter of the receiving portion 608 or the pipe 1803, in order to house and fix the receiving portion 608 and the pipe 1803.

The connector part 1706 is formed of the same material as the body 1713, and is formed as a cutout part in which a level difference is provided from the external diameter of the hollow, substantially cylindrical body 1713. This cutout part is formed by a predetermined distance in the axial direction from a proximal end 1714 (refer to Fig. 37A).

Next, the internal configuration of the electrode block 1703 (refer to Fig. 37B) will be described.
A pipe 1804 that constitutes the electrode block 1703 is also the same as the pipe 1803 except for the axial length thereof. The pipe 1804 is housed in and fixed to an outer layer part including a connector part 1707, a body 1716, the stimulation electrodes 105, and a connector part 1708 (refer to Fig. 38B).

The body 1716 is made of the same material as the body 1713, and is formed in a hollow, substantially cylindrical shape in the axial direction. The external diameter of the body 1716 is equal to the external diameter of the hollow, substantially cylindrical portion of the body 1713 of the insertion block 1702, and the internal diameter of the body 1716 is approximately equal to the external diameter of the pipe 1804 in order to house and fix the pipe 1804. The above-described four stimulation electrodes 105a to 105d are provided so as to be exposed to the surface of the body 1716.

The connector parts 1707 and 1708 are made of the same material as the body 1716 except for the above-described electrical connecting portions 1604 and 1605. The external diameter of the connector parts 1707 and 1708 is approximately equal to the external diameter of the body 1716, and the hollow portions formed in the connector parts 1707 and 1708 are formed in almost the same shape as the outer shell of the connector part 1706 of the insertion block 1702 so that the connector parts 1707 and 1708 are respectively connectable to the connector part 1706 of the insertion block 1702 or a connector part 1709 of the circuit block 1704. Moreover, the connector parts 1707 and 1708 respectively include the electrical connecting portions 1604 and 1605 including four electrodes (not shown) as described above.

Next, the internal configuration of the circuit block 1704 (refer to Fig. 37C) will be described.
A pipe 1805 is the same as the pipe 1803 except for the axial length thereof. The pipe 1805 is housed in and fixed to an outer layer part including the connector part 1709, a body 1719, and a connector part 1710 (refer to Fig. 38B).

The connector part 1709 is made of the same material as the connector parts 1706 to 1708. The connector part 1709 has the same shape as the connector part 1706 of the insertion block 1702 connectable to the connector part 1708 so as to be connectable to the connector part 1708 of the electrode block 1703, and the four connector pins 1606 are exposed and arranged on a cutout part (refer to Fig. 37C). The four connector pins 1606 of the connector part 1709 are respectively electrically connected to the four electrodes of the electrical connecting portion 1605 of the connector part 1708, in a state of the connector part 1709 being connected to the connector part 1708 of the electrode block 1703.

The body 1719 of the circuit block 1704 is made of the same material as the connector part 1709. The body 1739 is formed in a hollow, substantially cylindrical shape in the axial direction. The external diameter of the body 1719 is approximately equal to the external diameter of the body 1716 of the electrode block 1703, and the internal diameter of the body 1719 is made approximately equal to tha external diameter of the pipe 1805 in order to house and fix the pipe 1805.

The stimulation circuit 205 described in Fig. 3B and the coil portion 212 electrically connected to the stimulation circuit 205 are embedded in the body 1719. In addition, the coil portion 212 is formed so as to be wound with the axial direction as an axis. The stimulation circuit 205 is respectively connected to the four connector pins 1606 of the connector part 1709 via the conducting wires 1609 embedded in the body 1719 in order to be able to supply a generated electric stimulation signal to each stimulation electrode 105, independently. In addition, the connector part 1710 has the same shape as the connector parts 1707 and 1708 of the electrode block 1703, and all of these are made of the same material as the body 1719.

### <Description of ninth embodiment of the invention>

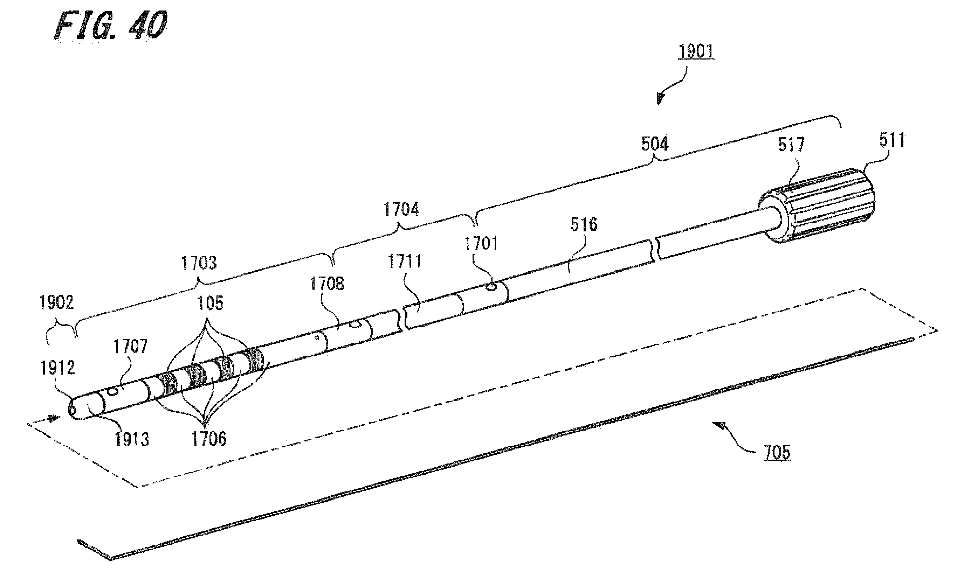

Next, a ninth embodiment of the invention will be described with reference to Figs. 40 to 42. An electric stimulator 1901 related to the ninth embodiment shown in Figs. 40 to 42 forms a four a guide wire in the electric stimulator 1501 related to the seventh embodiment. Portions whose configuration is common to the above-described respective embodiments will be designated by the same reference numerals, and the description thereof will be omitted. In addition, since the implantation procedure of the electric stimulator 1901 related to the ninth embodiment is the same as the implantation procedure of the electric stimulator 701 related to the third embodiment, the description thereof will be omitted.

### [11. Configuration of electric stimulator]

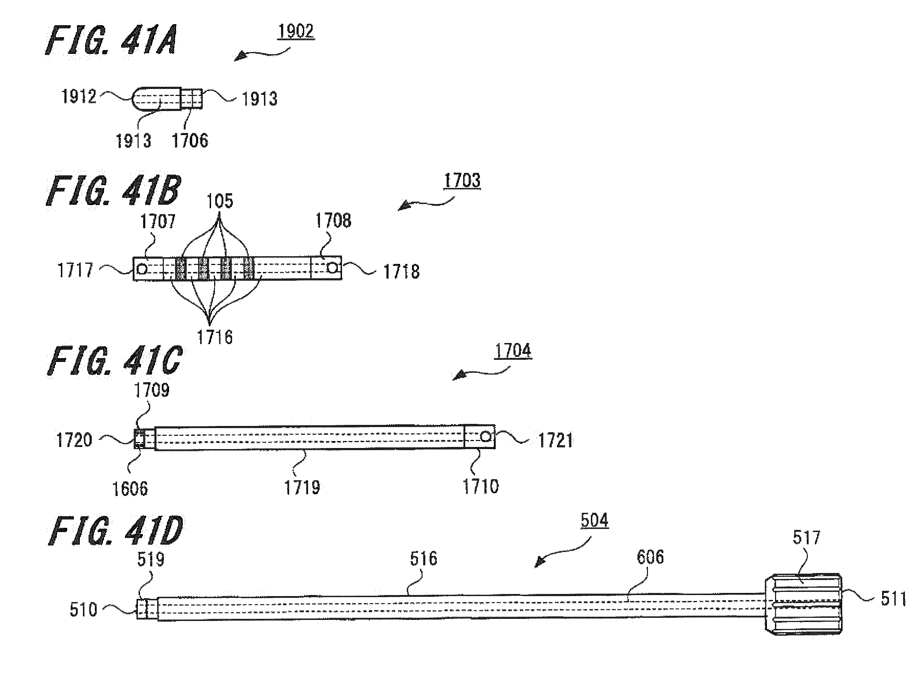

First, the general configuration of the electric stimulator related to the ninth embodiment will be described with reference to Fig. 40 and Figs. 41A to 41D.
Fig. 40 is a perspective view showing an overall electric stimulator related to the ninth embodiment of the invention.
Figs. 41A to 41D are exploded external views when the electric stimulator shown in Fig. 40 is seen from the top face.

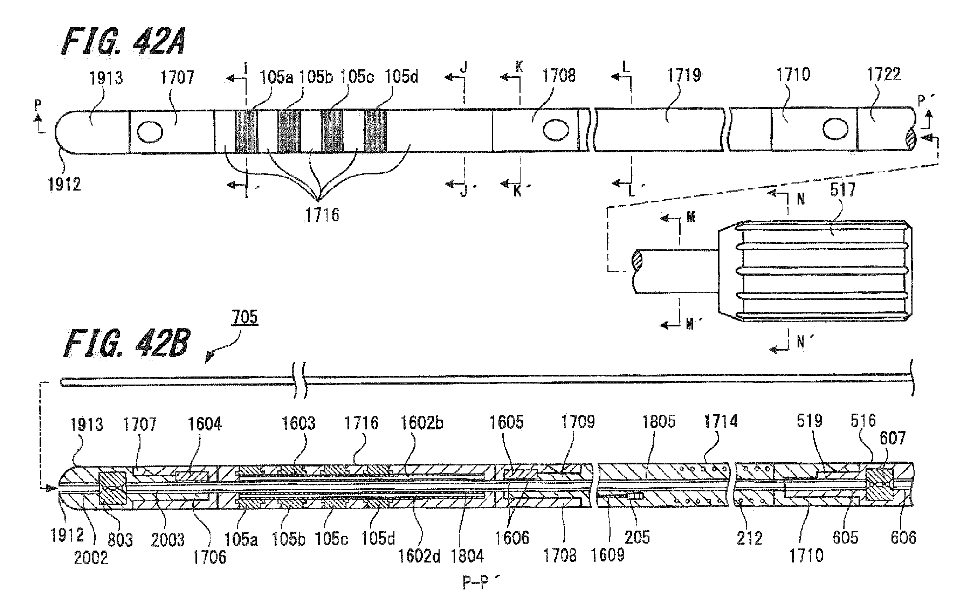

Similarly to the above-described respective electric stimulators, the electric stimulator 1901 is formed in the shape of a rod, generates an electric stimulation signal, and stimulates nerves or the like in a living body by the stimulation signal. Since the electric stimulator 1901 has the lumen for a guide wire on the axis as described above, the electric stimulator 1901 is formed in a hollow, substantially cylindrical shape. It is noted that the lumen for a guide wire, as shown in Figs. 41A to 41D, is provided as a through hole that opens to the proximal end 511 and also opens to a distal end 1912. Therefore, the electric stimulator 1901 has a configuration in which the insertion block 1702 of the electric stimulator 1701 of the eighth embodiment is replaced with a substantially cylindrical insertion block 1902 formed with a hollow portion (refer to Fig. 41A) that communicates in the axial direction. In addition, the internal configuration of the insertion block 1902 will be described in Figs. 42A and 42B.

Next, the internal configuration of the electric stimulator related to the ninth embodiment will be described with reference to Figs. 42A and 42B and Figs. 39A to 39F.
Figs. 42A and 42B are enlarged views showing the electric stimulator related to the ninth embodiment of the invention, and its axial internal structure.
Fig. 42A is an enlarged external view when the electric stimulator shown in Fig. 40 is seen from the top face.
Fig. 42B is a cross-sectional view showing a P-P' section of the electric stimulator shown in Fig. 40A.

Additionally, although Figs. 39A to 39F, as described above, are cross-sectional views showing the internal structure of a predetermined location in a vertical direction with respect to the axis of the electric stimulator 1701 related to the eighth embodiment of the invention, these drawings are also cross-sectional views showing the internal structure in a vertical direction with respect to the electric stimulator 1901 related to the ninth embodiment.

Here, only the internal configuration of the insertion block 1902 will be described.
Pipes 2002 and 2003 are the same as the pipes 1804 and 1805 except for the axial length thereof. The valve 803 described in Fig. 17B is provided between the pipe 2002 and the pipe 2003 in the axial direction. Even if the guide wire 705 is inserted and removed via the valve 803, liquids, such as body fluids, can be prevented from entering the insides of the electrode block 1703 and the circuit block 1704 from the hole provided in the distal end 1912 of a body 1913.

The pipes 2002 and 2003 and the valve 803 are housed and fixed in an outer layer part made up of the body 1913, and the connector part 1706.

Although the body 1913 is made of, for example, materials, such as silicone or polyurethane, the body 1913 has a substantially cylindrical hole in the distal end 1912 thereof. The diameter of this hole is approximately equal to the external diameter of the pipe 2002. In addition, the external diameter of the body 1913 is the same as that of the body 1713 (refer to Figs. 38A and 38B) of the eighth embodiment. In addition, since the connector part 1706 is the same as that described in the eighth embodiment, the description thereof is omitted.

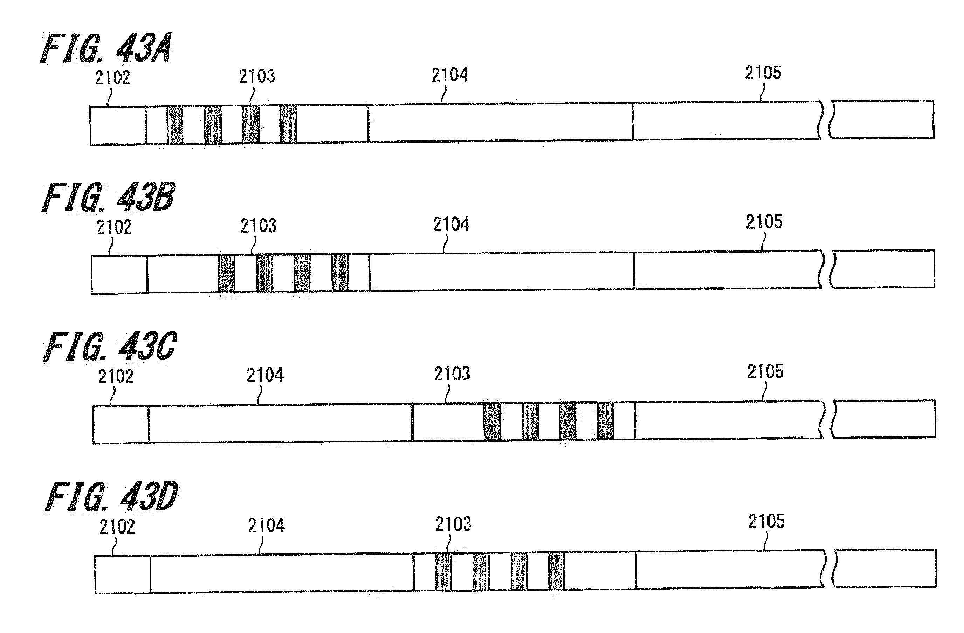

Meanwhile, in the above-described seventh to ninth embodiments, the electric stimulators 1501, 1701, and 1901, as shown in Fig. 43A, are connected in order of a insertion block 2102, a electrode block 2103, a circuit block 2104, and a support 2105 from a distal end side. However, respective connector parts provided at both ends of the electrode block 2103 of the respective embodiments have the same shape (refer to Figs. 34B, 38B, and 42B). Therefore, the electrode block 2103 in the direction of Fig. 43A can be reversed in the horizontal direction, and the insertion block 2102, the electrode block 2103, the circuit block 2104, and the support 2105 can also be mechanically connected in order from the distal end (refer to Fig. 43B). Since the connector parts at both ends of the electrode block 2103 respectively have the electrical connecting portions electrically connected to the stimulation electrodes (refer to Figs. 34B, 38B, and 42B), even if the respective blocks are connected as shown in Fig. 43B, an electric stimulation signal generated in the circuit block 2104 (more specifically, the stimulation circuit) is applied to the stimulation electrodes of the electrode block 2103. That is, as for the electrode block 2103, if the electrode block 2103 and the circuit block 2104 are mechanically connected in a direction as shown in Fig. 43B, the electrode block 2103 and the circuit block 2104 can be electrically connected.

Additionally, the shape of one connector part (the connector part that is not connected to the electrode block 2103) of the circuit block 2104 is the same as that of the connector parts at both ends of the electrode block 2103. Accordingly, as shown in Fig. 43C or Fig. 43D, the respective blocks are mechanically connected in order of the insertion block 2102, the circuit block 2104, the electrode block 2103, and the support 2105 from the distal end side. In addition, it is needless to say that the circuit block 2104 and the electrode block 2103 are electrically connected as described, even if the electric stimulator is in the state of Figs. 43C or 43D.

As described above, since the electric stimulators of the above-described seventh to ninth embodiments can change the connection between the respective blocks as in Figs. 43A to 43D, the position of the stimulation electrodes can be changed. Therefore, the position of the stimulation electrodes can be still more freely determined than when adjusting the length of only the support to determine the position the stimulation electrodes. That is, the electric stimulator can be completely implanted in a living body, holding the position of the stimulation electrodes without any further restrictions on patient's bodily features. Thereby, suitable nerves can be stimulated more reliably. In addition to this, it is needless to say that there are the same effects as those of the first to third embodiments.

### <Description of tenth embodiment of the invention>

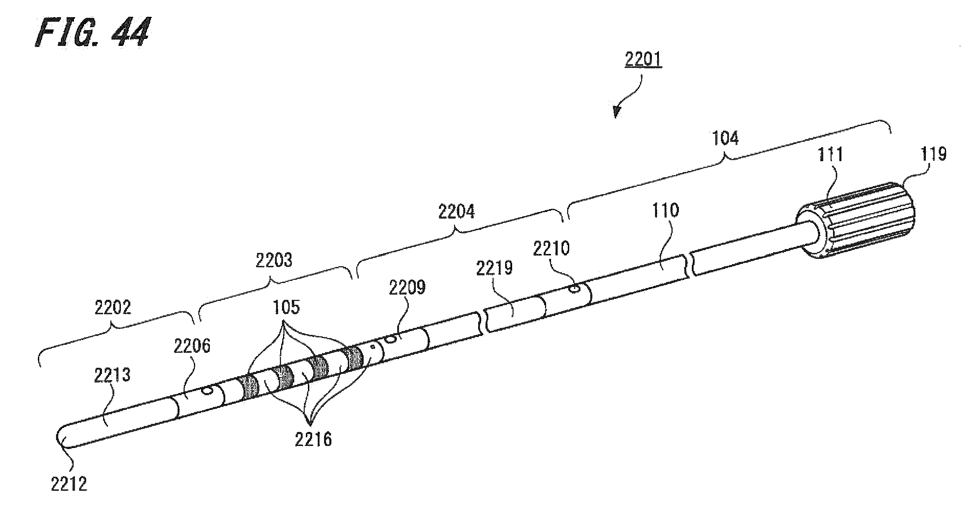

Next, a tenth embodiment of the invention will be described with reference to Figs. 44 to 47. Since the configuration of an electric stimulator 2201 related to the tenth embodiment shown in Figs. 44 to 47 is hardly changed from that of the electric stimulators related to the respective embodiments described above, common portions will be designated by the same reference numerals, and the description thereof will be omitted
In addition, since the implantation procedure of the electric stimulator 2201 related to the tenth embodiment is the same as the implantation procedure of the electric stimulator 101 related to the first embodiment, the description thereof will be omitted.

### [12. Configuration of electric stimulator]

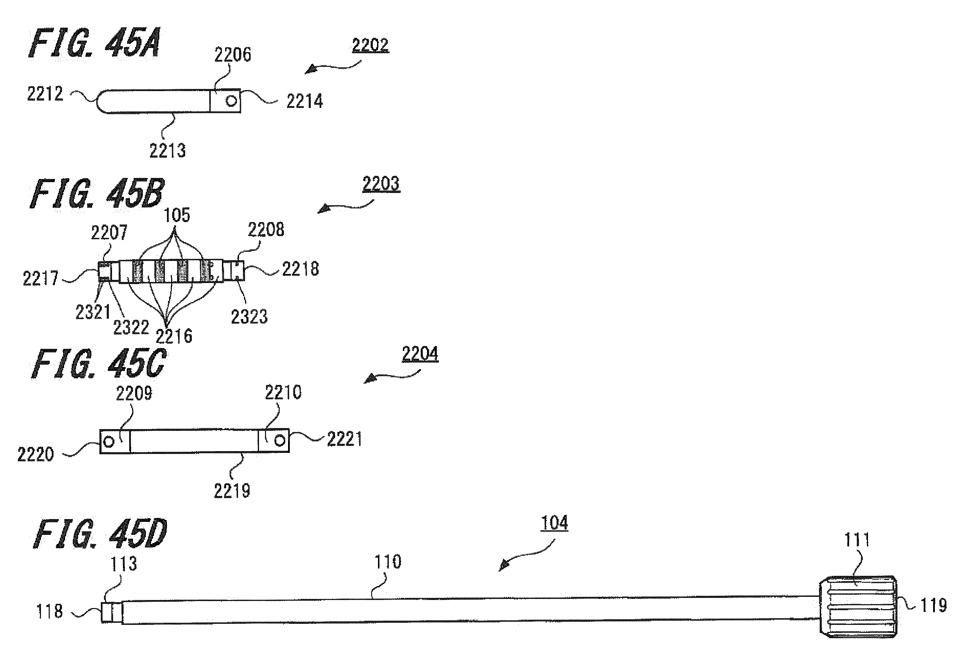

First, the general configuration of the electric stimulator related to the tenth embodiment will be described with reference to Fig. 44 and Figs. 45A to 45D.
Fig. 44 is a perspective view showing an overall electric stimulator related to the tenth embodiment of the invention.
Figs. 45A to 45D are exploded external views when the electric stimulator shown in Fig. 44 is seen from the top face.

Similarly to the above-described respective electric stimulators, the electric stimulator 2201 is formed in the shape of a rod, generates an electric stimulation signal, and stimulates nerves or the like in a living body by the stimulation signal. The electric stimulator 2201, as shown in Fig. 44, includes a circuit block 2202, an electrode block 2203, and a coil block 2204, instead of the electrode block 102 and the circuit block 103 of the electric stimulator 101 (refer to Fig. 1) related to the first embodiment.

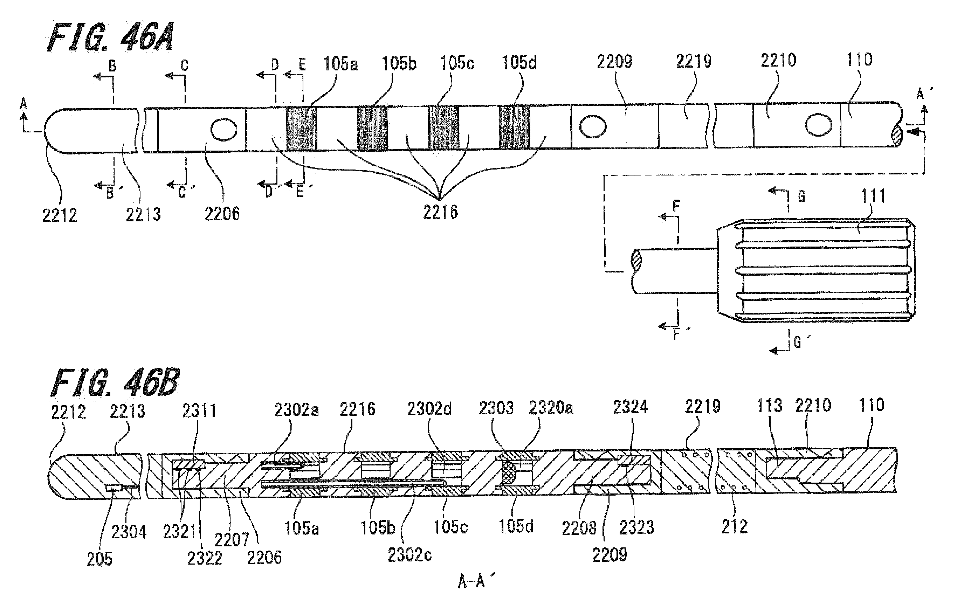

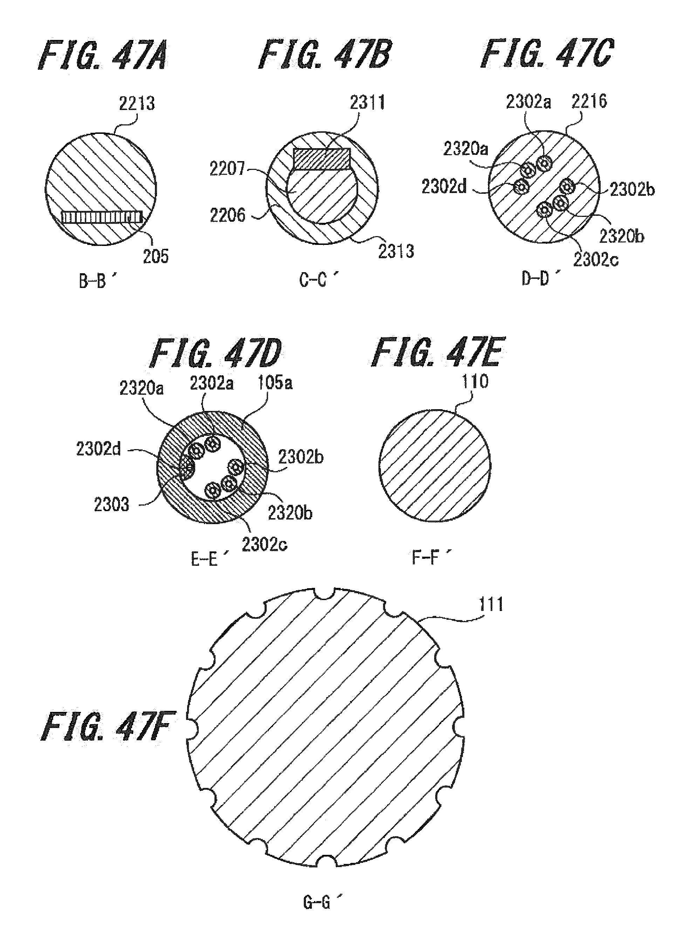

The circuit block 2202 includes a body 2213 in which a distal end 2212 is formed in a substantially hemispherical shape, and the other portions are formed in a substantially cylindrical shape. A connector part 2206 for connecting the circuit block 2202 to the electrode block 2203 is disposed on a proximal end 2214 side of the body 2213. In addition, the internal configuration of the circuit block 2202 will be described below in Figs. 46A and 46B and Figs. 47A to 47F.

The electrode block 2203 is formed in a substantially cylindrical shape with the same diameter as the substantially cylindrical portion of the circuit block 2202. This electrode block 2203 includes the above-described stimulation electrodes 105, and a body 2216 that is arranged at equal intervals such that, when the electric stimulator 2201 is implanted in a living body, the respective stimulation electrodes 105 are exposed to the living body. A connector part 2207 to be connected to the connector part 2206 of the circuit block 2202 is provided on a distal end 2217 side of the body 2216 such that the distal end 2217 of the body 2216 and the connector part 2206 of the circuit block 2202 are continuous with each other. Moreover, a connector part 2208 for connecting the coil block 2204 to a proximal end 2218 of the electrode block 2203 is disposed on the proximal end 2218 side of the body 2216. In addition, the internal configuration of the electrode block 2203 will be described below in Figs. 46A and 46B and Figs. 47A to 47F.

The coil block 2204 is formed in a substantially cylindrical shape with the same diameter as the electrode block 2203. The coil block 2204 includes a connector part 2209 that is connected to the connector part 2208 of the electrode block 2203 such that a distal end 2220 is continuous with the proximal end 2218 side of the body 2216 of the electrode block 2203. Additionally, the coil block 2204 is provided with a body 2219 that is continuous with the connector part 2209. Moreover, the coil block 2204 includes a connector part 2210 that is continuous with a proximal end 2221 side of the body 2219 to connect the support 104 to the proximal end 2221 of the coil block 2204. In addition, the internal configuration of the coil block 2204 will be described below in Figs. 46A and 46B and Figs. 47A to 47F.

Next, the internal configuration of the electric stimulator related to the tenth embodiment will be described with reference to Figs. 46A and 46B and Figs. 47A to 47F.
Figs. 46A and 46B are enlarged views showing the electric stimulator related to the tenth embodiment of the invention, and its axial internal structure.
Fig. 46A is an enlarged external view when the electric stimulator shown in Fig. 44 is seen from the top face.
Fig. 46B is a cross-sectional view showing an A-A' section of the electric stimulator shown in Fig. 46A.

Additionally, Figs. 47A to 47F are cross-sectional views showing the internal structure of a predetermined location in a vertical direction with respect to the axis of the electric stimulator related to the tenth embodiment of the invention.
Fig. 47A is a cross-sectional view showing a B-B' section of the electric stimulator shown in Fig. 46A.
Fig. 47B is a cross-sectional view showing a C-C' section of the electric stimulator shown in Fig. 46A.
Fig. 47C is a cross-sectional view showing a D-D' section of the electric stimulator shown in Fig. 46A.
Fig. 47D is a cross-sectional view showing an E-E' section of the electric stimulator shown in Fig. 46A.
Fig. 47E is a cross-sectional view showing an F-F' section of the electric stimulator shown in Fig. 46A.
Fig. 47F is a cross-sectional view showing a G-G' section of the electric stimulator shown in Fig. 46A.

First, the internal configuration of the circuit block 2202 will be described.
The body 2213 is made of resin materials that have pliability and biocompatibility, for example, materials, such as silicone or polyurethane. The distal end 2212 of the body 2213 is substantially hemispherical as described above, and the radius thereof is preferably within a range of about 0.5 mm to 1.5 mm. Portions of the body 2213 other than the distal end 2212 are formed in a substantially cylindrical shape, and the diameter thereof is preferably in a range of about 1 to 3 mm.

The stimulation circuit 205 described in Fig. 3B, and conducting wires 2304 that electrically connect the stimulation circuit 205 and the connector part 2206 are embedded in the body 2213, (refer to Figs. 46B and 47A). In addition, the conducting wires 2304 include conducting wires for electric supply for obtaining electric power for generating an electric stimulation signal from the coil block 2204, and conducting wires for a stimulation signal for applying the generated electric stimulation signal independently to the respective stimulation electrodes 105.

The connector part 2206 is made of the same material as the body 2213 except for an electrical connecting portion 2311 that will be described below, and is formed in a hollow, substantially cylindrical shape in the axial direction. The external diameter of the connector part 2206 is approximately equal to the external diameter of the body 2213, and the hollow portion of the connector part 2206 is formed in almost the same shape as the outer shell of the connector part 2207 so that the connector part 2206 is connectable to the connector part 2207 of the electrode block 2203. Moreover, the connector part 2206 includes the electrical connecting portion 2311 electrically connected to the conducting wires 2304, i.e., the conducting wires for electric supply, and the conducting wires for a stimulation signal. In addition, the shape of the outer shell of the connector part 2207 will be described below along with the internal configuration of the electrode block 2203.

Next, the internal configuration of the electrode block 2203 (refer to Fig. 45B) will be described.
The body 2216 is made of the same material as the body 2213 of the circuit block 2202. A portion of the body 2216 in the axial direction is formed in a hollow, substantially cylindrical shape, and the external diameter of the body 2216 is equal to the diameter of the substantially cylindrical portion of the body 2213 of the circuit block 2202. The four stimulation electrodes 105 are fixed so as to be exposed to the surface of the body 2216.

One end of each of conducting wires 2302a to 2302d is bonded to each of the stimulation electrodes 105a to 105d with solder 2303 (refer to Figs. 46B and 47D). The other end of each of the conducting wires 2302a to 2302d is electrically connected to the connector part 2207. In addition, locations other than the locations of the conducting wires 2302 that are bonded with the solder 2303 and the locations of the conducting wires 2302 that are electrically connected to the connector part 2207 are insulated and coated with PTFE or ETFE and are completely embedded in the body 2216 (refer to Fig. 47C).

The connector part 2207 is formed of the same material as the body 2216, and is formed as a cutout part in which a level difference is provided from the external diameter of the substantially cylindrical body 2216 (refer to Fig. 47B). This cutout part is formed by a predetermined distance in the axial direction from the distal end 2217 (refer to Fig. 45B). Additionally, six connector pins are exposed and arranged on the cutout part (refer to Figs. 45B and 46B). Four among the connector pins are connector pins 2321 for a stimulation electrode electrically connected independently to the stimulation electrodes 105a to 105d via the conducting wires 2302a to 2302d, respectively, and two connector pins are connector pins 2322 for electric supply electrically connected to the connector part 2208 via conducting wires 2320a and 2320b. When the connector part 2207 is connected to the connector part 2206 of the circuit block 2202, the connector pins 2321 for a stimulation electrode are electrically connected to the conducting wires for a stimulation signal of the conducting wires 2304 via the electrical connecting portion 2311, and similarly, the connector pins 2322 for electric supply are connected to the conducting wires for electric supply of the conducting wires 2304 via the electrical connecting portion 2311.

On the other hand, the connector part 2208 is made of the same material as the connector part 2207, and is formed in the same shape. The connector part 2208 is arranged such that two connector pins 2323 are exposed onto a cutout part, differently from the connector part 2207 (refer to Figs. 45B and 46B). The two connector pins 2323 are electrically connected to the connector pins 2322 for electric supply of the connector part 2207 via the conducting wires 2320, respectively. That is, when the connector part 2207 is connected to the connector part 2206 of the circuit block 2202, the two connector pins 2323 are connected to the stimulation circuit 205 via the electrical connecting portion 2311 and the conducting wires for electric supply of the conducting wires 2304.

Next, the internal configuration of the coil block 2204 (refer to Fig. 45C) will be described. The body 2219 is made of the same material as the body 2216 of the electrode block 2203.
The body 2219 is formed in a substantially cylindrical shape, and the diameter thereof is approximately equal to the external diameter of the body 2216 of the electrode block 2203. The coil portion 212 that is formed so as to be wound with the axial direction as an axis is embedded on the body 2219. In addition, the coil portion 212 is electrically connected to the connector part 2209 on the distal end 2220 side of the body 2219.

The connector part 2209 is made of the same material as the body 2219 except for an electrical connecting portion 2324 that will be described below, and is formed in a hollow, substantially cylindrical shape in the axial direction. The external diameter of the connector part 2209 is approximately equal to the external diameter of the body 2219, and the hollow portion of the connector part 2209 is formed in almost the same shape as the outer shell of the connector part 2208 so that the connector part 2209 is connectable to the connector part 2208 of the electrode block 2203.

The connector part 2209 further includes the electrical connecting portion 2324 independently electrically connected to one end and the other end of the coil portion 292, respectively. When the connector part 2209 and the connector part 2208 of the electrode block 2203 are connected together and the connector part 2206 of the circuit block 2202 and the connector part 2207 of the electrode block 2203 are connected together, the coil portion 212 is electrically connected to the stimulation circuit 205 of the circuit block 2202. In addition, although the connector part 2210 is the same shape as the connector part 2209, all of these are made of the same material as the body 2219.

### <Description of eleventh embodiment of the invention>

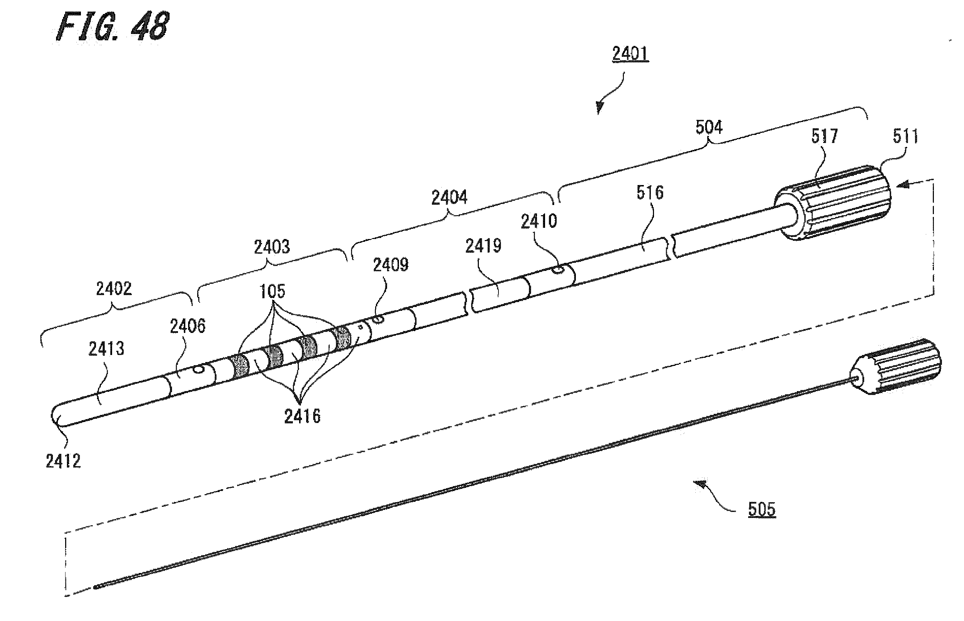

Next, an eleventh embodiment of the invention will be described with reference to Figs. 48 to 51. An electric stimulator 2401 related to the eleventh embodiment shown in Figs. 48 to 51 forms a lumen for a stylet in the electric stimulator 2201 related to the tenth embodiment. Portions whose configuration is common to the above-described respective embodiments will be designated by the same reference numerals, and the description thereof will be omitted. In addition, since the implantation procedure of the electric stimulator 2401 related to the eleventh embodiment is the same as the implantation procedure of the electric stimulator 501 related to the second embodiment, the description thereof will be omitted.

### [13. Configuration of electric stimulator]

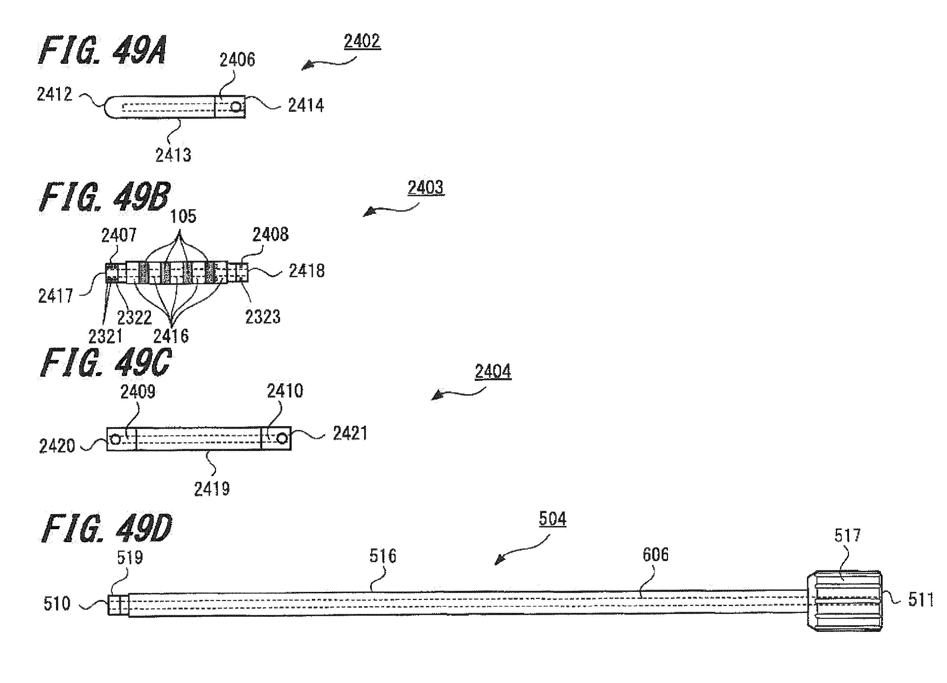

First, the general configuration of the electric stimulator related to the eleventh embodiment will be described with reference to Fig. 48 and Figs. 49A to 49D.
Fig. 48 is a perspective view showing an overall electric stimulator related to the eleventh embodiment of the invention.
Figs. 49A to 49D are exploded external views when the electric stimulator shown in Fig. 48 is seen from the top face.

As described above, the electric stimulator 2401 provides the lumen for a stylet in the axial direction of the electric stimulator 2201 of the tenth embodiment. Therefore, the electric stimulator 2401 is constituted by a circuit block 2402, an electrode block 2403, a coil block 2404, and the support 504 that form a hollow portion in the axial direction, instead of the circuit block 2202, the electrode block 2203, the coil block 2204, and the support 104 (refer to Fig. 11 or the like) shown in Fig. 44.

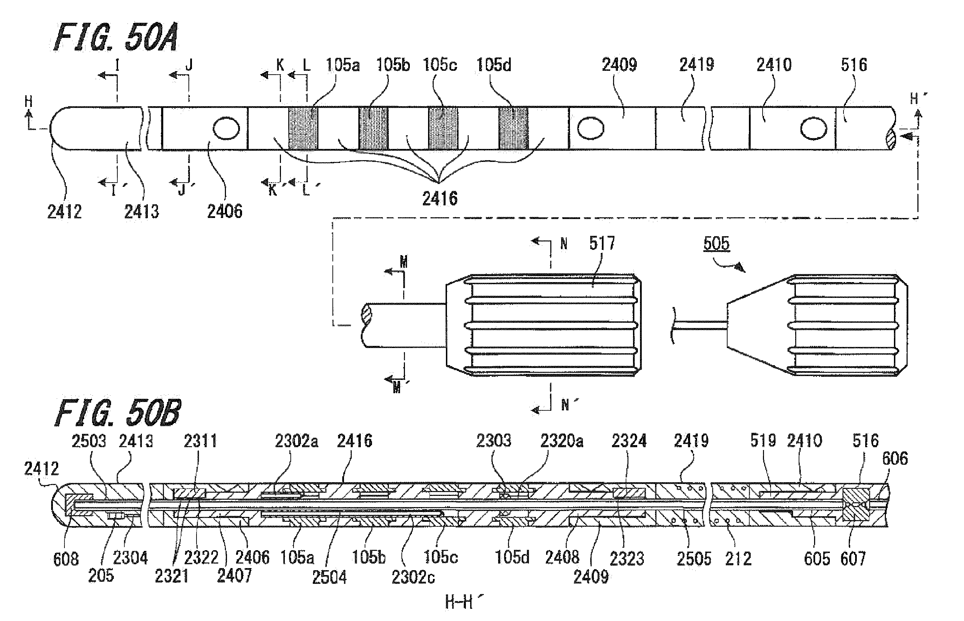

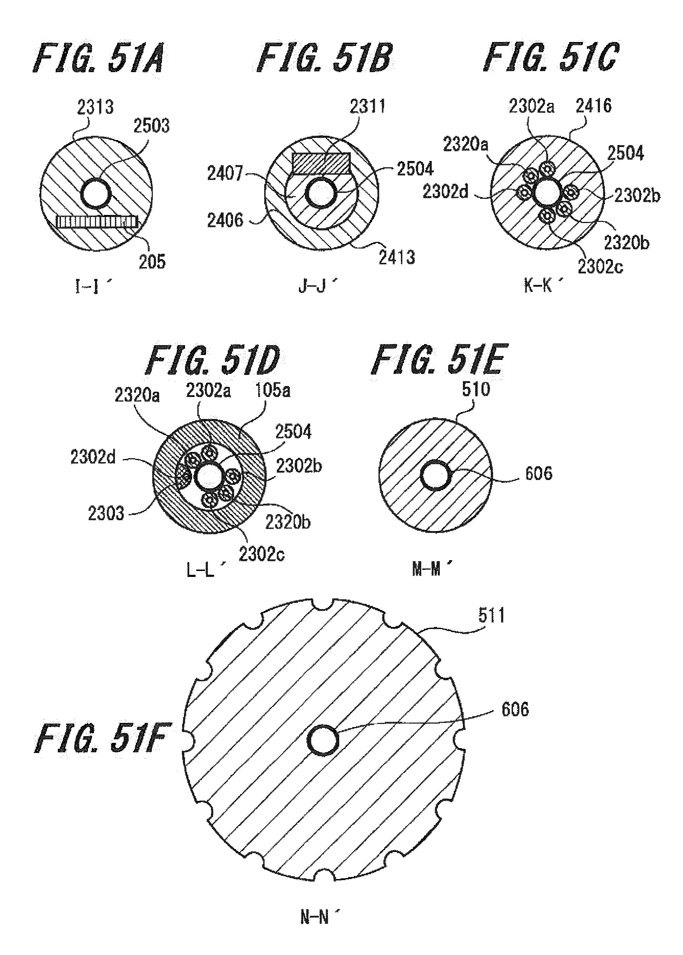

In the electric stimulator 2401, as shown in Figs. 49A to 49D, when the respective blocks are connected together in order of the circuit block 2402, the electrode block 2403, the coil block 2404, and the support 504, each of these blocks forms the lumen for a stylet that communicates in the axial direction of the blocks. In this case, as for the lumen for a stylet, an opening is provided in the proximal end 511, and this opening is connected up to the vicinity of a distal end 2412. In addition, the diameter of the lumen for a stylet is preferably approximately equal to or slightly longer than the diameter of the stylet 505. In addition, the internal configuration of the circuit block 2402, the electrode block 2403, and the coil block 2404 will be described below in Figs. 50A and 50B and Figs. 51A to 51F. In addition, since the internal structure of the support 504 has been described in Figs. 13A and 13B, the detailed description thereof is omitted.

Next, the internal configuration of the electric stimulator related to the eleventh embodiment will be described with reference to Figs. 50A and 50B and Figs. 51A to 51F.
Figs. 50A and 50B are enlarged views showing the electric stimulator related to the eleventh embodiment of the invention, and its axial internal structure.
Fig. 50A is an enlarged external view when the electric stimulator shown in Fig. 48 is seen from the top face.
Fig. 50B is a cross-sectional view showing an H-H' section of the electric stimulator shown in Fig. 50A.

Additionally, Figs. 51A to 51F are cross-sectional views showing the internal structure of a predetermined location in a vertical direction with respect to the axis of the electric stimulator related to the eleventh embodiment of the invention.
Fig. 51A is a cross-sectional view showing an I-I' section of the electric stimulator shown in Fig. 50A.
Fig. 51B is a cross-sectional view showing a J-J' section of the electric stimulator shown in Fig. 50A.
Fig. 51C is a cross-sectional view showing a K-K' section of the electric stimulator shown in Fig. 50A.
Fig. 51D is a cross-sectional view showing an L-L' section of the electric stimulator shown in Fig. 50A.
Fig. 51E is a cross-sectional view showing an M-M' section of the electric stimulator shown in Fig. 50A.
Fig. 51F is a cross-sectional view showing an N-N' section of the electric stimulator shown in Fig. 50A.

First, the internal configuration of the circuit block 2402 will be described.
A pipe 2503 is made of materials that have biocompatibility, insulation, and pliability, for example, PTFE or ETFE, and is formed in a hollow, substantially cylindrical shape in the axial direction. It is desirable that the external diameter of the pipe 2503 be about 0.1 mm to 1 mm, and the internal diameter of the pipe 2503 be approximately equal to or slightly longer than the diameter of the stylet 505 so that the stylet 505 can pass through the inside of the pipe 2503. One end (end at the distal end 2412) of such a pipe 2503 is connected to the receiving portion 608 described in Fig. 13B. The pipe 2503 and the receiving portion 608 are housed in and fixed to an outer layer part made up of a body 2413, and a connector part 2406 (refer to Fig. 50B).

The body 2413 is made of resin materials that have pliability and biocompatibility, for example, materials, such as silicone or polyurethane. The distal end 2412 of the body 2413 is substantially hemispherical, and the radius thereof is preferably within a range of about 0.5 mm to 1.5 mm. Portions of the body 2413 other than the distal end 2412 are formed in a substantially cylindrical shape that is hollow in the axial direction. The external diameter of the hollow, substantially cylindrical portion is desirably in a range of about 1 to 3 mm, and the internal diameter is approximately equal to the external diameter of the receiving portion 608 or the pipe 2503, in order to house and fix the receiving portion 608 and the pipe 2503. The stimulation circuit 205 described in Figs. 3B and 5, and the conducting wires 2304 that electrically connects the stimulation circuit 205 and the connector part 2406 are embedded in the body 2413, (refer to Fig. 51A).

The connector part 2406 is made of the same material as the body 2413 except for the electrical connecting portion 2311 that will be described below, and is formed in a hollow, substantially cylindrical shape in the axial direction. The external diameter of the connector part 2406 is approximately equal to the external diameter of the body 2413, and the hollow portion of the connector part 2406 is formed in almost the same shape as the outer shell of a connector part 2407 so that the connector part 2406 is connectable to the connector part 2407 of the electrode block 2403. In addition, since the conducting wires 2304 and the electrical connecting portion 2311 are the same as those in the tenth embodiment, these are designated by the same reference numerals, and the description thereof is omitted.

Next, the internal configuration of the electrode block 2403 will be described.
A pipe 2504 is the same as the pipe 2503 except for the axial length thereof. The pipe 2504 is housed in and fixed to an outer layer part including the connector part 2407, a body 2416, the stimulation electrodes 105, and a connector part 2408 (refer to Fig. 50B).

The body 2416 is made of the same material as the body 2413 of the circuit block 2402, and is formed in a hollow, substantially cylindrical shape in the axial direction. The external diameter of the body 2416 is equal to the external diameter of the hollow, substantially cylindrical portion of the body 2413 of the circuit block 2402, and the internal diameter of the body 2416 is approximately equal to the external diameter of the pipe 2504 in order to house and fix the pipe 2504. The four stimulation electrodes 105 are provided so as to be exposed to the surface of the body 2416.

One end of each of the conducting wires 2302a to 2302d is bonded to each of the stimulation electrodes 105a to 105d with the solder 2303 (refer to Figs. 50B and 51D). The other end of each of the conducting wires 2302a to 2302d is electrically connected to the connector part 2407. In addition, locations other than the locations of the conducting wires 2302 that are bonded with the solder 2303 and the locations of the conducting wires 2302 that are electrically connected to the connector part 2407 are insulated and coated with PTFE or ETFE and are completely embedded in the body 2415 (refer to Fig. 51 C).

The connector part 2407 is formed of the same material as the body 2416, and is formed as a cutout part in which a level difference is provided from the external diameter of the substantially cylindrical body 2416 (refer to Fin. 51 B). This cutout part is formed by a predetermined distance in the axial direction from a distal end 2417. Additionally, a total of the six connector pins 2321 and 2322 are exposed and arranged on the cutout part (refer to Figs. 49B). Moreover, a substantially cylindrical shape that has a diameter equal to the external diameter of the pipe 2504 is formed on the axis of the connector part 2407 so as to house and fix the pipe 2504.

On the other hand, the connector part 2408 is made of the same material as the connector part 2407, and is formed in the same shape. The connector part 2408 is arranged such that the two connector pins 2323 are exposed onto a cutout part, differently from the connector part 2407 (refer to Figs. 49B). In addition, since the connector pins 2321 to 2323 are the same as those in the tenth embodiment, these are designated by the same reference numerals, and the description thereof is omitted.

Next, the internal configuration of the coil block 2404 (refer to Fig. 49C) will be described.
A pipe 2505 is the same as the pipes 2503 and 2504 except for the axial length thereof. The pipe 2505 is housed in and fixed to an outer layer part including a connector part 2409, a body 2419, and a connector part 2410 (refer to Fig. 50B).

The body 2419 is made of the same material as the body 2416 of the electrode block 2403. The body 2419 is formed in a hollow, substantially cylindrical shape in the axial direction. The external diameter of the body 2419 is approximately equal to the external diameter of the body 2416 of the electrode block 2403, and the internal diameter of the body 2419 is made approximately equal to the external diameter of the pipe 2505 in order to house and fix the pipe 2505. The coil portion 212 that is formed so as to be wound with the axial direction as an axis is embedded on the body 2419. In addition, the coil portion 212 is electrically connected to the connector part 2409 on a distal end 2420 (refer to Fig. 49C) side of the body 2419.

The connector part 2409 is made of the same material as the body 2419 except for the electrical connecting portion 2324, and is formed in a hollow, substantially cylindrical shape in the axial direction. The external diameter of the connector part 2409 is approximately equal to the external diameter of the body 2419, and the hollow portion of the connector part 2409 is formed in almost the same shape as the outer shell of the connector part 2408 so that the connector part 2409 is connectable to the connector part 2408 of the electrode block 2403. In addition, since the electrical connecting portion 2324 is the same as that described in the tenth embodiment, this is designated by the same reference numeral, and the description thereof is omitted. In addition, although the connector part 2410 is the same shape as the connector part 2409, this is made of the same material as the body 2419.

### <Description of twelfth embodiment of the invention>

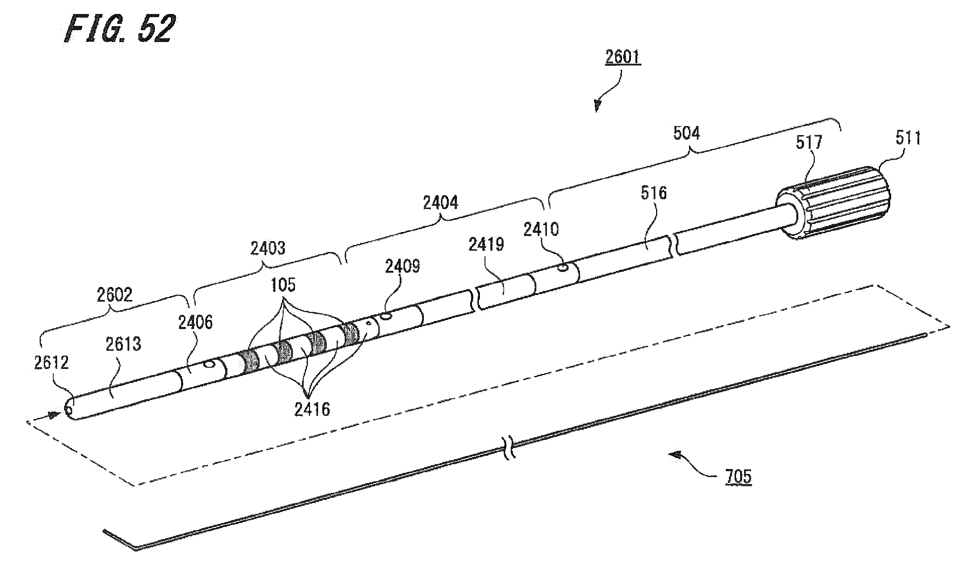

Next, a twelfth embodiment of the invention will be described with reference to Figs. 52 to 54. An electric stimulator 2601 related to the twelfth embodiment shown in Figs. 52 to 54 forms a lumen for a guide wire in the electric stimulator 2201 related to the tenth embodiment. Portions whose configuration is common to the above-described respective embodiments will be designated by the same reference numerals, and the description thereof will be omitted, In addition, since the implantation procedure of the electric stimulator 2601 related to the twelfth embodiment is the same as the implantation procedure of the electric stimulator 701 related to the third embodiment, the description thereof will be omitted.

### [14. Configuration of electric stimulator]

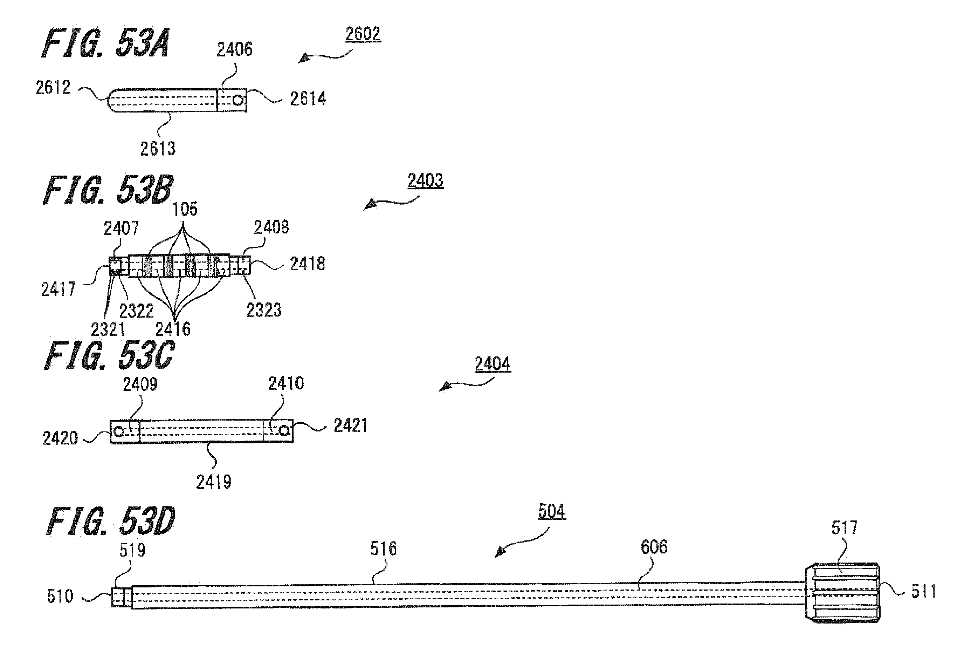

First, the general configuration of the electric stimulator related to the twelfth embodiment will be described with reference to Fig. 52 and Figs. 53A to 53D.
Fig. 52 is a perspective view showing an overall electric stimulator related to the twelfth embodiment of the invention.
Figs. 53A to 53D are exploded external views when the electric stimulator shown in Fig. 52 is seen from the top face.

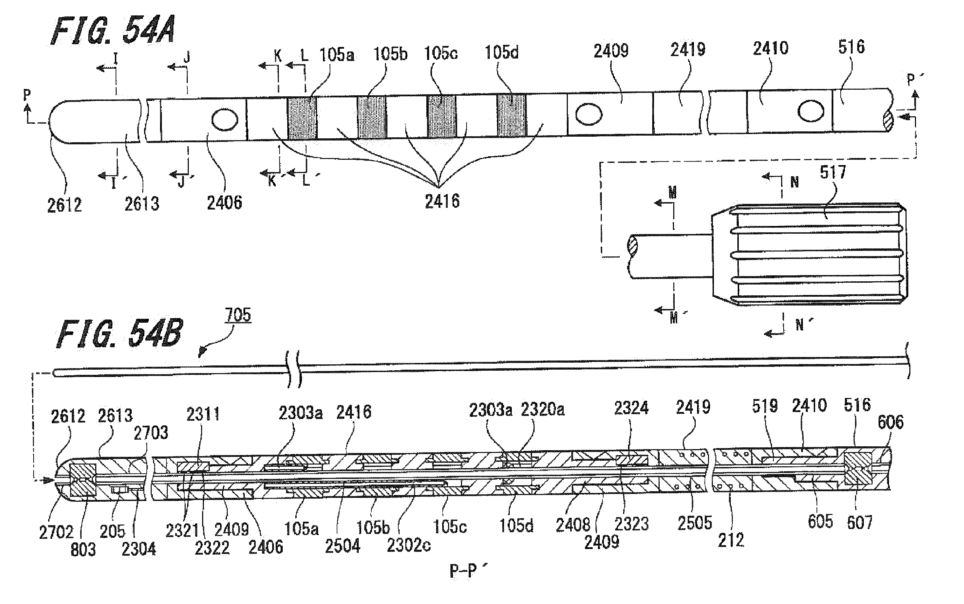

Similarly to the above-described respective electric stimulators, the electric stimulator 2601 is formed in the shape of a rod, generates an electric stimulation signal, and stimulates nerves or the like in a living body by the stimulation signal. Since the electric stimulator 2601 has the lumen for a guide wire on the axis as described above, the electric stimulator 2601 is formed in a hollow, substantially cylindrical shape. It is noted that the lumen for a guide wire is provided as a through hole that opens to the proximal end 511 and also opens to a distal end 2612. Therefore, the electric stimulator 2601 has a configuration in which the circuit block 2402 of the electric stimulator 2401 of the eleventh embodiment is replaced with a substantially cylindrical circuit block 2602 formed with a hollow portion that communicate in the axial direction. In addition, the internal configuration of the circuit block 2602 will be described in Figs. 54A and 54B.

Next, the internal configuration of the electric stimulator related to the twelfth embodiment will be described with reference to Figs. 54A and 54B and Figs. 51A to 51F. Figs. 54A and 54B are enlarged views showing the electric stimulator related to the twelfth embodiment of the invention, and its axial internal structure.
Fig. 54A is an enlarged external view when the electric stimulator shown in Fig. 52 is seen from the top face.
Fig. 54B is a cross-sectional view showing a P-P' section of the electric stimulator shown in Fig. 54A.

Additionally, although Figs. 51A to 51F, as described above, are cross-sectional views showing the internal structure of a predetermined location in a radial direction with respect to the axis of the electric stimulator 2401 related to the tenth embodiment of the invention, these drawings are also cross-sectional views showing the internal structure in a radial direction with respect to the electric stimulator 2601 related to the twelfth embodiment.

Here, only the internal configuration of the circuit block 2602 (refer to Fig. 53A) will be described. Pipes 2702 and 2703 are the same as the pipes 2504 to 2505 except for the axial length thereof.
The valve 803 described in Fig. 17B is provided between the pipe 2702 and the pipe 2703 in the axial direction. Even if the guide wire 705 is inserted and removed via the valve 803, liquids, such as body fluids, can be prevented from entering the insides of the circuit block 2602, the electrode block 2403 and the coil block 2404 from the hole provided in the distal end 2612 of a body 2613.

The pipes 2702 and 2703 and the valve 803 are housed in and fixed to an outer layer part made up of the body 2613, and the connector part 2406 (refer to Fig. 54B).

Although the body 2613 is made of, for example, materials, such as silicone or polyurethane, the body 2613 has a substantially cylindrical hole in the distal end 2612 thereof. The diameter of this hole is approximately equal to the external diameter of the pipe 2702. In addition, the external diameter of the body 2613 is the same as that of the body 2413 (refer to Figs. 50A and 50B) of the eleventh embodiment. In addition, since the connector part 2406 is the same as that described in the eleventh embodiment, the description thereof is omitted.

As described above, in the tenth to twelfth embodiments, the electrode block is provided between the circuit block and the coil block. Thus, the circuit block and the coil block can be arranged to be apart from each other. Since the influence of electromagnetic waves on the stimulation circuit of the circuit block can be reduced when the electromagnetic waves for performing communication/electric supply from an external device (a controller outside of the body) are transmitted to the coil portion of the coil block, the probability of occurrence of malfunction, failure, or the like of the apparatus, can be lowered.

### <Modifications>

In addition, although the electrode block, the circuit block, and the support are made attachable and detachable, respectively, by the connectors in the above-described respective embodiments, the connectors may be omitted and the electrode block and the circuit block may be integrated in advance, the circuit block and the support may be integrated in advance, or all the blocks may be integrated in advance. Additionally, although the rechargeable battery is used as a power source in the above-described respective embodiments, a primary battery may be used instead of the rechargeable battery, or a capacitor may be used instead of the rechargeable battery and the electric stimulator may be operated, always receiving electric supply from a controller outside the body.

Additionally, in the above-described respective embodiments, when the electric stimulator is implanted into a living body, a form in which the electric stimulator passes through the epidural needle is used. However, it is possible to further improve the precision of the arrangement of the stimulation electrodes into a living body by passing the electric stimulator through the inside of a cannula and inserting the electric stimulator into the living body after the cannula having pliability is led through the epidural needle in advance to near a part to be stimulated.

Additionally, in the above-described tenth to twelfth embodiments, the circuit block, the electrode block, the coil block, and the support are connected in order from the distal end side. However, even if the coil block, the electrode block, the circuit block, and the support are connected in order from the distal end side, since the coil block and the circuit block are apart from each other, it is needless to say that the effects described in the tenth to twelfth embodiments are obtained.

Additionally, in the above-described tenth to twelfth embodiments, the coil block includes only with the coil portion. However, a portion of the charging section, for example, a rectifier circuit, may be built in the coil block, or a portion of the coil portion (except a coil) may be built in the circuit block.

Additionally, in the above-described seventh to twelfth embodiments, it is needless to say that the supports in these respective embodiments can be substituted with the supports related to the above-described fourth to sixth embodiments.

Additionally, in the above-described first to third and seventh to twelfth embodiments, the fixtures related to the above-described fourth to sixth embodiments may be attached to the cut surface of the support.

Additionally, in the above-described embodiments, the form in which the electrode block and the circuit block are connected together is equivalent to the stimulation circuit block set forth in the claims.

Although the respective embodiments of the invention have been described hitherto, the invention is not limited to the above respective embodiments, and it is needless to say that other modifications or applications are included without departing from the scope of the invention set forth in the claims.

### Reference Signs List

101, 501, 701, 901, 1101, 1301, 1501, 1701, 1901, 2201, 2401, 2601: ELECTRIC STIMULATOR
102, 502, 702, 1503, 1703, 2103, 2203, 2403: ELECTRODE BLOCK,
103, 503, 1504, 1704, 2104, 2202, 2402, 2502: CICUIT BLOCK
104, 504, 902, 1102, 1402, 1404, 1406, 1410. 2105: SUPPORT
105: STIMULATION ELECTRODE
106, 108, 110, 512, 514, 516, 712, 1513, 1516, 1519, 1713, 1716, 1719, 1913, 2213, 2216, 2219, 2413, 2416, 2419 AND 2613: BODY
107, 109, 112, 113, 513, 515, 518, 519, 1506 TO 1510, 1706 TO 1710, 2206 TO 2210, 2406 TO 2410: CONNECTOR PART
111,517: HOLDER PART
205: STIMULATION CIRCUIT
210, 1606, 2321 TO 2323: CONNECTOR PIN
211, 224, 1604, 1605, 2311, 2324: ELECTRICAL CONNECTING PORTION
212: COIL PORTION
402: EPIDURAL NEEDLE
404: LIVING BODY
405: EPIDURAL SPACE
406: THREAD
505: STYLET
603 TO 606, 802, 1803 TO 1805, 2002, 2003, 2503 TO 2505, 2702, 2703: PIPE
608: RECEIVING PORTION
705: GUIDE WIRE
607, 803: VALVE
903, 1103: FIRST BODY
904, 1104, 1403, 1407: SECOND BODY
905: FIXTURE
1002, 1202: FIXTURE FIXING PORTION
1003, 1203: EASILY-CUT PORTION
1408: BICONICAL PORTION
1409: MARKING PORTION
1413: RING
1502, 1702, 1902, 2102: INSERTION BLOCK
2204, 2404: COIL BLOCK

## Claims

1. A rod-shaped electric stimulator implanted in a living body, the electric stimulator comprising:
a stimulation circuit block including a stimulation electrode that stimulates nerves or muscles in the living body and a stimulation circuit that is electrically connected to the stimulation electrode to apply a stimulation signal to the stimulation electrode; and
a support that is connected to the stimulation circuit block to hold the stimulation electrode at an implantation position in the living body,
wherein the stimulation circuit block and the support, except for at least one end of the support, have a shape capable of being inserted into a duct of a tubular lead-in tool.

2. The electric stimulator according to Claim 1,
wherein the support has a connector at the other end thereof, and the stimulation circuit block includes connectors connectable to the connector of the support at both ends thereof.

3. The electric stimulator according to Claim 1 or 2,
wherein the stimulation electrode is provided at the stimulation circuit block so as to become asymmetric to an axial center of the stimulation circuit block.

4. The electric stimulator according to any one of Claims 1 to 3,
wherein the stimulation circuit block is separable into an electrode block having the stimulation electrode, and a circuit block having the stimulation circuit.

5. The electric stimulator according to Claim 4,
wherein each of the electrode block and the circuit block includes a connector connectable to the connector of the support at one end thereof, and a connector that allows connection between the electrode block and the circuit block at the other end thereof, and
when the connector at the other end of the electrode block and the connector at the other end of the circuit block are connected together, the stimulation electrode and the stimulation circuit are electrically connected together.

6. The electric stimulator according to Claim 5,
wherein the connector at one end of the electrode block and the connector at the other end of the circuit block are connectable together, and
when the connector at one end of the electrode block and the connector at the other end of the circuit block are connected together, the stimulation electrode and the stimulation circuit are electrically connected together.

7. The electric stimulator according to Claim 6,
wherein the connector at the other end of the electrode block is connectable to the connector of the support.

8. The electric stimulator according to any one of Claims 4 to 7,
wherein the stimulation electrode is provided at the electrode block so as to become asymmetric to an axial center of the electrode block.

9. The electric stimulator according to any one of Claims 5 to 8, further comprising, when one connector of the electrode block or the circuit block is not connected, an insertion block having a connector connectable to the connector that is not connected.

10. The electric stimulator according to any one of Claims 1 to 9, wherein the support is not provided with an electric line, and is capable of being cut in arbitrary positions.

11. The electric stimulator according to any one of Claims 1 to 10,
wherein the support is formed with a lumen that penetrates from one end to the other end, and the stimulation circuit block is formed with a lumen that communicates with the lumen of the support.

12. The electric stimulator according to any one of Claims 1 to 11,
wherein at least a portion of the support is formed with a plurality of easily-cut portions at predetermined intervals in the axial direction of the support, and the support is cut in the easily-cut portions.

13. The electric stimulator according to Claim 12,
wherein the support is fixed to a tissue in the living body in the easily-cut portions.

14. The electric stimulator according to any one of Claims 1 to 13,
wherein the support has a graduation for measuring how deep the electric stimulator is implanted in the living body.

15. The electric stimulator according to any one of Claims 1 to 14, further comprising a fixture connected so as to cover a cut surface of the support when the support is cut, and the fixture is fixed to a tissue in the living body.

16. The electric stimulator according to any one of Claims 1 to 15,
wherein the stimulation circuit block is substantially cylindrical.

17. The electric stimulator according to Claim 16,
wherein the support has almost the same cylindrical shape as the stimulation circuit block.

18. The electric stimulator according to any one of Claims 1 to 17,
wherein the stimulation circuit block further includes a power supply part that supplies electric power for generating a stimulation signal to the stimulation circuit.

19. The electric stimulator according to Claim 18,
wherein the power supply part has a coil capable of generating electric power by electromagnetic induction from the outside.

20. The electric stimulator according to any one of Claims 1 to 19,
wherein the stimulation circuit block and the support are pliable to a bendable degree.

21. The electric stimulator according to Claim 19,
wherein the coil is wound with the axial direction of the stimulation circuit block as an axis.

22. The electric stimulator according to any one of Claims 1 to 21,
wherein the stimulation electrode is placed in a spinal epidural space, and spinal nerves are stimulated by the stimulation electrode.
